# EUROPEAN PATENT SPECIFICATION

(11) **EP 3 479 696 B1**
(45) Date of publication and mention of the grant of the patent: **09.06.2021**
(21) Application number: 17820499.6
(22) Date of filing: 27.06.2017
(51) Int. Cl.: C07D 413/06, A01N 43/34, A01N 43/707, A01N 43/72, A01N 25/02, A01N 25/08, A01N 25/30, A01N 25/04, A01N 25/14, A01N 25/12

(54) **PYRIDINE-BASED COMPOUND INCLUDING ISOXAZOLINE RING, AND USE THEREOF AS HERBICIDE**
PYRIDINBASIERTE VERBINDUNG MIT EINEM ISOXAZOLINRING UND VERWENDUNG DAVON ALS HERBIZID
COMPOSÉ À BASE DE PYRIDINE COMPRENANT UN CYCLE ISOXAZOLINE, ET SON UTILISATION COMME HERBICIDE

(30) Priority: 27.06.2016 KR 20160080169
(43) Date of publication of application: 08.05.2019
(73) Proprietor: Moghu Research Center Ltd., Yuseong-gu, Daejeon 34115 (KR); Korea Research Institute of Chemical Technology, Yuseong-gu, Daejeon 34114 (KR)
(72) Inventor: KO, Young Kwan, Daejeon 34140 (KR); KIM, Eun Ae, Daejeon 34071 (KR); LEE, Ill Young, Sejong 30098 (KR); KOO, Dong Wan, Daejeon 34152 (KR); RYU, Jae Wook, Daejeon 34075 (KR); YON, Gyu Hwan, Daejeon 34140 (KR); YEOM, Hyun Suk, Daejeon 34022 (KR); LIM, Hee Nam, Cheonan-si Chungcheongnam-do 31118 (KR); LEE, So-Young, Daejeon 35272 (KR); PARK, Chan Yong, Incheon 21100 (KR); KWAK, Mi-Young, Daejeon 34178 (KR); KOO, Suk-Jin, Daejeon 34049 (KR); HWANG, Ki-Hwan, Daejeon 34048 (KR); KIM, Sung-Hun, Daejeon 34568 (KR); LIM, Jong-Su, Daejeon 35398 (KR); LEE, Dong-Guk, Daejeon 34187 (KR); CHUNG, Kun-Hoe, Daejeon 34071 (KR); CHO, Nam-Gyu, Daejeon 34386 (KR); NAM, Jun-Ho, Daejeon 34888 (KR)
(74) Representative: Ladendorf, Oliver
(86) International application number: PCT/KR2017/006738
(87) International publication number: WO 2018/004223

(56) References cited:
- EP-B1- 1 284 969
- WO-A1-99/24409
- WO-A2-2005/034952
- KR-B1- 101 093 102
- US-A1- 2003 199 699
- US-A1- 2010 222 392

## Description

### [Technical Field]

The present disclosure relates to a pyridine-based compound including an isoxazoline ring, which is a non-aromatic heterocycle, and use thereof as a herbicide.

### [Prior Art]

Herbicides are crop protection agents that do not damage crops, and suppress the growth of weeds or kills weeds. According to a chemical structure, herbicides are classified into amino acid herbicides, sulfonylurea herbicides, imidazolinone herbicides, triazine herbicides, acetamide herbicides, dinitroaniline herbicides, aryloxyphenoxypropionate herbicides, urea herbicides, carbamate herbicides, bipyridyl herbicides, pyridine herbicides, phenoxyacetic acid herbicides, diphenyl ether herbicides, cyclohexanedione herbicides, and the like.

As the pyridine herbicides, picloram, fluroxypyr, clopyralid, aminopyralid, triclopyr, and the like are used. These pyridine herbicides are used for various crops including wheat, maize, sugarcane, rice, fruits and vegetables, grasses, and the like. However, the pyridine herbicides need to be used in great amounts, production processes therefor are complicated, and the production costs are high. Accordingly, there is a need to perform researches and development continuously.

Many pyridine compounds and their herbicidal activity characteristics are disclosed in the art including, for example, US Patent Publication No. 2015-0164074 disclosing 4-amino-6-(halo-substituted-alkyl)-picolinic acid and use thereof as herbicides, US Patent Publication 2014-0274695 discloses 4-amino-6-(heterocyclic) picolinate and 6-amino-2-(heterocyclic) pyrimidine-4-carboxylate and use thereof as herbicides, US Patent Publication 2014-0274696 discloses 4-amino-6-(4-substituted-phenyl)-picolinate and 6-amino-2-(4-substituted-phenyl)-pyrimidine-4-carboxylate and use thereof as herbicides, US Patent Publication 2014-0274702 discloses 4-amino-6-(heterocyclic)picolinate and 6-amino-2-(heterocyclic)pyrimidine-4-carboxylate and use thereof as herbicides, US Patent Publication 2014-0274703 discloses 4-amino-6-(pyridyl and 2-substituted-phenyl)-picolinate and 6-amino-2-(pyridyl and 2-substituted-phenyl)-pyrimidine-4-carboxylate and use thereof as herbicides, US Patent Publication 2014-0274701 discloses 4-amino-6-(heterocyclic)picolinate and 6-amino-2-(heterocyclic)pyrimidine-4-carboxylate and use thereof as herbicides, and US Patent Publication 2015-0133301 discloses 3-alkoxy, thioalkyl, and amino-4-amino-6-(substituted)picolinate and use thereof as herbicides. 1 284 969 B1 discloses phenyl-based compounds comprising an isoxazoline ring and their use as herbicides. However, a pyridine-based compound containing an isoxazoline ring and use thereof as a herbicide according to the present disclosure have not been disclosed.

### [Detailed Description of the Invention]

### [Technical Goal of the Invention]

The present disclosure has been induced by the requirements described above, and the present disclosure has been completed by confirming that any one compound selected from a pyridine-based compound including an isoxazoline ring represented by Formula 1 and an agrochemically acceptable salt thereof has a selectivity with respect to a cultivated crop and does not harm useful crop, such as wheat, maize, and rice, and has excellent herbicidal effects before and after generation of weeds for the removal of broad-leaved weeds.

### [Technical Solution]

Embodiments of the present disclosure provide any one compound selected from a pyridine-based compound containing an isoxazoline ring represented by Formula 1 and an agrochemically acceptable salt thereof: 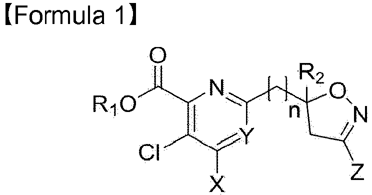 wherein, in Formula 1,
R₁ is H, a C₁ to C₄ alkyl group, or a benzyl group;
R₂ is H or a C₁ to C₂ alkyl group;
n is an integer of 0 to 3;
X is a halogen or -NR₃R₄ (wherein R₃ and R₄ are each independently H, a C₁ to C₂ alkyl group, or a C(O)methyl group);
Y is CH, C-halogen, or N; and
Z is H, halogen, a C₁ to C₄ alkyl group, a C₁ to C₄ halo alkyl group, a C₁ to C₄ alkoxy group, a C₁ to C₄ alkoxy carbonyl group, a phenyl group substituted with at least one R₅ (wherein R₅ is halogen, a C₁ to C₄ alkyl group, a C₁ to C4 alkoxy group, or a C₁ to C₄ halo alkyl group), or a heterocyclic group (wherein a heterocyclic group is a 5-membered or 6-membered saturated or unsaturated ring containing at least one selected from N, O, and S).

In addition, the present disclosure provides a herbicide including, as an active ingredient, the compound of Formula I, an agrochemically acceptable salt thereof, or a combination of the compound of Formula I and the agrochemically acceptable salt thereof.

The present disclosure provides a herbicidal composition including, as an active ingredient, a compound selected from a pyridine-based compound containing an isoxazoline ring represented by Formula 1 and an agrochemically acceptable salt thereof, or a combination of the pyridine-based compound containing an isoxazoline ring represented by Formula 1 and the agrochemically acceptable salt thereof, in an amount of about 0.1 wt% to about 99.9 wt%; and
at least one additive selected from a surfactant, a solid diluent, and a liquid diluent, in an amount of about 0.1 wt% to about 99.9 wt%.

The present disclosure provides a method of preparing a pyridine-based compound containing an isoxazoline ring represented by Formula 1 by reacting a compound represented by Formula 2 with a compound represented by Formula 3:

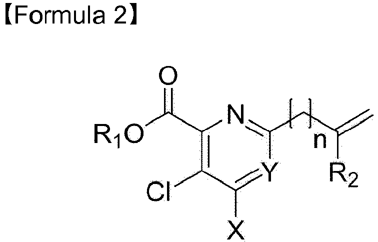

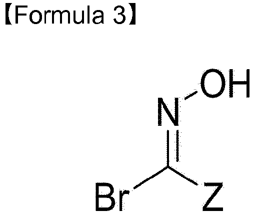

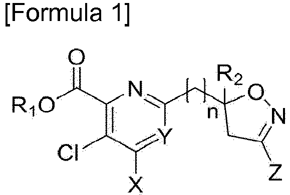

R₁, R₂, n, X, Y, and Z in Formulae 1, 2, and 3 are the same as described in Formula 1 above.

### [Effect of the Invention]

According to any one compound selected from a pyridine-based compound containing an isoxazoline ring represented by Formula 1 and an agrochemically acceptable salt thereof, a herbicide including, as an active ingredient, the compound of Formula I, the agrochemically acceptable salt thereof, or a combination of the compound of Formula I and the agrochemically acceptable salt thereof, and a method of preparing the pyridine-based compound containing an isoxazoline ring represented by Formula 1 by reacting a compound represented by Formula 2 with a compound represented by Formula 3, due to the selectivity to cultivated crops, excellent herbicidal effects may be obtained before and after the generation of weeds for the removal of weeds having broad leaves, without harming useful crops, such as weeds, maize, and rice.

### [Description of Embodiment]

The present disclosure provides any one compound selected from a pyridine-based compound containing an isoxazoline ring represented by Formula 1 and an agrochemically acceptable salt thereof. 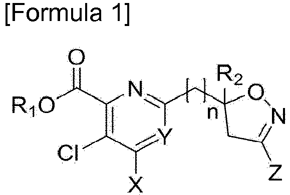 wherein, in Formula 1,
R₁ is H, a C₁ to C₄ alkyl group, or a benzyl group;
R₂ is H or a C₁ to C2 alkyl group;
n is an integer of 0 to 3;
X is a halogen or -NR₃R₄ (wherein R₃ and R₄ are each independently H, a C₁ to C₂ alkyl group, or a C(O)methyl group);
Y is CH, C-halogen, or N; and
Z is H, halogen, a C₁ to C₄ alkyl group, a C₁ to C₄ halo alkyl group, a C₁ to C₄ alkoxy group, a C₁ to C₄ alkoxy carbonyl group, a phenyl group substituted with at least one R₅ (wherein R₅ is halogen, a C₁ to C₄ alkyl group, a C₁ to C₄ alkoxy group, or a C₁ to C₄ halo alkyl group), or a heterocyclic group (wherein a heterocyclic group is a 5-membered or 6-membered saturated or unsaturated ring containing at least one selected from N, O, and S).

In one embodiment,
R₁ is H, a methyl group, an ethyl group, or a benzyl group;
R₂ is H or a methyl group;
n is an integer of 0 to 1;
X is Cl or -NR₃R₄ (wherein R₃ and R₄ are each independently H, a C₁ to C₂ alkyl group, or a C(O)methyl group);
Y is CH, CCI, CF, or N; and
Z is H, Br, Cl, a C₁ to C₄ alkyl group, a C₁ to C₄ halo alkyl group, a C₁ to C₄ alkoxy group, a C₁ to C₄ alkoxy carbonyl group, a phenyl group substituted with at least one R₅ (wherein R₅ is F, Cl, or a methoxy group) or
a heterocyclic group selected from

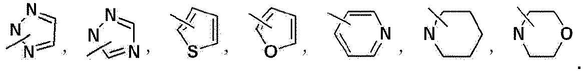

In one embodiment,
R₁ is H, a methyl group, an ethyl group, or a benzyl group;
R₂ is H or a methyl group;
n is an integer of 0 to 1;
X is -NR₃R₄ (wherein R₃ and R₄ are each independently H, a methyl group, or a C(O)methyl group);
Y is CH, CCI, CF, or N; and
Z is H, Br, Cl, a methyl group, a tert-butyl group, or a trifluoromethyl group.

Examples of the pyridine-based compound containing the isoxazoline ring represented by Formula 1 are as follows:
methyl 4-amino-3-chloro-6-(3-chloro-4,5-dihydroisoxazol-5-yl)picolinate;
4-amino-3-chloro-6-(3-chloro-4,5-dihydroisoxazol-5-yl)picolinic acid;
methyl 4-amino-3-chloro-6-(3-methyl-4,5-dihydroisoxazol-5-yl)picolinate;
4-amino-3-chloro-6-(3-methyl-4,5-dihydroisoxazol-5-yl)picolinic acid;
methyl 4-amino-3-chloro-6-(3-bromo-4,5-dihydroisoxazol-5-yl)picolinate;
4-amino-3-chloro-6-(3-bromo-4,5-dihydroisoxazol-5-yl)picolinic acid;
methyl 4-amino-3-chloro-6-(3-trifluoromethyl-4,5-dihydroisoxazol-5-yl)picolinate;
4-amino-3-chloro-6-(3-trifluoromethyl-4,5-dihydroisoxazol-5-yl)picolinic acid;
methyl 4-amino-3-chloro-6-(3-tert-butyl-4,5-dihydroisoxazol-5-yl)picolinate;
4-amino-3-chloro-6-(3-tert-butyl-4,5-dihydroisoxazol-5-yl)picolinic acid;
methyl 4-amino-3-chloro-6-(4,5-dihydroisoxazol-5-yl)picolinate;
methyl 4-amino-3-chloro-6-(3-bromo-5-methyl-4,5-dihydroisoxazol-5-yl)picolinate;
4-amino-3-chloro-6-(3-bromo-5-methyl-4,5-dihydroisoxazol-5-yl)picolinic acid;
methyl 4-amino-3-chloro-6-(3-chloro-5-methyl-4,5-dihydroisoxazol-5-yl)picolinate;
methyl 4-amino-6-((3-chloro-4,5-dihydroisoxazol-5-yl)methyl)-3-chloropicolinate;
4-amino-6-((3-chloro-4,5-dihydroisoxazol-5-yl)methyl)-3-chloropicolinic acid;
ethyl 4-amino-3-chloro-6-(3-chloro-4,5-dihydroisoxazol-5-yl)picolinate;
isopropyl 4-amino-3-chloro-6-(3-chloro-4,5-dihydroisoxazol-5-yl)picolinate;
isobutyl 4-amino-3-chloro-6-(3-chloro-4,5-dihydroisoxazol-5-yl)picolinate;
benzyl 4-amino-3-chloro-6-(3-chloro-4,5-dihydroisoxazol-5-yl)picolinate;
4-methylbenzyl 4-amino-3-chloro-6-(3-chloro-4,5-dihydroisoxazol-5-yl)picolinate;
4-trifluoromethylbenzyl 4-amino-3-chloro-6-(3-chloro-4,5-dihydroisoxazol-5-yl)picolinate;
4-methoxybenzyl 4-amino-3-chloro-6-(3-chloro-4,5-dihydroisoxazol-5-yl)picolinate;
4-bromobenzyl 4-amino-3-chloro-6-(3-chloro-4,5-dihydroisoxazol-5-yl)picolinate;
4-fluorobenzyl 4-amino-3-chloro-6-(3-chloro-4,5-dihydroisoxazol-5-yl)picolinate;
phenyl 4-amino-3-chloro-6-(3-chloro-4,5-dihydroisoxazol-5-yl)picolinate;
lithium 4-amino-3-chloro-6-(3-chloro-4,5-dihydroisoxazol-5-yl)picolinate;
sodium 4-amino-3-chloro-6-(3-chloro-4,5-dihydroisoxazol-5-yl)picolinate;
ethyl 4-amino-6-(3-bromo-4,5-dihydroisoxazol-5-yl)-3-chloropicolinate;
isopropyl 4-amino-6-(3-bromo-4,5-dihydroisoxazol-5-yl)-3-chloropicolinate;
isobutyl 4-amino-6-(3-bromo-4,5-dihydroisoxazol-5-yl)-3-chloropicolinate;
benzyl 4-amino-3-chloro-6-(3-bromo-4,5-dihydroisoxazol-5-yl)picolinate;
4-methylbenzyl 4-amino-6-(3-bromo-4,5-dihydroisoxazol-5-yl)-3-chloropicolinate;
4-trifluoromethylbenzyl 4-amino-6-(3-bromo-4,5-dihydroisoxazol-5-yl)-3-chloropicolinate;
4-methoxybenzyl 4-amino-6-(3-bromo-4,5-dihydroisoxazol-5-yl)-3-chloropicolinate;
4-bromobenzyl 4-amino-6-(3-bromo-4,5-dihydroisoxazol-5-yl)-3-chloropicolinate;
4-fluorobenzyl 4-amino-6-(3-bromo-4,5-dihydroisoxazol-5-yl)-3-chloropicolinate;
phenyl 4-amino-3-chloro-6-(3-bromo-4,5-dihydroisoxazol-5-yl)picolinate;
methyl 6-amino-5-chloro-2-(3-bromo-4,5-dihydroisoxazol-5-yl)pyrimidine-4-carboxylate;
6-amino-5-chloro-2-(3-bromo-4,5-dihydroisoxazol-5-yl)pyrimidine-4-carboxylic acid;
methyl 6-amino-5-chloro-2-(3-chloro-4,5-dihydroisoxazol-5-yl)pyrimidine-4-carboxylate;
6-amino-5-chloro-2-(3-chloro-4,5-dihydroisoxazol-5-yl)pyrimidine-4-carboxylic acid;
methyl 3-chloro-6-(3-chloro-4,5-dihydroisoxazol-5-yl)-4-(methylamino)picolinate;
methyl 3-chloro-6-(3-chloro-4,5-dihydroisoxazol-5-yl)-4-(dimethylamino)picolinate;
3-chloro-6-(3-chloro-4,5-dihydroisoxazol-5-yl)-4-(dimethylamino)picolinic acid;
methyl 4-amino-3,5-dichloro-6-(3-chloro-4,5-dihydroisoxazol-5-yl)picolinate;
4-amino-3,5-dichloro-6-(3-chloro-4,5-dihydroisoxazol-5-yl)picolinic acid;
methyl 4-amino-3-chloro-6-(3-chloro-4,5-dihydroisoxazol-5-yl)-5-fluoropicolinate;
4-amino-3-chloro-6-(3-chloro-4,5-dihydroisoxazol-5-yl)-5-fluoropicolinic acid; and
agrochemically acceptable salts thereof.

Examples of the agrochemically acceptable salts of the pyridine-based compound containing the isoxazoline ring represented by Formula 1 are a metal salt, a salt with an organic base, a salt with an inorganic acid, a salt with an organic acid, and a salt with a basic or acidic amino acid.

Examples of the metal salt are alkali metal salts such as sodium salts or potassium salts; alkaline earth metal salts such as calcium salts, magnesium salts, or barium salts; and aluminum salts. Examples of the salt with an organic base are a salt with trimethylamine, a salt with triethylamine, a salt with pyridine, a salt with picoline, a salt with 2,6-lutidine, a salt with ethanolamine, a salt with diethanolamine, a salt with triethanolamine, a salt with cyclohexylamine, a salt with dicyclohexylamine, and a salt with N,N-dibenzylethylenediamine. Examples of the salt with an inorganic acid are a salt with a hydrochloric acid, a salt with a hydrobromic acid, a salt with an nitric acid, a salt with a sulfuric acid, and a salt with a phosphoric acid. Examples of the salt with an organic acid are a salt with formic acid, a salt with acetic acid, a salt with trifluoroacetic acid, a salt with phthalic acid, a salt with fumaric acid, a salt with oxalic acid, a salt with tartaric acid, a salt with maleic acid, a salt with citric acid, a salt with succinic acid, a salt with methanesulfonic acid, a salt with benzenesulfonic acid, and a salt with p-toluenesulfonic acid. Examples of the salt with a basic amino acid are a salt with arginine, a salt with lysine, and a salt with ornithine. Examples of the salt with an acidic amino acid are a salt with aspartic acid and a salt with glutamic acid.

In addition, the present disclosure provides a herbicide including, as an active ingredient, the compound of Formula I, the agrochemically acceptable salt thereof, or a combination of the compound of Formula I and the agrochemically acceptable salt thereof.

The herbicide has selectivity with respect to cultivated crops, and may be used for a pre-emergence of weeds to remove weeds having broad leaves.

The herbicide has selectivity with respect to cultivated crops, and may be used for a post-emergence of weeds to remove weeds having broad leaves.

The cultivated crop may be maize, wheat, or rice, but is not limited thereto, and the weeds having broad leaves may be Quamoclit pennata, Abutilon theophrasti Medicus, Aeschynomene indica, Xanthium strumarium, or Catchweed bedstraw, but are not limited thereto.

The present disclosure provides a herbicidal composition including, as an active ingredient, a compound selected from a pyridine-based compound containing an isoxazoline ring represented by Formula 1 and an agrochemically acceptable salt thereof, or a combination of the pyridine-based compound containing an isoxazoline ring represented by Formula 1 and the agrochemically acceptable salt thereof, in an amount of about 0.1 wt% to about 99.9 wt%; and at least one additive selected from a surfactant, a solid diluent, and a liquid diluent, in an amount of about 0.1 wt% to about 99.9 wt%. 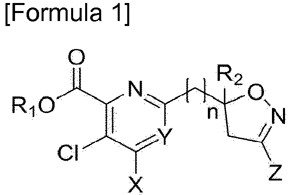 wherein, in Formula 1,
R₁ is H, a C₁ to C₄ alkyl group, or a benzyl group;
R₂ is H or a C₁ to C2 alkyl group;
n is an integer of 0 to 3;
X is a halogen or -NR₃R₄ (wherein R₃ and R₄ are each independently H, a C₁ to C₂ alkyl group, or a C(O)methyl group);
Y is CH, C-halogen, or N; and
Z is H, halogen, a C₁ to C₄ alkyl group, a C₁ to C₄ halo alkyl group, a C₁ to C₄ alkoxy group, a C₁ to C₄ alkoxy carbonyl group, a phenyl group substituted with at least one R₅ (wherein R₅ is halogen, a C₁ to C₄ alkyl group, a C₁ to C₄ alkoxy group, or a C₁ to C₄ halo alkyl group), or a heterocyclic group (wherein a heterocyclic group is a 5-membered or 6-membered saturated or unsaturated ring containing at least one selected from N, O, and S).

The herbicidal composition may be formulated in any one of wettable powder, suspensions, emulsions, fine suspensions, liquids, dispersible liquids, granular wettable powder, granules, powder, liquid wettable powder, floating granules, and tablets, but the formulation thereof is not limited thereto.

The herbicidal composition may further include, in addition to the active ingredient, at least one component selected from an acetyl-CoA carboxylase (ACCase) inhibitor; an acetolactate synthase (ALS) inhibitor; an auxin herbicide; an auxin transport inhibitor; a carotenoid biosynthesis inhibitor; an 5-enolpyruvylshikimate 3-phosphate synthase(ESPS) inhibitor; a glutamine synthetase inhibitor; a lipid biosynthesis inhibitor; a mitotic inhibitor; a protoporphyrinogen IX oxidase inhibitor; a photosynthesis inhibitor; a synergist; a growth material; a cell wall biosynthesis inhibitor; and any known herbicide.

The present disclosure provides a method of preparing the pyridine-based compound containing the isoxazoline ring represented by Formula 1 by reacting the compound represented by Formula 2 with the compound represented by Formula 3. 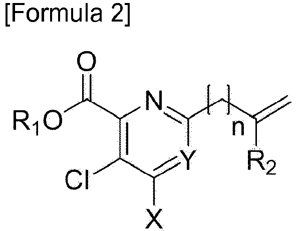 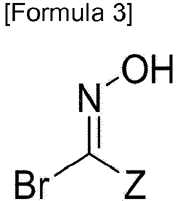 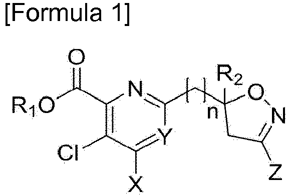 R₁, R₂, n, X, Y, and Z in Formulae 1, 2, and 3 are the same as described in Formula 1 above.

According to the method, as described in Reaction Scheme 1 illustrated below, the compound represented by Formula 2 in which the 6th position is substituted with a vinyl group or an aryl group is reacted with a bromo oxime compound represented by Formula 3 under a base condition to obtain the compound represented by Formula 1. 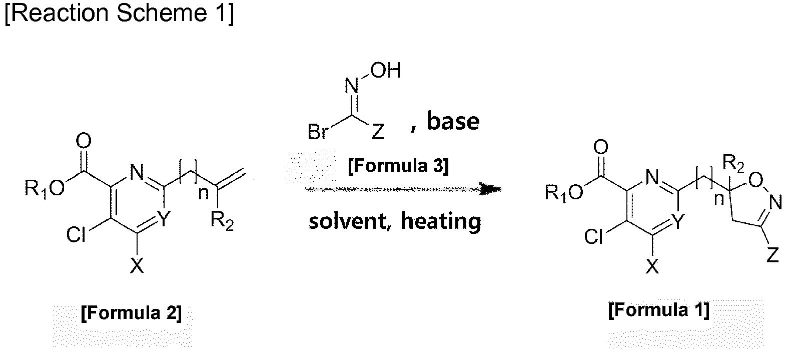 R₁, R₂, n, X, Y, and Z are the same as defined by Formula 1.

In Reaction Scheme 1, the base may be potassium hydrogen carbonate (KHCO₃), sodium hydrogen carbonate (NaHCO₃), potassium carbonate (K₂CO₃), sodium carbonate (Na₂CO₃), or triethylamine (Et₃N), and a solvent may be a mixture of water and an organic solvent or an organic solvent alone. In this regard, an available organic solvent herein may be ethyl acetate (EtOAc), dimethylformamide (DMF), methylene chloride (CH₂Cl₂), ethyl ether (Et₂O), tetrahydrofuran (THF), ethyl alcohol (EtOH), dichloroethane (ClCH₂CH₂Cl), or the like. The reaction temperature may be in the range of about 40 °C to about 80 °C. After the reaction was completed, the reactant was diluted by using an organic solvent, followed by washing with water, drying and concentrating, and purifying by column chromatography.

Hereinafter, embodiments of the present disclosure will be described in more detail with reference to Examples. These examples are provided herein for illustrative purpose only, and do not limit the scope of the present disclosure, which is obvious to one of ordinary skill in the art.

### [Example]

Compounds represented by Formula 1 synthesized according to Examples are shown in Table 1, and one of ordinary skill in the art may easily synthesize the compound shown in Table 2 by using known synthesis methods or modification thereof.

**Table 1. Examples of compound represented by Formula 1 according to the present disclosure**

| | | | | | | | |
|---|---|---|---|---|---|---|---|
| 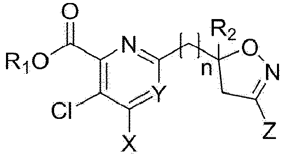 | | | | | | | |

| No | R₁ | R₂ | X | Y | Z | n | ¹H NMR |
|---|---|---|---|---|---|---|---|
| 1 | Me | H | NHAc | CH | Cl | 0 | ¹H NMR (300 MHz, CDCl₆) δ 8.77 (s, 1H), 7.95 (brs, 1H), 5.81 (t, *J =* 9.3 Hz, 1H), 4.02 (s, 3H), 3.61 (d, *J* =9.3 Hz, 2H), 2.33 (s, 3H) |
| 2 | Me | H | NHAc | CH | Me | 0 | ¹H NMR (300 MHz, CDCl₆) δ 8.73 (s, 1H), 7.93 (brs, 1H) 5.63 (dd, *J* = 10.1, 6.9 Hz, 1H), 4.01 (s, 3H), 3.42 (dd, *J* = 17.3, 10.1 Hz, 1H), 3.27 (dd, *J* = 17.3, 6.9 Hz, 1H), 2.31 (s, 3H), 2.03 (s, 3H) |
| 3 | Me | H | NHAc | CH | Br | 0 | ¹H NMR (300 MHz, CDCl₆) δ 8.74 (s, 1H), 7.96 (brs, 1H), 5.72 (t, *J* =9.3 Hz, 1H), 4.00 (s, 3H), 3.63 (d, *J*=9.3 Hz, 2H), 2.31 (s, 3H) |
| 4 | Me | H | NHAc | CH | CF₆ | 0 | ¹H NMR (300 MHz, CDCl₆) δ 8.73 (s, 1H), 7.95 (brs, 1H), 5.89 (dd, *J* = 11.6, 7.7 Hz, 1H), 4.00 (s, 3H), 3.60-3.67 (m, 2H), 2.32 (s, 3H) |
| 5 | Me | H | NHAc | CH | *t*-B | 0 | ¹H NMR (300 MHz, CDCl₆) δ 8.69 (s, 1H), 7.92 (brs, 1H), 5.61 (dd, *J* = 10.1, 6.6 Hz, 1H), 4.01 (s, 3H), 3.43 (dd, *J* = 16.9, 10.1 Hz, 1H), 3.27 (dd, *J* = 16.9, 6.6 Hz, 1H), 2.31 (s, 3H), 1.24 (s, 9H) |
| 6 | Me | H | NHAc | CH | H | 0 | ¹H NMR (300 MHz, CDCl₆) δ 8.67 (s, 1H), 7.98 (brs, 1H), 7.20 (s, 1H), 5.60 (dd, *J* = 11.1, 7.1 Hz, 1H), 3.99 (s, 3H), 3.42 (ddd, *J* = 17.3, 11.1, 1.7 Hz, 1H), 3.37 (ddd, 3H) 17.3, 7.1, 1.7 Hz, 1H), 2.30 (s, |
| 7 | Me | H | NHAc | CH | Ph | 0 | ¹H NMR (300 MHz, CDCl₆) δ 8.78 (s, 1H), 7.93 (brs, 1H), 7.68-7.71 (m, 2H), 7.39-7.41 (m, 3H) 5.81 (dd, *J*= 10.1, 7.3 Hz, 1H), 3.99 (s, 3H), 3.81 (dd, *J* = 16.9, 10.1 Hz, 1H), 3.70 (dd, *J* = 16.9, 7.3 Hz, 1H), 2.30 (s, 3H) |
| 8 | Me | H | NHAc | CH | 2-F-Ph | 0 | ¹H NMR (300 MHz, CDCl₆) & 8.78 (s, 1H), 7.93 (brs, 1H), 7.83-7.92 (m, 1 H), 7.32-7.46 (m, 1 H), 7.04 - 7.23 (m, 2H), 5.82 (dd, *J* = 11.1, 7.3 Hz, 1 H), 4.01 (s, 3H), 3.92 (ddd, *J*=17.5, 11.1, 2.6 Hz, 1H), 3.76 (ddd, *J*=17.5, 7.3, 2.6 Hz, 1H) |
| 9 | Me | H | NHAc | CH | 2-F, 3-OMe, 4-Cl-P h | 0 | ¹H NMR (300 MHz, CDCl₆) δ 8.76 (s, 1H), 7.93 (s, 1H), 7.53 (dd, *J* = 8.7, 7.2 Hz, 1H), 7.18 (dd, *J*=8.7, 1.9 Hz, 1H), 5.82 (dd, *J* =11.3, 7.5 Hz, 1H), 4.00 (s, 3H), 3.96 (s, 3H), 3.88 (ddd, *J*=17.8, 11.3, 2.4 Hz, 1H), 3.74 (ddd, *J*=17.8, 7.5, 2.4 Hz, 1H), 2.30 (s, 3 H) |
| 10 | Me | Me | NHAc | CH | Br | 0 | ¹H ¹H NMR (500 MHz, CDCl₆) δ 8.83 (s, 1H), 7.93 (brs, 1H), 3.99 (s, 3H), 3.87 (d, *J* = 17.4 Hz, 1H), 3.25 (d, J = 17.4 Hz, 1H), 2.30 (s, 3H), 1.78 (s, 3H) |
| 11 | Me | Me | NHAc | CH | Cl | 0 | ¹H NMR (300 MHz, CDCl₆) δ 8.82 (s, 1H), 7.98 (brs, 1H), 3.99 (s, 3H), 3.83 (d, *J* = 17.3 Hz, 1H), 3.20 (d, *J* = 17.3 Hz, 1H), 2.31 (s, 3H), 1.78 (s, 3H) |
| 12 | Me | H | NHAc | CH | Br | 1 | ¹H NMR (500 MHz, CDCl₆) δ 8.48 (s, 1H), 7.93 (brs, 1H), 5.10-5.19 (m, 1H), 4.00 (s, 3H), 3.29-3.35 (m, 1H), 3.23-3.27 (m, 1H), 3.08-3.14 (m, 2H), 2.30 (s, 3H) |
| 13 | Me | H | NHAc | CH | Cl | 1 | ¹H NMR (300 MHz, CDCl₆) δ 8.48 (s, 1H), 7.95 (brs, 1H), 5.13-5.28 (m, 1H), 3.17-3.36 (m, 2H), 2.99-3.17 (m, 2H), 2.30 (s, 3H) |
| 14 | Me | H | NHAc | CH | 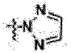 | 0 | |
| 15 | Me | H | NHAc | CH | 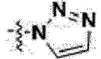 | 0 | |
| 16 | Me | H | NHAc | CH | OMe | 0 | |
| 17 | Me | H | NHAc | CH | SCN | 0 | |
| 18 | Me | H | NHAc | CH | 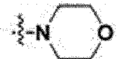 | 0 | |
| | Me | H | NHAc | I CCl | 19 Br | 0 | ¹H NMR (300 MHz, CDCl₆) δ 7.58 (brs, 1H), 6.10 (dd, *J* = 11.0, 8.0 Hz, 1H), 4.10 (dd, *J* = 17.3, 8.0 Hz, 1H), 3.99 (s, 3H), 3.48 (dd, *J* = 17.3, 11.0 Hz, 1H), 2.28 (s, 3H) |
| 20 | Me | H | NHAc | CCl | Cl | 0 | ¹H NMR (300 MHz, CDCl₆) δ 7.62 (s, 1H), 6.17 (dd, *J* = 11.0, 8.0 Hz, 1H), 4.07 (dd, *J* = 17.3, 8.0 Hz, 1H), 3.99 (s, 3H), 3.43 (dd, J = 17.3, 11.0 Hz, 1H), 2.28 (s, 3H) |
| 21 | Me | H | NHAc | CH | 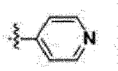 | 0 | |
| 22 | Me | H | NHAc | CH | 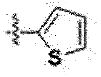 | 0 | ¹H NMR (300 MHz, CDCl₆) δ 8.79 (s, 1H), 7.95 (s, 1H), 7.41 (dd, *J* = 5.1, 1.0 Hz, 1H), 7.24 (dd, *J* = 3.6, 1.0 Hz, 1H), 7.07 (dd, J = 5.1, 3.7 Hz, 1H), 5.82 (dd, *J* = 10.7, 7.5 Hz, 1H), 4.02 (s, 3H), 3.79 (qd, *J* = 16.7, 9.1 Hz, 2H), 2.31 (s, 3H) |
| 23 | Et | H | NHAc | CH | OEt | 0 | |
| 24 | Me | H | NHAc | CH | 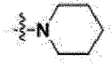 | 0 | |
| 25 | Me | H | NHAc | CH | 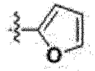 | 0 | |
| 26 | Me | H | NHAc | CH | 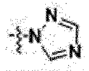 | 0 | |
| 27 | Me | H | NHAc | CH | C(O)OMe | 0 | |
| 28 | Et | H | NHAc | CH | Br | 0 | ¹H NMR (300 MHz, CDCl₆) δ 8.68 (s, 1H), 8.04 (brs, 1H), 5.71 (t, *J* = 9.2 Hz, 1 H), 4.47 (g, *J* = 7.1 Hz, 2 H), 3.64 (d, *J* = 9.2 Hz, 2 H), 2.31 (s, 3 H), 1.43 (t, *J* = 7.1 Hz, 3 H) |
| 29 | Bn | H | NHAc | CH | Br | 0 | ¹H NMR (300 MHz, CDCl₆) δ 8.71 (s, 1H), 7.91 (brs, 1H), 7.30-7.52 (m, 5H), 5.64-5.79 (m, 1H), 5.44 (s, 2H), 3.53-373 (m, 2H), 2.29 (s, 3 H) |
| 30 | Me | H | NHAc | CF | Br | 0 | ¹H NMR (300 MHz, CDCl₆) δ 7.40 (s, 1H), 6.00 (ddd, *J* = 11.1, 8.3, 1.2 Hz, 1H), 3.95-4.06 (m, 4H), 3.53 (dd, *J* = 17.2, 11.1 Hz, 1H), 2.31 (s, 3H) |
| 31 | Me | H | NHAc | CF | Cl | 0 | ¹H NMR (300 MHz, CDCl₆) δ 7.43 (s, 1H), 6.05 (ddd, *J* = 11.1, 8.4, 1.4 Hz, 1H), 3.92-4.02 (m, 4H), 3.41-3.53 (m, 1H), 2.28-2.30 (s, 3H) |
| 32 | Bn | H | NHAc | CF | Br | 0 | ¹H NMR (300 MHz, CDCl₆) δ 7.33-7.46 (m, 6H), 5.97 (ddd, *J* = 11.1, 7.8, 1.2 Hz, 1H), 5.42 (s, 2H), 4.00 (dd, *J* = 17.2, 7.8 Hz, 1H), 3.50 (dd, *J* = 17.2, 11.1 Hz, 1H), 2.27 (s, 3H) |
| 33 | Me | H | NH₂ | CH | Br | 0 | ¹H NMR (300 MHz, CDCl₆) δ 6.97 (s, 1H), 5.64 (dd, *J* = 11.1, 7.0 Hz, 1H), 4.90 (brs, 2H), 3.99 (s, 3H), 3.65 (dd, *J* = 17.5, 11.1 Hz, 1H), 3.43 (dd, *J* = 17.5, 7.0 Hz, 1H) |
| 34 | H | H | NHz | CH | Br | 0 | ¹H NMR (500 MHz, CD₈OD) δ 6.94 (s, 1H), 5.66 (dd, *J* = 10.1, 7.6 Hz, 1H), 3.75 (dd, *J* = 17.4, 10.1 Hz, 1H), 3.43 (dd, *J* = 17.4, 7.6 Hz, 1H) |
| 35 | Me | H | NH₂ | CH | Me | 0 | ¹H NMR (300 MHz, CDCl₆) δ 7.00 (s, 1H), 5.58 (dd, *J* = 11.1, 5.6 Hz, 1H), 5.32 (brs, 2H), 3.99 (s, 3H), 3.43 (dd, *J* = 17.4, 11.1 Hz, 1H), 3.17 (dd, *J* = 17.4, 5.6 Hz, 1H), 2.01 (s, 3H) |
| 36 | H | H | NH₂ | CH | Me | 0 | ¹H NMR (300 MHz, CD₆OD) δ 6.95 (s, 1H), (dd, *J* = 11.1, 6.1 Hz, 1H), 3.56 (dd, *J* = 17.6, 11.1 Hz, 1H), 3.14 (dd, *J* = 17.6, 6.1 Hz, 1H), 2.02 (s, 3H) |
| 37 | Me | H | NH₂ | CH | Cl | 0 | ¹H NMR (300 MHz, CDCl₆) δ 6.98 (s, 1H), 5.73 (dd, *J* = 11.1, 7.0 Hz, 1H), 5.09 (brs, 2H), 3.98 (s, 3H), 3.64 (dd, *J* = 17.5, 11.1 Hz, 1H), 3.44 (dd, *J* = 17.5, 7.0 Hz, 1H) |
| 38 | H | H | NH₂ | CH | Cl | 0 | ¹H NMR (500 MHz, CD₆OD) δ 7.08 (s, 1H), 5.74 (dd, *J* = 10.1, 7.6 Hz, 1H), 3.74 (dd, *J* = 17.4, 10.1 Hz, 1H), 3.62 (dd, *J* = 17.4, 7.6 Hz, 1H) |
| 39 | Me | H | NHz | CH | CF₆ | 0 | ¹H NMR (300 MHz, CDCl₆) δ 6.92 (s, 1H), 5.87 (dd, *J* = 11.1, 6.8 Hz, 1H), 4.92 (brs, 2H), 4.00 (s, 3H), 3.52-3.63 (m, 2H) 2.01 (s, 3H) |
| 40 | H | H | NH₂ | CH | CF₆ | 0 | NMR (300 MHz, CD₆OD) S 6.92 (s, 1H), 5.84 (dd, *J* = 11.6, 7.7 Hz, 1H), 3.76 (dd, *J* =17.8, 11.6 Hz, 1H), 3.48 (dd, *J* = 17.8, 7.7 Hz, 1H) |
| 41 | Me | H | NH₂ | CH | *t*-Bu | 0 | ¹H NMR (300 MHz, CDCl₆) δ 6.98 (s, 1H) 5.54 (dd, *J* = 10.1, 5.9 Hz, 1H), 4.85 (brs, 2H), 3.98 (s, 3H), 3.45 (dd, J = 17.0, 10.1 Hz, 1H), 3.17 (dd, *J* = 17.0, 5.9 Hz, 1H), 1.18 (s, 9H) |
| 42 | H | H | NH₂ | CH | *t*-Bu | 0 | ¹H NMR (300 MHz, CD₈OD) δ 6.90 (s, 1H), 5.57 (dd, *J* = 11.1, 6.2 Hz, 1H), 3.58 (dd, *J* = 17.4, 11.1 Hz, 1H), 3.18 (dd, *J* = 17.4, 6.2 Hz, 1H), 1.20 (s, 9H) |
| 43 | Me | H | NH₂ | CH | H | 0 | ¹H NMR (300 MHz CD₈OD) δ 7.31 (s, 1H), 6.91 (s, 1H), 5.40 (dd, *J* = 11.3, 6.8 Hz, 1H), 3.94 (s, 3H), 3.44 (ddd, *J* = 17.4, 11.4, 1.6 Hz, 1H), 3.19 (ddd, *J* = 17.4, 6.8, 1.6 Hz, 1H) |
| 44 | H | H | NH₂ | CH | H | 0 | |
| 45 | Me | H | NH₂ | CH | Ph | 0 | ¹H NMR (300 MHz, CDCl₈) δ 7.68-7.71 (m, 2H), 7.39-7.43 (m, 3H), 7.05 (s, 1H), 5.78 (dd, *J* = 11.2, 5.9 Hz, 1H), 4.84 (brs, 2H), 4.01 (s, 3H), 3.85 (dd, *J* = 17.0, 11.2 Hz, 1H), 3.62 (dd, *J* = 17.0, 5.9 Hz, 1H) |
| 46 | H | H | NH₂ | CH | Ph | 0 | ¹H NMR (300 MHz, DMSO-d₆) δ 13.43 (brs, 1H), 7.71-7.73 (m, 2H), 7.43-7.73 (m, 3H), 6.89 (s, 1 H), 6.73 (brs, 2H), 5.59 (m, 1H), 3.85 (m, 1H), 3.37 (m, 1H) |
| 47 | Me | H | NH₂ | CH | 2-F-Ph | 0 | ¹H NMR (300 MHz, CDCl₈) δ 7.79-7.85 (m, 1H), 7.35-7.45 (m, 1H), 7 .0 7-7.22 (m, 2H), 7 .05 (s, 1H), 5.78 (dd, *J* = 11.3, 6.0 Hz, 1H), 4.90 (brs, 2H), 4.00 (s, 3H), 3.93 (ddd, *J* = 17.3, 11.3, 2.6 Hz, 1H), 3.68 (ddd, *J* = 17.3, 6.0, 2.6 Hz, 1H) |
| 48 | H | H | NHz | CH | 2-F-Ph | 0 | ¹H NMR (300 MHz, DMSO-d₆) δ 7.74-7.79 (m, 1H), 7.45-7.59 (m, 1H), 7.24-7.40 (m, 2H), 6.89 (s, 1H), 6.78 (brs, 2H), 5.62 (dd, *J* = 11.1, 6.0 Hz, 1H), 3.87 (dd, *J* = 17.1, 11.5 Hz, 1H), 3.59 (dd, *J* = 17.1, 6.0 Hz, 1H) |
| 49 | Me | H | NHz | CH | 2-F, **3-OMe, 4-Cl-Ph** | 0 | ¹H NMR (300 MHz, CDCl₈) δ 7.45 (dd, *J* = 8.7, 7.2 Hz, 1H), 7 .17 (dd. J= 8.7, 1.5 Hz, 1H), 7,01 (s, 1H), 5.76 (dd, *J* = 11.3, 6.0 Hz, 1H), 4.87 (brs, 2H), 3.99 (s, 3H), 3.96 (s, 3H), 3.88 (ddd, *J* = 17.7, 11.3, 2.3 Hz, 1H), 3.66 (ddd, *J* = 17.7, 6.0, 2.3 Hz, 1H) |
| 50 | H | H | NH₂ | CH | 2-F, 3-OMe, 4-Cl-Ph | 0 | ¹H NMR (300 MHz CD₈OD) δ 7.49 (dd, *J* = 8.7, 7.0 Hz, 1H), 7.29 (dd, *J =* 8.7, 1.5 Hz, 1H), 6.98 (s, 1H), 5.78 (dd, J = 11.3, 6.8 Hz, 1H), 3.98 (ddd, *J* = 17.6, 11.3, 2.1 Hz, 1H), 3.63 (ddd, *J* = 17.6, 6.8, 2.1 Hz, 1H) |
| 51 | Me | Me | NHz | CH | Cl | 0 | ¹H NMR (500 MHz, CDCl₈) δ 7 .09 1H ), 4.09 (brs, 2H), 3.98 (s, 3H) 3.72 (d, *J* = 17.4 Hz, 1H), 3.22 (d, *J* = 17.4 Hz, 1H), 1.75 (s, 3H) |
| 52 | H | Me | NH₂ | CH | Cl | 0 | ¹H NMR (500 MHz, CD₆OD) δ 7.05 (s, 1H), 3.64 (d, *J =* 17.4 Hz, 1H), 3.39 (d, *J* = 17.4 Hz, 1H), 1.74 (s, 3H) |
| 53 | Me | Me | NH₂ | CH | Br | 0 | ¹H NMR (500 MHz, CDCl₆) δ 7.10 (s, 1H), 5.05 (brs, 2H), 3.98 (s, 3H), 3.77 (d, *J =* 17.4 Hz, 1H), 3.26 (d, *J =* 17.4 Hz, 1H), 1.75 (s, 3H) |
| 54 | H | Me | NH₂ | CH | Br | 0 | ¹H NMR (500 MHz, CH₆OD) δ 7.05 (s, 1H), 3.87 (d, *J* = 17.4 Hz, 1H), 3.43 (d, *J =* 17.4 Hz, 1H), 1.75 (s, 3H) |
| 55 | Me | H | NH₂ | CH | Cl | 1 | ¹H NMR (300 MHz, CDCl₆) δ 6.69 (s, 1H), 5.14 (m, 1H), 4.77 (brs, 2H), 3.99 (s, 3H), 3.20-3.32 (m, 1H), 2.95-3.15 (m, 3H) |
| 56 | H | H | NH₂ | CH | Cl | 1 | ¹H NMR (300 MHz, CD₈OD) δ 6.39 (s, 1H), 5.00-5.18 (m, 1H), 3:47 (m, 1H), 2.98-3.19 (m, 3H) |
| 57 | Me | H | NH₂ | CH | 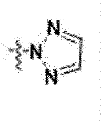 | 0 | ¹H NMR (300 MHz, CDCl₈) δ 7.88 (s, 2H), 7.11 (s, 1H), 5.92 (dd, *J* = 11.0, 6.2 Hz, 1H), 4.94 (s, 2H), 4.21-4.09 (m, 1H), 4.05-3.93 (m, 4H) |
| 58 | H | H | NH₂ | CH | 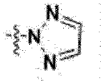 | 0 | |
| 59 | Me | H | NH₂ | CH | 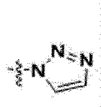 | 0 | ¹H NMR (300 MHz, CDCl₈) δ 8.26 (d, *J* = 1.2 Hz, 1H), 7.81 (d, *J* = 1.2 Hz, 1H), 7.01 (s, 1H), 5.90(t, *J* = 8.9 Hz, 1H), 5.02 (s, 2H), 4.21 - 4.12 (m, 2H), 3.99 (s, 3H) |
| 60 | H | H | NH₂ | CH | 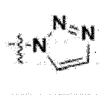 | 0 | |
| 61 | Me | H | NH₂ | CH | OMe | 0 | ¹H NMR (300 MHz, CDCl₈) δ 6.99 (d, *J* = 0.4 Hz, 1H), 5.68 (dd, *J* = 11.0, 6.3 Hz, 1H), 4.97 (s, 2H), 4.00 (s, 3H), 3.71 (dd, *J* = 17.6, 11.2 Hz, 1H), 3.49 (dd, *J* = 17.6, 6.4 Hz, 1H) |
| 62 | H | H | NH₂ | CH | OMe | 0 | |
| 63 | Me | H | NH₂ | CH | SCN | 0 | |
| 64 | H | H | NH₂ | CH | SCN | 0 | |
| 65 | Me | H | NH₂ | CH | 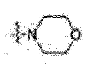 | 0 | ¹H NMR (300 MHz, CDCl₈) δ 7.10 (s, 1H), 5.54 (dd, *J* = 10.2, 5.0 Hz, 1H), 4.89 (s, 2H), 3.98 (s, 3H), 3.74-3.66 (m, 4H), 3.47 (dd, *J* = 15.8, 10.2 Hz, 1H), 3.31-3.23 (m, 1H), 3.23-3.15 (m, 4H) |
| 66 | H | H | NH₂ | CH | 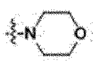 | 0 | ¹H NMR (300 MHz, DMSO-d₆) δ 6.90 (s, 1H), 6.73 (s, 2H), 5.32 (dd, J= 10.2, 6.5 Hz, 1H), 3.66-3.53 (m, 4H), 3.45 (dd, *J*= 16.1, 10.3 Hz, 2H), 3.14-3.07 (m, 4H) |
| 67 | Me | H | NH₂ | CCl | Cl | 0 | ¹H NMR (300 MHz, CDCl₆) δ 6.02 (dd, *J* = 8.5, 11.0 Hz, 1H), 5.37 (brs, 2H), 4.12 (dd, *J* = 8.5, 17.3 Hz, 1H), 3.97 (s, 3H), 3.41 (dd, *J* = 11.00, 17.3 Hz, 1H) |
| 68 | H | H | NH₂ | CCl | Cl | 0 | ¹H NMR (300 MHz, CD₈OD) δ 6.08 (dd, *J* = 11.0, 8.2 Hz, 1H), 4.00 (dd, *J* = 17.3, 8.2 Hz, 1H), 3.54 (dd, J = 17.3, 11.0 Hz, 1H) |
| 69 | Me | H | NH₂ | CH | 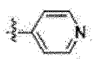 | 0 | |
| 70 | H | H | NHz | CH | 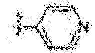 | 0 | ¹H NMR (300 MHz, DMSO-d₆) δ 8.67 (d. *J* = 6.0 Hz, 2H), 7.65 (d, *J* = 6.0 Hz, 2H), 6.63 (s, 1H), 6.17 (s, 2H), 5.61 (dd, *J* = 11.2, 7.8 Hz, 1H), 3.77 (dd, *J* = 17.2, 11.3 Hz, 1H), 3.58 (dd, *J* = 17.3, 7.7 Hz, 1H) |
| 71 | Me | H | NHz | CH | 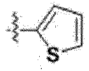 | 0 | ¹H NMR (300 MHz, CDCl₈) δ 7.42 (dd, *J =* 5.1, 1.0 Hz, 1H), 7.28 (s, 1H), 7.22 (dd, J = 3.6, 1.0 Hz, 1H), 7.07 (dd, *J =* 5.5, 3.2 Hz, 2H), 5.78 (dd, *J* = 11.1. 5.8 Hz, 1H), 4.93 (s, 2H), 4.01 (s, 3H), 3.87 (dd, *J=* 16.8, 11.1 Hz, 1H), 3.62 (dd, *J* = 16.8, 5.8 Hz, 1H) |
| 72 | H | H | NHz | CH | 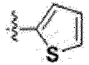 | 0 | ¹H NMR (300 MHz, DMSO-d₆) δ 7.76-7.68 (m, 1H), 7.49-7.42 (m, 1H), 7.16 (dd, *J* = 5.1, 3.7 Hz, 1H), 6.88 (s, 1H), 6.75 (s, 2H), 5.62 (dd, *J* = 11.1, 6.9 Hz, 1H), 3.85 (dd, *J* = 17.1, 11.1 Hz, 2H), 3.58 (dd, *J* = 17.0, 6.9 Hz, 2H) |
| 73 | Et | H | NH₂ | CH | Br | 0 | ¹H NMR (300 MHz, CDCl₆) δ 6.96 (s, 1H), 5.62 (dd, *J* = 11.1, 6.5 Hz, 1H), 5.00 (brs, 2H), 4.42 (q, *J* = 7.2 Hz, 2H), 3.63 (dd, *J*= 17.5, 11.1 Hz, 1H), 3.45 (dd, *J*= 17.4, 6.5 Hz, 1H), 1.42 (t, *J* = 7.2 Hz, 3H) |
| 74 | Et | H | NHz | CH | Cl | 0 | ¹H NMR (300 MHz, CDCl₆) δ 6.96 (d, *J*= 0.5 Hz, 1H), 5.71-5.57 (m. 1H), 5.01 (s, -2H), 4.46 (q, *J*=7.1 Hz, 2H), 3.60 (ddd, *J*=24.1, 17.6, 8.8 Hz, 2H), 1.43 (t, *J* = 7.1 Hz, 3H) |
| 75 | Et | H | NH₂ | CH | OEt | 0 | ¹H NMR (300 MHz, CDCl₈) δ 7.10-7.04 (m, 1H), 5.58 (dd, *J* = 10.3, 5.9 Hz, 1H), 4.98 (s, 2H), 4.46 (q, *J* = 7.1 Hz, 2H), 4.18 (qd, *J* = 7.1, 3.8 Hz, 2H), 3.34 (ddd, *J* = 22.5, 16.6, 8.2 Hz, 2H), 1.42 (t, *J* = 7.1 Hz, 3H), 1.34 (t, *J* = 7.1 Hz, 3H) |
| 76 | Me | H | NH₂ | CH | 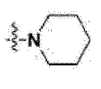 | 0 | ¹H NMR (500 MHz, CDCl₆) δ 7.19 (s, 1H), 5.51 (dd, *J* = 10.2, 5.1 H z, 1H), 5.22 (s, 2H), 3.97 (s, 3H), 3.56-3.41 (m, 1H), 3.24 (dd, *J* = 15.7, 5.1 Hz, 1H), 3.16 (s, 4H), 1.56 (s, 6H) |
| 77 | H | H | NH₂ | CH | 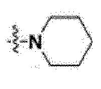 ¹H | 0 | NMR (300 MHz, DMSO-d₆) δ 7.02 (s, 1H), 5.52 (dd, *J* = 10.2, 5.8 Hz, 2H), 3.55 (dd, *J* = 16.2, 10.2 Hz, 1H), 3.26-3.17 (m, 1H), 3.13 (s, 4H), 1.51 (s, 6H) |
| 78 | Me | H | NH₂ | CH | 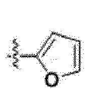 | 0 | ¹H NMR (300 MHz, CDCl₈) δ 7.54 (d, *J* = 1.2 Hz, 1H), 7.03 (s, 1H), 673 (d, *J* = 3.5 Hz, 1H), 6.50 (dd, *J* = 3.4, 1.8 Hz, 1H), 535 (dd, *J* = 11.1, 5.7 Hz, 1H), 4.85 (s, 2H), 4.01 (s, 3H), 3.81 (dd, *J* = 16.9, 11.1 Hz, 1H), 3.56 (dd, *J* = 16.9, 5.6 Hz, 1H) |
| 79 | H | H | NH₂ | CH | 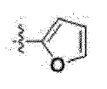 | 0 | ¹H NMR (300 MHz, DMSO-d₆) δ 7.87 (d, *J* = 1.2 Hz, 1H), 6.96 (d, *J*= 3.3 Hz, 1H), 6.86 (s, 1H), 6.75 (s, 2H), 6.65 (dd, *J*=3.4, 1.8 Hz, 1H), 5.58 (dd, *J* = 11.1, 6.7 Hz, 1H), 3.76 (dd, *J =* 17.0, 11.1 Hz, 1H), 3.49 (dd, *J* = 17.0, 6.7 Hz, 1H) |
| 80 | Me | H | NH₂ | CH | 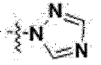 | 0 | |
| 81 | H | H | NH₂ | CH | 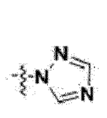 | 0 | ¹H NMR (300 MHz, DMSO-d₆) δ 9.18 (s, 1H), 8.32 (s, 1H), 6.93 (s, 1H), 6.85 (s, 2H), 5.80 (dd, *J* = 11.0, 7.5 Hz, 1H), 4.14-3.74 (m, 2H) |
| 82 | Me | H | NH₂ | CH | C(O)OMe | 0 | ¹H NMR (300 MHz, CDCl₆) S 6.96-6.92 (m, 1H), 5.80 (dd, *J* = 11.6, 6.9 Hz, 1H), 5.03 (s, 2H), 3.98 (s, 3H), 3.89 (s, 3H), 3.59 (ddd, *J* = 25.0, 18.1, 9.3 Hz, 2H) |
| 83 | *iso*-Pr | H | NH₂ | CH | Br | 0 | ¹H NMR (300 MHz, CDCl₆) δ 6.94 (s, 1H), 5.65 (dd, *J* = 11.1, 6.5 Hz, 1H), 5.32 (m, 1H), 5.00 (brs, 2H), 3.67 (dd, *J* = 17.5, 11.1 Hz, 1H), 3.51 (dd, *J* = 17.4, 6.5 Hz, 1H), 1.41 (d, *J* = 6.3 Hz, 6H) |
| 84 | *iso*-Bu | H | NH₂ | CH | Br | 0 | ¹H NMR (500 MHz, CDCl₆) δ 6.95 (s, 1H), 5.65 (dd, *J* = 11.1, 6.4 Hz, 1H), 4.95 (brs, 2H), 4.18 (d, *J* = 6.9 Hz, 2H), 3.67 (dd, *J* = 17.5, 11.1 Hz, 1H), 3.52 (dd, *J* = 17.4, 6.4 Hz, 1H), 2.09 (m, 1H), 1.02 (d, *J* = 6.4 Hz, 6H) |
| 85 | Ph | H | NH₂ | CH | Br | 0 | ¹H NMR (500 MHz, CDCl₆) δ 7.44 (m, 2H), 7.29 (m, 3H), 7.02 (s, 1H), 5.77 (dd, *J* = 11.2, 6.4 Hz, 1H), 5.00 (brs, 2H), 3.66 (dd, *J* = 17.6, 11.2 Hz, 1H), 3.53 (dd, *J* = 17.6, 6.4 Hz, 1H) |
| 86 | Bn | H | NH₂ | CH | Br | 0 | ¹H NMR (500 MHz, CDCl₆) δ 7.4- 7.46 (m, 2H), 7.32-7.39 (m, 3H), 6.93 (s, 1H), 5.61 (dd, *J* = 11.1, 6.5 Hz, 1H=, 5.42 (s, 2H), 4.93 (brs, 2H), 3.62 (dd, *J* = 17.5, 11.1 Hz, 1H), 3.47 (dd, *J* = 17.5, 6.5 Hz, 1H) |
| 87 | (4-Me-P h)CH₂ | H | NH₂ | CH | Br | 0 | ¹H NMR (500 MHz, CDCl₆) δ 7.35 (d, *J* = 8.0 Hz, 2H), 7.18 (d, *J* = 8.0 Hz, 2H), 6.92 (s, 1H), 5.63 (dd, *J* = 11.1, 6.5 Hz, 1H), 5.38 (s, 2H), 4.88 (brs, 2H), 3.65 (dd, *J* = 17.5, 11.1 Hz, 1H), 3.48 (dd, *J* = 17.5, 6.5 Hz, 1H), 2.35 (s, 3H) |
| 88 | (4-CF₈-Ph)CH₂ | H | NH₂ | CH | Br | 0 | ¹H NMR (500 MHz, CDCl₆) δ 7.66 (d, *J* = 8.3 Hz, 2H), 7.59 (d, *J* = 8.3 Hz, 2H), 6.97 (s, 1H), 5.66 (dd, *J* = 11.1, 6.5 Hz, 1H), 5.48 (s, 2H), 4.88 (brs, 2H), 3.69 (dd, *J* = 17.5, 11.1 Hz, 1H), 3.50 (dd, *J* = 17.5, 6.5 Hz, 1H) |
| 89 | (4-OMe-Ph)CH₂ | H | NH₂ | CH | Br | 0 | ¹H NMR (500 MHz, CDCl₆) δ 7.39 (d, *J* = 8.3 Hz, 2H), 6.91 (d, *J* = 8.3 Hz, 2H), 6.89 (s, 1H), 5.62 (dd, *J* = 11.1, 6.5 Hz, 1H), 5.35 (s, 2H), 4.90 (brs, 2H), 3.81(s, 3H), 3.65 (dd, *J* = 17.5, 11.1 Hz, 1H), 3.48 (dd, *J* = 17.5, 6.5 Hz, 1H) |
| 90 | (4-F-Ph) CH₂ | H | NH₂ | CH | Br | 0 | ¹H NMR (500 MHz, CDCl₆) S 7.44 (m, 2H), 7.07 (m, 2H), 6.94 (s, 1 H), 5.63 (dd, *J =* 11.2, 6.4 Hz, 1H), 5.38 (s, 2H), 4.90 (brs, 2H), 3.66 (dd, *J* = 17.4, 11.2 Hz, 1H), 3.49 (dd, *J* = 17.4, 6.4 Hz, 1H) |
| 91 | (4-Br-Ph)CH₂ | H | NH₂ | CH | Br | 0 | ¹H NMR (500 MHz, CDCl₆) δ 7.52 (d, *J* = 8.3 Hz, 2H), 7.34 (d, *J* = 8.3 Hz, 2H), 6.96 (s, 1H), 5.65 (dd, *J* = 11.2, 6.4 Hz, 1H), 5.37 (s, 2H), 4.91 (brs, 2H), 3.67 (dd, *J* = 17.4, 11.2 Hz, 1H), 3.50 (dd, *J* = 17.4, 6.4 Hz, 1H) |
| 92 | *iso*-Pr | H | NH₂ | CH | Cl | 0 | ¹H NMR (300 MHz, CDCl₆) δ 6.93 (s, 1H), 5.71 (dd, *J* = 11.1, 6.5 Hz, 1H), 5.32 (m, 1H), 4.97 (brs, 2H), 3.63 (dd, *J* = 17.5, 11.1 Hz, 1H), 3.47 (dd, *J* = 17.4, 6.5 Hz, 1H), 1.41 (d, *J* = 6.4 Hz, 6H) |
| 93 | *iso*-Bu | H | NH₂ | CH | Cl | 0 | ¹H NMR (300 MHz, CDCl₆) δ 6.96 (s, 1H), 5.72 (dd, *J* = 11.1, 6.4 Hz, 1H), 5.02 (brs, 2H), 4.18 (d, *J* = 6.8 Hz, 2H), 3.62 (dd, *J* = 17.5, 11.1 Hz, 1H), 3.48 (dd, *J* = 17.4, 6.4 Hz, 1H), 2.10 (m, 1H), 1.02 (d, *J* = 6.8 Hz, 6H) |
| 94 | Ph | H | NH₂ | CH | Cl | 0 | ¹H NMR (500 MHz, CDCl₆) δ 7.44 (m, 2H), 7.29 (m, 3H), 7.02 (s, 1H), 5.77 (dd, *J* = 11.2, 6.4 Hz, 1H), 5.00 (brs, 2H), 3.66 (dd, *J* = 17.6, 11.2 Hz, 1H), 3.53 (dd, *J* = 17.6, 6.4 Hz, 1H) |
| 95 | Bn | H | NH₂ | CH | Cl | 0 | ¹H NMR (300 MHz, CDCl₆) δ 7.42-7.51 (m, 2H), 7.30-7.42 (m, 3H), 6.96 (s, 1H), 5.72 (dd, *J* = 11.1, 6.5 Hz, 1H), 5.42 (s, 2H), 4.98 (brs, 2H), 3.63 (dd, *J* = 17.5, 11.1 Hz, 3.45 (dd, *J* = 17.5, - 6.5 Hz), 1H) |
| 96 | (4-Me-P h)CH₂ | H | NH₂ | CH | Cl | 0 | ¹H NMR (300 MHz, CDCl₆) δ 7.34 (d, *J* = 7.9 Hz, 2H), 7.17 (d, *J* = 7.9 Hz, 2H), 6.91 (s, 1H), 5.68 (dd, *J* = 11.0, 6.5 Hz, 1H), 5.37 (s, 2H), 4.96 (brs, 2H), 3.59 (dd, *J* = 17.5, 11.0 Hz, 1H), 3.44 (dd, *J* = 17.5, 6.5 Hz, 1H), 2.34 (s, 3H) |
| 97 | (4-CF₈-Ph)CH₂ | H | NH₂ | CH | Cl | 0 | ¹H NMR (300 MHz, CDCl₆) δ 7.65 (d, *J* = 8.3 Hz, 2H), 7.58 (d, *J* = 8.3 Hz, 2H), 6.97 (s, 1H), 5.72 (dd, *J =* 11.1, 6.5 Hz, 1H), 5.47 (s, 2H), 4.90 (brs, 2H), 3.64 (dd, *J* = 17.5, 11.1 Hz, 1H), 3.46 (dd, *J* = 17.5, 6.5 Hz, 1H) |
| 98 | (4-OMe-Ph)CH₂ | H | NH₂ | CH | Cl | 0 | ¹H NMR (500 MHz, CDCl₆) S 7.40 (d, *J* = 8.7 Hz, 2H), 6.93 (s, 1H), 6.90 (d, *J=* 8.7 Hz, 2H), 5.71 (dd, *J* = 11.1, 6.5 Hz, 1H), 5.36 (s, 2H), 4.86 (brs, 2H), 3.81(s, 3H), 3.62 (dd, *J* = 17.5, 11.1 Hz, 1H), 3.44 (dd, *J* = 17.5, 6.5 Hz, 1H) |
| 99 | (4-F-Ph) CH₂ | H | NH₂ | CH | Cl | 0 | ¹H NMR (500 MHz, CDCl₆) δ 7.44 (m, 2H), 7.07 (m, 2H), 6.95 (s, 1H), 5.71 (dd, *J* = 11.2, 6.4 Hz, 1H), 5.38 (s, 2H), 4.90 (brs, 2H), 3.62 (dd, *J* = 17.4, 11.2 Hz, 1H), 3.45 (dd, *J* = 17.4, 6.4 Hz, 1H) |
| 100 | (4-Br-P h)CH₂ | H | NH₂ | CH | Cl | 0 | ¹H NMR (500 MHz, CDCl₆) δ 7.50 (d, *J* = 8.3 Hz, 2H), 7.32 (d, *J* = 8.3 Hz, 2H), 6.95 (s, 1H), 5.69 (dd, *J* = 11.1, 6.4 Hz, 1H), 5.35 (s, 2H), 5.03 (brs, 2H), 3.61 (dd, *J* = 17.3, 11.1 Hz, 1H), 3.4 5 (dd, *J* = 17.3, 6.4 Hz, 1H) |
| 101 | Me | H | NH₂ | CF | Cl | 0 | ¹H NMR (300 MHz, CDCl₆) δ 5.97 (ddd, *J* = 11.1, 8.7, 1.4 Hz, 1H), 4.98 (brs, 2H), 3.97(s, 3H), 3.96 (dd, *J* = 17.0, 8.7 Hz, 1H), 3.41 (dd, *J* = 17.0, 11.3 Hz, 1H) |
| 102 | H | H | NH₂ | CF | Cl | 0 | ¹H NMR (300 MHz, CD₈OD) δ 6.00 (ddd, *J* = 11.3, 8.5, 1.4 Hz, 1H), 3.89 (dd, *J* = 17.0, 8.5 Hz, 1H), 3.53 (dd, *J* = 17.0, 11.3 Hz, 1H) |
| 103 | Bn | H | NH₂ | CF | Cl | 0 | ¹H NMR (300 MHz, CDCl₆) δ 7.51-7.42 (m, 2H), 7.42-7.30 (m, 3H), 5.89 (ddd, *J* = 11.1, 8.1, 1.4 Hz, 1H), 5.40 (s, 2H), 4.92 (brs, 2H), 4.00 (dd, *J* = 8.1, 17.3 Hz, 1H), 3.45 (dd, *J* = 11.1, 17.3 Hz, 1H) |
| 104 | Me | H | NHz | N | Cl | 0 | ¹H NMR (500 MHz, CDCl₆) δ 6.28 (brs, 2H), 5.69 (dd, *J* = 11.5, 7.8 Hz, 1H), 3.99 (s, 3H), 3.66 (dd, *J* = 17.4, 11.5 Hz, 1H), 3.54 (dd, *J* = 17.4, 7.8 Hz, 1H) |
| 105 | H | H | NH₂ | N | Cl | 0 | ¹H NMR (500 MHz, CD₆OD) δ 5.61 (dd, *J* = 11.1, 7.8 Hz, 1H), 3.72 (dd, *J* = 17.4, 11.1 Hz, 1H), 3.59 (dd, *J* = 17.4, 7.8 Hz, 1H) |
| 106 | Me | H | NHz | N | Br | 0 | ¹H NMR (300 MHz, CDCl₆ δ 6.38 (brs, 2H), 5.62 (dd, *J* = 11.1, 7.8 Hz, 1H), 3.99 (s, 3H), 3.70 (dd, *J* = 17.4, 11.1 Hz, 1H), 3.59 (dd, *J* = 17.4, 7.8 Hz, 1H) |
| 107 | H | H | NHz | N | Br | 0 | ¹H NMR (300 MHz, CD₈OD) δ 5.53 (dd, *J* = 11.1, 7.7 Hz, 1H), 3.75 (dd, *J* = 17.4, 11.1 Hz, 1H), 3.62 (dd, *J* = 17.4, 7.7 Hz, 1H) |
| 108 | Na | H | NHz | CH | Cl | 0 | ¹H NMR (300 MHz, CD₈OD) δ 6.78 (s, 1H), 5.55 (dd, *J* = 11.1, 7.5 Hz, 1H), 3.70 (dd, *J* = 17.4, 11.1 Hz, 1H), 3.46 (dd, *J* = 17.4, 7.5 Hz, 1H) |
| 109 | Li | H | NHz | CH | Cl | 0 | ¹H NMR (300 MHz, CD₆OD) δ 6.77 (s, 1H), 5.53 (dd, *J* = 11.1, 7.5 Hz, 1H), 3.68 (dd, *J* = 17.4, 11.1 Hz, 1H), 3.44 (dd, *J* = 17.4, 7.5 Hz, 1H) |
| 110 | K | H | NH₂ | CH | Cl | 0 | ¹H NMR (300 MHz, CD₈OD) δ 6.76 (s, 1H), 5.53 (dd, *J* = 11.1, 7.5 Hz, 1H), 3.68 (dd, *J* = 17.4, 11.1 Hz, 1H), 3.44 (dd, *J* = 17.4, 7.5 Hz, 1H) |
| 111 | Me | H | Cl | CH | Br | 0 | ¹H NMR (300 MHz, CDCl₆) δ 7.78 (s, 1H), 5.74 (dd, *J* = 11.1, 6.3 Hz, 1H), 4.01 (s,3H), 3.74 (dd, *J* = 17.6, 11.1 Hz, 1H), 3.53 (dd, *J* = 17.6, 6.3 Hz, 1H) |
| 112 | H | H | Cl | CH | Br | 0 | ¹H NMR (300 MHz, CD₈OD) δ 7.81 (s, 1H), 5.76 (dd, *J* = 11.0, 7.1 Hz, 1H), 3.77 (dd, *J* = 17.5, 11.1 Hz, 1H), 3.61 (dd, *J* = 17.5, 7.1 Hz, 1H) |
| 113 | Me | H | Cl | CH | Cl | 0 | ¹H NMR (300 MHz, CDCl₆) δ 7.79 (s, 1H), 5.82 (dd, *J* = 11.3, 6.3 Hz, 1H), 4.02 (s, 3H), 3.71 (dd, *J* = 17.6, 1 1.3 Hz, 1H), 3.50 (dd, *J* = 17.5, 6.3 Hz, 1H) |
| 114 | H | H | Cl | CH | CI | 0 | ¹H NMR (300 MHz, CD₆OD) δ 7.82 (s, 1H), 5.85 (dd, *J* = 11.0, 7.4 Hz, 1H), 3.74 (dd, *J* = 17.5, 11.0 Hz, 1H), 3.58 (dd, *J* = 17.5, 7.3 Hz, 1H) |
| 115 | Me | H | NHMe | CH | Cl | 0 | ¹H NMR (300 MHz, CDCl₆ δ 6.84 (s, 1H), 5.76 (dd, *J* = 11.1, 7.0 Hz, 1H), 5.22 (brs, 1H), 3.97 (s, 3H), 3.66 (dd, *J* = 17.5, 11.1 Hz, 1H), 3.48 (dd, *J* = 17.5, 7.0 Hz, 1H), 3.00 (d, *J* = 4.9 Hz, 3H) |
| 116 | Me | H | NMe₂ | CH | Cl | 0 | ¹H NMR (300 MHz, CDCl₆) δ 7.06 (s, 1H), 5.76 (dd, *J* = 11.0, 6.9 Hz, 1H), 3.98 (s, 3H), 3.64 (dd, *J* = 17.6, 11.0 Hz, 1H), 3.50 (dd, *J* = 17.6, 6.9 Hz, 1H), 3.03 (s, 6H) |
| 117 | H | H | NMe₂ | CH | Cl | 0 | ¹H NMR (300 MHz, CD₈OD) δ 7.08 (s, 1H), 5.76 (dd, *J* = 10.9; 7.9 Hz, 1H), 3.68 (dd, *J* = 17.3, 10.9 Hz, 1H), 3.52 (dd, *J* = 17.3, 7.9 Hz, 1 H), 3.08 (s, 6 H) |

### Example 1. Preparation of methyl 4-amino-3-chloro-6-(3-chloro-4,5-dihydroisoxazol-5-yl)picolinate

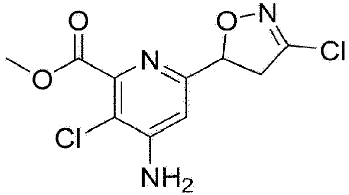

### Process 1) methyl 4-acetamido-3-chloro-6-vinylpicolinate

To a solution of methyl 4-acetamido-3,6-dichloropicolinate (10 g, 38.0 mmol) in EtOH (200 ml) were added potassium vinyltrifluoroborate (5.70 g, 41.8 mmol), [1,1'-bis(diphenylphosphino)ferrocene]dichloropalladium(II) (621 mg, 0.76 mmol), and triethylamine (5.83 ml, 41.8 mmol) at room temperature. The mixture was degassed with nitrogen for 1 hour and heated under reflux for 18 hours under nitrogen. The reaction mixture was cooled to room temperature, filtered, concentrated under reduced pressure, and partitioned between ethyl acetate and water. The aqueous layer was separated and extracted with ethyl acetate (x 3) and the combined organic layers were dried (MgSO₄), filtered, and concentrated under reduced pressure. The residue was purified by silica gel column chromatography (30% EtOAc/hexane) to afford the title compound (9.6 g, 99%).
¹H NMR(300MHz, CDCl₃) δ 8.65(s, 1H), 7.93(brs, 1H), 6.81 (dd, *J*=17.5, 10.8Hz, 1H), 6.27(d, *J*=17.4Hz, 1H), 5.59(d, *J*=10.8Hz, 1H), 5.32(s, 1H), 4.02(s, 3H), 2.33(s, 3H)

### Process 2) methyl 4-acetamido-3-chloro-6-(3-chloro-4,5-dihydroisoxazol-5-yl)picolinate

To a solution of 2-(hydroxyimino)acetic acid (107 mg, 1.2 mmol) in DME(dimethoxyethane) (5 ml) was added N-chlorosuccinimide (320 mg, 2.4 mmol). And the reaction mixture was heated to 110 °C for 1 hour, cooled to room temperature and filtered. To the filtrate were added methyl 4-acetamido-3-chloro-6-vinylpicolinate (254 mg, 1.0 mmol) and a solution of sodium hydrogen carbonate (NaHCO₃) (210 mg, 2.5 mmol) in water (0.5 ml) and the reaction mixture was heated at 80 °C under reflux for 18 hours. After completion of the reaction, the mixture was partitioned between ethyl acetate and water. The aqueous layer was separated and extracted with ethyl acetate (x 3) and the combined organic layers were dried (MgSO₄), filtered, and concentrated under reduced pressure. The residue was purified by silica gel column chromatography (50% EtOAc/hexane) to afford the title compound (232 mg, 70%).
¹H NMR(300MHz, CDCl₃) δ8.77(s, 1H), 7.95(brs, 1H), 5.81 (t, *J*=9.3Hz, 1H), 4.02(s, 3H), 3.61 (d, *J*=9.3Hz, 2 H), 2.33(s, 3 H)

### Process 3) methyl 4-amino-3-chloro-6-(3-chloro-4,5-dihydroisoxazol-5-yl)picolinate

To a solution of methyl 4-acetamido-3-chloro-6-(3-chloro-4,5-dihydroisoxazol-5-yl)picolinate (730 mg, 2.20 mmol) in MeOH (10 ml) was slowly added acetyl chloride (1.3 ml, 17.58 mmol) at 0 °C and the reaction mixture was heated under reflux for 1 hour. After completion of the reaction, the mixture was cooled to room temperature and diluted with water. The solution was extracted with ethyl acetate (x 3) and the combined organic layers were dried (MgSO₄), filtered, and concentrated under reduced pressure. The residue was purified by silica gel column chromatography (50% EtOAc/hexane) to afford the title compound (430 mg, 67%).
¹H NMR(300MHz, CDCl₃) δ6.99(s, 1H), 5.74(dd, J=11.1, 7.0Hz, 1H), 4.99(brs, 2H), 3.99(s, 3H), 3.66(dd, *J*=17.5, 11.1Hz, 1H), 3.45(dd, *J*=17.5, 7.0Hz, 1H)

### Example 2. Preparation of 4-amino-3-chloro-6-(3-chloro-4,5-dihydroisoxazol-5-yl)picolinic acid

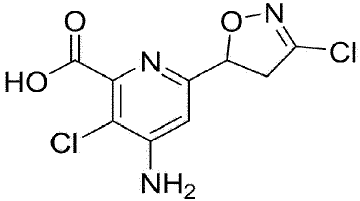

To a solution of methyl 4-amino-3-chloro-6-(3-chloro-4,5-dihydroisoxazol-5-yl)picolinate (230 mg, 0.79 mmol) in THF(tetrahydrofuran)(3 ml) was added a solution of lithium hydroxide (40 mg, 0.95 mmol) in water (0.5 ml) and the reaction mixture was stirred at room temperature for 3 hours. After completion of the reaction, the mixture was acidified with 1N HCl, extracted with ethyl acetate and the organic layer was dried (MgSO₄), filtered and concentrated under reduced pressure to afford the title compound (215 mg, 98%) without the purification by silica gel column chromatography.
¹H NMR(500MHz, CD₃OD) δ7.09(s, 1H), 5.75(dd, *J*=10.1, 7.7Hz, 1H), 3.75(dd, *J*=17.4, 10.1Hz, 1H), 3.63(dd, *J*=17.4, 7.7Hz, 1H)

### Example 3. Preparation of methyl 4-amino-3-chloro-6-(3-methyl-4,5-dihydroisoxazol-5-yl)picolinate

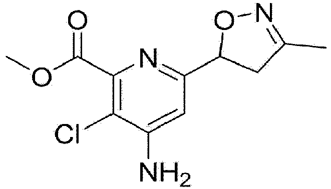

### Process 1) methyl 4-acetamido-3-chloro-6-(3-methyl-4,5-dihydroisoxazol-5-yl)picolinate

To a solution of methyl 4-acetamido-3-chloro-6-vinylpicolinate (300 mg, 1.18 mmol) in benzene (5 ml) were added phenyl isocyanate(0.28 ml, 2.60 mmol), nitroethane (93 *µℓ*, 1.3 mmol) and triethylamine (8 *µℓ*, 0.06 mmol) at 0 °C, and the reaction mixture was heated at 90 °C under reflux for 18 hours. After completion of the reaction, the mixture was partitioned between ethyl acetate and water. The aqueous layer was separated and extracted with ethyl acetate (x 3) and the combined organic layers were dried (MgSO₄), filtered, and concentrated under reduced pressure. The residue was purified by silica gel column chromatography (50% EtOAc/hexane) to afford the title compound (256 mg, 70%).
¹H NMR(300MHz, CDCl₃) δ8.73(s, 1H), 7.93(brs, 1H), 5.63(dd, *J*=10.1, 6.9Hz, 1H), 4.01 (s, 3H), 3.42(dd, *J*=17.3, 10.1 Hz, 1H), 3.27(dd, *J*=17.3, 6.9Hz, 1H), 2.31 (s, 3H), 2.03(s, 3H)

### Process 2) methyl 4-amino-3-chloro-6-(3-methyl-4,5-dihydroisoxazol-5-yl)picolinate

To a solution of methyl 4-acetamido-3-chloro-6-(3-methyl-4,5-dihydroisoxazol -5-yl)picolinate (220 mg, 0.71 mmol) in MeOH (4 ml) was slowly added acetyl chloride (0.42 ml, 5.65 mmol) at 0 °C and the reaction mixture was heated under reflux for 1 hour. After completion of the reaction, the mixture was cooled to room temperature and diluted with water. The solution was extracted with 10% MeOH/ dichloromethane (x 3) and the combined organic layers were dried (MgSO₄), filtered, and concentrated under reduced pressure. The residue was purified by silica gel column chromatography (50% EtOAc/hexane) to afford the title compound (72 mg, 38%).
¹H NMR(300MHz, CDCl₃) δ7.00(s, 1H), 5.58(dd, *J*=11.1, 5.6Hz, 1H), 5.32(brs, 2H), 3.99(s, 3H), 3.43(dd, *J*=17.4, 11.1Hz, 1H), 3.17(dd, *J*=17.4, 5.6Hz, 1H), 2.01 (s, 3H)

### Example 4. Preparation of 4-amino-3-chloro-6-(3-methyl-4,5-dihydroisoxazol-5-yl)picolinic acid

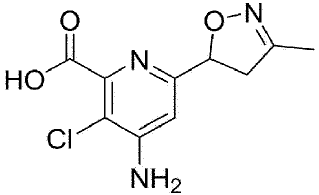

To a solution of methyl 4-amino-3-chloro-6-(3-methyl-4,5-dihydroisoxazol-5-yl)picolinate (45 mg, 0.17 mmol) in THF (0.5 ml) was added a solution of lithium hydroxide (8.4 mg, 0.20 mmol) in water (0.25 ml) and the reaction mixture was stirred at room temperature for 3 hours. After completion of the reaction, the mixture was acidified with 1N HCl, extracted with ethyl acetate and the organic layer was dried (MgSO₄), filtered and concentrated under reduced pressure to afford the title compound (32 mg, 75%) without the purification by silica gel column chromatography.
¹H NMR(300MHz, CD₃OD) δ6.95(s, 1H), 5.60(dd, *J*=11.1, 6.1Hz, 1H), 3.56(dd, *J*=17.6, 11.1Hz, 1H), 3.14(dd, *J*=17.6, 6.1Hz, 1H), 2.02(s, 3H)

### Example 5. Preparation of methyl 4-amino-3-chloro-6-(3-bromo-4,5-dihydroisoxazol-5-yl)picolinate

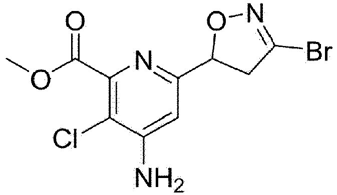

### Process 1) methyl 4-acetamido-3-chloro-6-(3-bromo-4,5-dihydroisoxazol-5-yl)picolinate

To a solution of methyl 4-acetamido-3-chloro-6-vinylpicolinate (3.5 g, 13.74 mmol) in EtOAc (50 ml) were added dibromoaldoxime (4.18 g, 2.89 mmol) and a solution of sodium hydrogen carbonate (NaHCO₃) (2.89 g, 34.36 mmol) in water (5 ml) and the reaction mixture was heated at 80 °C under reflux for 18 hours. After completion of the reaction, the mixture was partitioned between ethyl acetate and water. The aqueous layer was separated and extracted with ethyl acetate (x 3) and the combined organic layers were dried (MgSO₄), filtered, and concentrated under reduced pressure. The residue was purified by silica gel column chromatography (50% EtOAc/hexane) to afford the title compound (4.2 g, 81%).
¹H NMR(300MHz, CDCl₃) δ8.74(s, 1H), 7.96(brs, 1H), 5.72(t, *J*=9.3Hz, 1 H), 4.00(s, 3H), 3.63(d, *J*=9.3Hz, 2H), 2.31 (s, 3H)

### Process 2) methyl 4-amino-3-chloro-6-(3-bromo-4,5-dihydroisoxazol-5-yl)picolinate

To a solution of methyl 4-acetamido-3-chloro-6-(3-bromo-4,5-dihydroisoxazol -5-yl)picolinate (3.54 g, 9.29 mmol) in MeOH (40 ml) was slowly added BF₃-OEt₂ (5.16 ml, 41.82 mmol) at 0 °C and the reaction mixture was heated under reflux for 1 hour. After completion of the reaction, the mixture was cooled to room temperature and diluted with water. The solution was extracted with ethyl acetate (x 3) and the combined organic layers were washed with saturated aqueous sodium hydrogen carbonate (NaHCO₃) solution, dried (MgSO₄), filtered, and concentrated under reduced pressure. The residue was purified by silica gel column chromatography (40% EtOAc/hexane) to afford the title compound (2.86 g, 92%).
¹H NMR(300MHz, CDCl₃) δ6.97(s, 1H), 5.64(dd, *J*=11.1, 7.0Hz, 1H), 4.90 (brs, 2H), 3.99(s, 3H), 3.65(dd, *J*=17.5, 11.1 Hz, 1H), 3.43(dd, *J*=17.5, 7.0Hz, 1H)

### Example 6. Preparation of 4-amino-3-chloro-6-(3-bromo-4,5-dihydroisoxazol-5-yl)picolinic acid

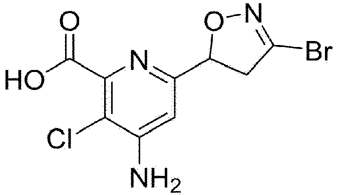

To a solution of methyl 4-amino-3-chloro-6-(3-bromo-4,5-dihydroisoxazol-5-yl)picolinate (2.5 g, 7.47 mmol) in THF (30 ml) was added a solution of lithium hydroxide (376 mg, 8.97 mmol) in water (10 ml) and the reaction mixture was stirred at room temperature for 1 hour. After completion of the reaction, the mixture was acidified with 1N HCl, extracted with ethyl acetate and the organic layer was dried (MgSO₄), filtered and concentrated under reduced pressure to afford the title compound (2.2 g, 92%) without the purification by silica gel column chromatography.
¹H NMR(500MHz, CD₃OD) δ6.94(s, 1H), 5.66(dd, *J*=10.1, 7.6Hz, 1H), 3.75(dd, *J*=17.4, 10.1Hz, 1H), 3.43(dd, *J*=17.4, 7.6Hz, 1H)

### Example 7. Preparation of methyl 4-amino-3-chloro-6-(3-trifluoromethyl-4,5-dihydroisoxazol-5-yl)picolinate

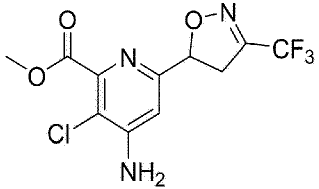

### Process 1) methyl 4-acetamido-3-chloro-6-(3-(trifluoromethyl)-4,5-dihydroisoxazol-5-yl)picolinate

To a solution of methyl 4-acetamido-3-chloro-6-vinylpicolinate (300 mg, 1.18 mmol) in EtOAc (5 ml) were added 2,2,2-trifluoro-N-hydroxyacetimidobromide (406 mg, 1.42 mmol) and a solution of sodium hydrogen carbonate (NaHCO₃) (248 mg, 2.95 mmol) in water (0.5 ml) and the reaction mixture was heated at 80 °C under reflux for 18 hours. After completion of the reaction, the mixture was partitioned between ethyl acetate and water. The aqueous layer was separated and extracted with ethyl acetate (x 3) and the combined organic layers were dried (MgSO₄), filtered, and concentrated under reduced pressure. The residue was purified by silica gel column chromatography (50% EtOAc/hexane) to afford the title compound (228 mg, 53%).
¹H NMR(300MHz, CDCl₃) δ 8.73(s, 1H), 7.95(brs, 1H), 5.89(dd, *J*=11.6, 7.7Hz, 1H), 4.00(s, 3H), 3.60-3.67(m, 2H), 2.32(s, 3H)

### Process 2) methyl 4-amino-3-chloro-6-(3-(trifluoromethyl)-4,5-dihydroisoxazol-5-yl)picolinate

To a solution of methyl 4-acetamido-3-chloro-6-(3-trifluoromethyl-4,5-dihydroisoxazol-5-yl)picolinate (173 mg, 0.47 mmol) in MeOH (4 ml) was slowly added acetyl chloride (0.28 ml, 3.78 mmol) at 0 °C and the reaction mixture was heated under reflux for 1 hour. After completion of the reaction, the mixture was cooled to room temperature and diluted with water. The solution was extracted with 10% MeOH/dichloromethane (x 3) and the combined organic layers were dried (MgSO₄), filtered, and concentrated under reduced pressure. The residue was purified by silica gel column chromatography (50% EtOAc/hexane) to afford the title compound (132 mg, 86%).
¹H NMR(300MHz, CDCl₃) δ 6.92(s, 1H), 5.87(dd, *J*=11.1, 6.8Hz, 1H), 4.92(brs, 2H), 4.00(s, 3H), 3.52-3.63(m, 2H)

### Example 8. Preparation of 4-amino-3-chloro-6-(3-trifluoromethyl-4,5-dihydroisoxazol-5-yl)picolinic acid

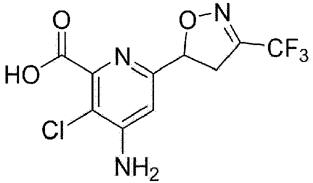

To a solution of methyl 4-amino-3-chloro-6-(3-trifluoromethyl-4,5-dihydroisoxazol-5-yl)picolinate (94 mg, 0.29 mmol) in THF (1 ml) was added a solution of lithium hydroxide (14.6 mg, 0.35 mmol) in water (0.5 ml) and the reaction mixture was stirred at room temperature for 3 hours. After completion of the reaction, the mixture was acidified with 1N HCl, extracted with ethyl acetate and the organic layer was dried (MgSO₄), filtered and concentrated under reduced pressure to afford the title compound (77 mg, 86%) without the purification by silica gel column chromatography.
¹H NMR(300MHz, CD₃OD) δ 6.92(s, 1H), 5.84(dd, *J*=11.6, 7.7Hz, 1H), 3.76(dd, *J*=17.8, 11.6Hz, 1H), 3.48(dd, *J*=17.8, 7.7Hz, 1H)

### Example 9. Preparation of methyl 4-amino-3-chloro-6-(3-tert-butyl-4,5-dihydroisoxazol-5-yl)picolinate

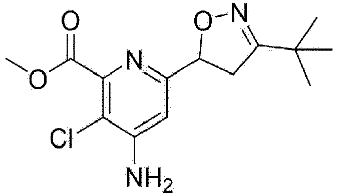

### Process 1) methyl 4-acetamido-3-chloro-6-(3-tert-butyl-4,5-dihydroisoxazol-5-yl)picolinate

To a solution of pivalaldehyde oxime (191 mg, 1.88 mmol) in dichloromethane (5 ml) were added N-chlorosuccinimide (273 mg, 2.04 mmol) and DMF (12 *µ*ℓ, 0.16 mmol) and the reaction mixture was heated to 35 °C. After pivalaldehyde oxime had been consumed, the reaction solution was cooled to 0 °C. And then, methyl 4-acetamido-3-chloro-6-vinylpicolinate (400 mg, 1.57 mmol) and sodium hydrogen carbonate (NaHCO3) (330 mg, 3.93 mmol) were added to the reaction mixture and the reaction mixture was stirred at 30 °C for 18 hours. After completion of the reaction, the mixture was partitioned between dichloromethane and water. The aqueous layer was separated and extracted with dichloromethane (x 3) and the combined organic layers were dried (MgSO₄), filtered, and concentrated under reduced pressure. The residue was purified by silica gel column chromatography (30% EtOAc/hexane) to afford the title compound (380 mg, 68%).
¹H NMR(300MHz, CDCl₃) δ 8.69(s, 1H), 7.92(brs, 1H), 5.61 (dd, *J*=10.1, 6.6Hz, 1H), 4.01 (s, 3H), 3.43(dd, *J*=16.9, 10.1 Hz, 1H), 3.27(dd, *J*=16.9, 6.6Hz, 1H), 2.31 (s, 3H), 1.24(s, 9H)

### Process 2) methyl 4-amino-3-chloro-6-(3-tert-butyl-4,5-dihydroisoxazol-5-yl)picolinate

To a solution of methyl 4-acetamido-3-chloro-6-(3-tert-butyl-4,5-dihydroisoxazol-5-yl)picolinate(320 mg, 0.90 mmol) in MeOH (6 ml) was slowly added acetyl chloride (0.54 ml, 7.24 mmol) at 0 °C and the reaction mixture was heated under reflux for 1 hour. After completion of the reaction, the mixture was cooled to room temperature and diluted with water. The solution was extracted with ethyl acetate (x 3) and the combined organic layers were dried (MgSO₄), filtered, and concentrated under reduced pressure to afford the title compound (262 mg, 93%).
¹H NMR(300MHz, CDCl₃) δ 6.98(s, 1H), 5.54(dd, *J*=10.1, 5.9Hz, 1H), 4.85(brs, 2H), 3.98(s, 3H), 3.45(dd, *J*=17.0, 10.1Hz, 1H), 3.17(dd, *J*=17.0, 5.9Hz, 1H), 1.18(s, 9H)

### Example 10. Preparation of 4-amino-3-chloro-6-(3-tert-butyl-4,5-dihydroisoxazol-5-yl)picolinic acid

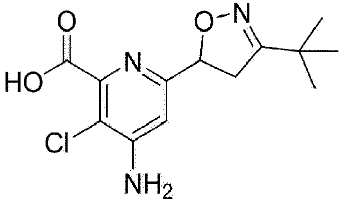

To a solution of methyl 4-amino-3-chloro-6-(3- tert-butyl-4,5-dihydroisoxazol-5-yl)picolinate (198mg, 0.64 mmol) in THF (2 ml) was added a solution of lithium hydroxide (32 mg, 0.76 mmol) in water (1 ml) and the reaction mixture was stirred at room temperature for 3 hours. After completion of the reaction, the mixture was acidified with 1N HCl, extracted with ethyl acetate and the organic layer was dried (MgSO₄), filtered and concentrated under reduced pressure to afford the title compound (163 mg, 86%) without the purification by silica gel column chromatography.
¹H NMR(300MHz, CD₃OD) δ 6.90(s, 1H), 5.57(dd, *J*=11.1, 6.2Hz, 1H), 3.58(dd, *J*=17.4, 11.1 Hz, 1H), 3.18(dd, *J*=17.4, 6.2Hz, 1H), 1.20(s, 9H)

### Example 11. Preparation of methyl 4-amino-3-chloro-6-(4,5-dihydroisoxazol-5-yl)picolinate

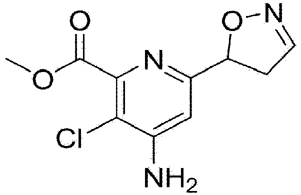

### Process 1) methyl 4-acetamido-3-chloro-6-(4,5-dihydroisoxazol-5-yl))picolinate

To a solution of methyl 4-acetamido-3-chloro-6-vinylpicolinate (400 mg, 1.57 mmol) in benzene (6 ml) and dichloromethane (6 ml) were added nitromethane (0.17 ml. 3.14 mmol), triethylamine (0.66 ml, 4.71 mmol) and trimethylsilyl chloride (0.6 ml, 4.71 mmol) at 0 °C and the reaction mixture was heated at 60 °C for 2 hours. The mixture was filtered and concentrated under reduced pressure. The residue was dissolved in diethyl ether, and p-toluenesulfonic acid (65.6 mg, 0.35 mmol) was added to the solution. The reaction mixture was stirred at room temperature for 1 hour and washed with sodium hydrogen carbonate (NaHCO₃) aqueous solution. The mixture was extracted with ethyl acetate (x 3), dried (MgSO₄), filtered, and concentrated under reduced pressure. The residue was purified by silica gel column chromatography (50% EtOAc/hexane) to afford the title compound (610 mg, 77%).
¹H NMR(300MHz, CDCl₃) δ 8.67(s, 1H), 7.98(brs, 1H), 7.20(s, 1H), 5.60(dd, *J*=11.1, 7.1Hz, 1H), 3.99(s, 3H), 3.42(ddd, *J*=17.3, 11.1, 1.7Hz, 1H), 3.37(ddd, *J*=17.3, 7.1, 1.7Hz, 1H), 2.30(s, 3H)

### Process 2) methyl 4-amino-3-chloro-6-(4,5-dihydroisoxazol-5-yl)picolinate

To a solution of methyl 4-acetamido-3-chloro-6-(4,5-dihydroisoxazol-5-yl)picolinate (43 mg, 0.14 mmol) in MeOH (1 ml) was slowly added acetyl chloride (0.1 ml, 1.15 mmol) at 0 °C and the reaction mixture was heated under reflux for 1 hour. After completion of the reaction, the mixture was cooled to room temperature and diluted with water. The solution was extracted with ethyl acetate (x 3) and the combined organic layers were dried (MgSO₄), filtered, and concentrated under reduced pressure to afford the title compound (13 mg, 92%).
¹H NMR(300MHz CD₃OD) δ 7.31 (s, 1H), 6.91 (s, 1H), 5.40(dd, *J*=11.3, 6.8Hz, 1H), 3.94(s, 3H), 3.44(ddd, *J*=17.4, 11.4, 1.6Hz, 1H), 3.19(ddd, *J*=17.4, 6.8, 1.6Hz, 1H)

### Example 12. Preparation of methyl 4-amino-3-chloro-6-(3-bromo-5-methyl-4,5-dihydroisoxazol-5-yl)picolinate

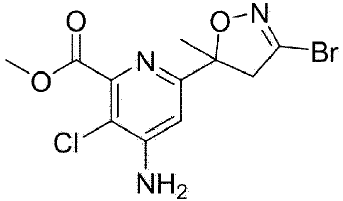

### Process 1) methyl 4-acetamido-3-chloro-6-(prop-1-en-2-yl)picolinate

To a solution of methyl 4-acetamido-3,6-dichloropicolinate (10 g, 38.01 mmol) in EtOH (130 ml) were added potassium trifluoro(prop-1-en-2-yl)borate (8.44 g, 57.02 mmol), [1,1'-bis(diphenylphosphino)ferrocene]dichloropalladium(II) (556mg, 0.76 mmol), and triethylamine (7.9 ml, 57.02 mmol) at room temperature. The mixture was degassed with nitrogen for 1 hour and heated under reflux for 18 hours under nitrogen. The reaction mixture was cooled to room temperature, filtered, concentrated under reduced pressure, and partitioned between ethyl acetate and water. The aqueous layer was separated and extracted with ethyl acetate (x 3) and the combined organic layers were dried (MgSO₄), filtered, and concentrated under reduced pressure. The residue was purified by silica gel column chromatography (30% EtOAc/hexane) to afford the title compound (9.42 g, 92%).
¹H NMR(500MHz, CDCl₃) δ 8.83(s, 1H), 7.93(brs, 1H), 3.99(s, 3 H), 3.87(d, *J*=17.4Hz, 1H), 3.25(d, *J*=17.4Hz, 1H), 2.30(s, 3H), 1.78(s, 3H)

### Process 2) methyl 4-acetamido-3-chloro-6-(3-bromo-5-methyl-4,5-dihydroisoxazol-5-yl)picolinate

To a solution of methyl 4-acetamido-3-chloro-6-(prop-1-en-2-yl)picolinate (5 g, 18.61 mmol) in EtOAc (60 ml) were added dibromoaldoxime (5.66 g, 27.91 mmol) and a solution of sodium hydrogen carbonate (NaHCO₃) (3.91 g, 46.52 mmol) in water (6 ml) and the reaction mixture was heated at 80 °C for 18 hours. After completion of the reaction, the mixture was partitioned between ethyl acetate and water. The aqueous layer was separated and extracted with ethyl acetate (x 3) and the combined organic layers were dried (MgSO₄), filtered, and concentrated under reduced pressure. The residue was purified by silica gel column chromatography (40% EtOAc/hexane) to afford the title compound (5.1 g, 70%).
¹H NMR(500MHz, CDCl₃) δ 8.83(s, 1H), 7.93(brs, 1H), 3.99(s, 3H), 3.87(d, *J*=17.4Hz, 1H), 3.25(d, *J*=17.4Hz, 1H), 2.30(s, 3H), 1.78(s, 3H)

### Process 3) methyl 4-amino-3-chloro-6-(3-bromo-5-methyl-4,5-dihydroisoxazol-5-yl)picolinate

To a solution of methyl 4 -acetamido-3-chloro- 6 -(3-bromo-5-methyl-4,5-dihydroisoxazol-5-yl)picolinate (300 mg, 0.77 mmol) in MeOH (15 ml) was slowly added BF₃-OEt₂ (0.43 ml, 3.46 mmol) at 0 °C and the reaction mixture was heated under reflux for 1 hour. After completion of the reaction, the solution was extracted with ethyl acetate (x 3) and the combined organic layers were washed with saturated aqueous sodium hydrogen carbonate (NaHCO₃) solution, dried (MgSO₄), filtered, and concentrated under reduced pressure. The residue was purified by silica gel column chromatography (40% EtOAc/hexane) to afford the title compound (247 mg, 92%).
¹H NMR(500MHz, CDCl₃) δ 7.10(s, 1H), 5.05(brs, 2H), 3.98(s, 3H), 3.77(d, *J*=17.4Hz, 1H), 3.26(d, *J*=17.4Hz, 1H), 1.75(s, 3H)

### Example 13. Preparation of 4-amino-3-chloro-6-(3-bromo-5-methyl-4,5-dihydroisoxazol-5-yl)picolinic acid

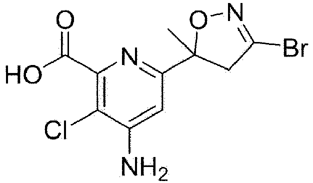

To a solution of methyl 4-amino-3-chloro-6-(3-bromo-5-methyl-4,5-dihydroisoxazol-5-yl)picolinate (150 mg, 0.43 mmol) in THF (1 ml) was added a solution of lithium hydroxide (22 mg, 0.52 mmol) in water (0.5 ml) and the reaction mixture was stirred at room temperature for 1 hour. After completion of the reaction, the mixture was acidified to pH 3-4 with 1N HCl, extracted with ethyl acetate and the organic layer was dried (MgSO₄), filtered and concentrated under reduced pressure to afford the title compound (88 mg, 92%) without the purification by silica gel column chromatography .
¹H NMR(500MHz, CD₃OD) δ 7.05(s, 1H), 3.67(d,*J*=17.4Hz, 1H), 3.43(d, *J*=17.4Hz, 1H), 1.75(s, 3H)

### Example 14. Preparation of methyl 4-amino-3-chloro-6-(3-chloro-5-methyl-4,5-dihydroisoxazol-5-yl)picolinate

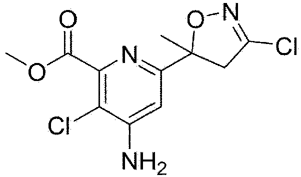

### Process 1) methyl 4-acetamido-3-chloro-6-(3-chloro-5-methyl-4,5-dihydroisoxazol-5-yl)picolinate

To a solution of 2-(hydroxyimino)acetic acid (119 mg, 1. 34 mmol) in DME (5 ml) was added N-chlorosuccinimide (358 mg, 2.68 mmol) and the reaction mixture was heated to 110 °C for 1 hour, cooled to room temperature and filtered. To the filtrate were added methyl 4-acetamido-3-chloro-6-(prop-1-en-2-yl)picolinate (300 mg, 1.12 mmol) and a solution of sodium hydrogen carbonate (NaHCO₃) (235 mg, 2.79 mmol) in water (0.5 ml) and the reaction mixture was heated at 80 °C under reflux for 18 hours. After completion of the reaction, the mixture was partitioned between ethyl acetate and water. The aqueous layer was separated and extracted with ethyl acetate (x 3) and the combined organic layers were dried (MgSO₄), filtered, and concentrated under reduced pressure. The residue was purified by silica gel column chromatography (50% EtOAc/hexane) to afford the title compound (271 mg, 70%).
¹H NMR(300MHz, CDCl₃) δ 8.82(s, 1H), 7.98(brs, 1H), 3.99(s, 3H), 3.83(d, *J*=17.3Hz, 1H), 3.20(d, *J*=17.3Hz, 1H), 2.31 (s, 3H), 1.78(s, 3H)

### Process 2) methyl 4-amino-3-chloro-6-(3-chloro-5-methyl-4.5-dihydroisoxazol-5-yl)picolinate

To a solution of methyl 4-acetamido-3-chloro-6-(3-chloro-5-methyl-4,5-dihydroisoxazol-5-yl)picolinate (1.5 g, 4.33 mmol) in MeOH (15 ml) was slowly added acetyl chloride (2.47 ml, 34.67 mmol) at 0 °C and the reaction mixture was heated under reflux for 1 hour. After completion of the reaction, the mixture was cooled to room temperature and diluted with water. The solution was extracted with ethyl acetate (x 3) and the combined organic layers were dried (MgSO₄), filtered, and concentrated under reduced pressure. The residue was purified by silica gel column chromatography (50% EtOAc/hexane) to afford the title compound (997 mg, 76%).
¹H NMR(300MHz, CDCl₃) δ 7.08(s, 1H), 4.87(brs, 2H), 3.98(s, 3H), 3.70(d, *J*=17.3Hz, 1H), 3.20(d, *J*=17.4Hz, 1H), 1.75(s, 3H)

### Example 15. Preparation of methyl 4-amino-6-((3-chloro-4,5-dihydroisoxazol-5-yl)methyl)-3-chloropicolinate

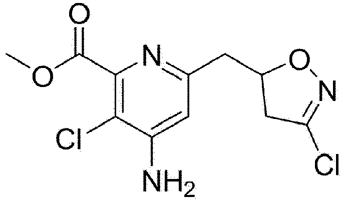

### Process 1) methyl 4-acetamido-3-chloro-6-((3-chloro-4,5-dihydroisoxazol-6-yl)methyl)picolinate

To a solution of 2-(hydroxyimino)acetic acid (249 mg, 2.79 mmol) in DME (10 ml) was added N-chlorosuccinimide (745mg, 5.58 mmol), the reaction mixture was heated to 110 °C for 1 hour, cooled to room temperature and filtered. To the filtrate were added methyl 4-acetamido-3-chloro-6-allylpicolinate (500 mg, 1.86 mmol) and a solution of sodium hydrogen carbonate (NaHCO₃) (391 mg, 4.6 mmol) in water (1 ml) and the reaction mixture was heated at 80 °C for 16 hours. After completion of the reaction, the mixture was partitioned between ethyl acetate and water. The aqueous layer was separated and extracted with ethyl acetate (x 3) and the combined organic layers were dried (MgSO₄), filtered, and concentrated under reduced pressure. The residue was purified by silica gel column chromatography (40% EtOAc/hexane) to afford the title compound (300 mg, 47%).
¹H NMR(300MHz, CDCl₃) δ 8.48(s, 1H), 7.95(brs, 1H), 5.13-5.28(m, 1H), 4.00(s, 3H), 3.17-3.36(m, 2H), 2.99-3.17(m, 2H), 2.30(s, 3H)

### Process 2) methyl 4-amino-3-chloro-6-((3-chloro-4,5-dihydroisoxazol-6-yl)methyl)picolinate

To a solution of methyl 4-acetamido-3-chloro-6-((3-chloro-4,5-dihydroisoxazol-6-yl)methyl)picolinate (230 mg, 0.66 mmol) in MeOH (3 ml) was slowly added acetyl chloride (0.4 ml, 5.32 mmol) at 0 °C and the reaction mixture was heated under reflux for 1 hour. After completion of the reaction, the mixture was cooled to room temperature and diluted with water. The solution was extracted with ethyl acetate (x 3) and the combined organic layers were dried (MgSO₄), filtered, and concentrated under reduced pressure. The residue was purified by silica gel column chromatography (50% EtOAc/hexane) to afford the title compound (141 mg, 76%).
¹H NMR(300MHz, CDCl₃) δ 6.68(s, 1H), 5.04-5.22(m, 1H), 4.85(brs, 2H), 3.97(s, 3H), 3.17-3.35(m, 1H), 2.89-3.16(m, 3H)

### Example 16. Preparation of 4-amino-6-((3-chloro-4,5-dihydroisoxazol-5-yl)methyl)-3-chloropicolinic acid

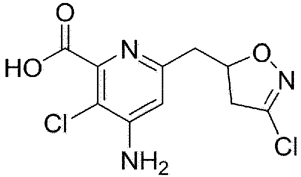

To a solution of methyl 4-amino-3-chloro-6-((3-chloro-4,5-dihydroisoxazol-6-yl)methyl)picolinate (90 mg, 0.29 mmol) in THF (1 ml) was added a solution of lithium hydroxide (15 mg, 0.45 mmol) in water (0.5 ml) and the reaction mixture was stirred at room temperature for 3 hours. After completion of the reaction, the mixture was acidified with 1N HCl, extracted with ethyl acetate and the organic layer was dried (MgSO₄), filtered and concentrated under reduced pressure to afford the title compound (80 mg, 94%) without the purification by silica gel column chromatography.
¹H NMR(300MHz, CD₃OD) δ 6.87(s, 1H), 5.03-5.20(m, 1H), 3.47(m, 1H), 3.03-3.18(m, 3H)

### Example 17. Preparation of ethyl 4-amino-3-chloro-6-(3-chloro-4,5-dihydroisoxazol-5-yl)picolinate

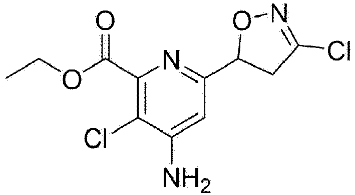

To a solution of 4-amino-3-chloro-6-(3-chloro-4,5-dihydroisoxazol-5-yl)picolinic acid (250 mg, 0.91 mmol) in DMF (3 ml) were added potassium carbonate (K₂CO₃) (250mg, 1.81 mmol) and iodoethane (109 *µℓ*, 1.36 mmol). The reaction mixture was stirred at room temperature for 2 hours and partitioned between ethyl acetate and water. The aqueous layer was separated and extracted with ethyl acetate (x 3) and the combined organic layers were dried (MgSO₄), filtered, and concentrated under reduced pressure. The residue was purified by silica gel column chromatography (40% EtOAc/hexane) to afford the title compound (209 mg, 76%).
¹H NMR(500MHz, CDCl₃) δ 6.96(s, 1H), 5.72(dd, *J*=11.0, 6.4Hz, 1H), 5.03(brs, 2H), 4.46(q, *J*=7.3Hz, 2H), 3.63(dd, *J*=17.4, 11.0Hz, 1H), 3.46(dd, *J*=17.4, 6.4Hz, 1H), 1.42(t, *J*=7.3Hz, 3H)

### Example 18. Preparation of isopropyl 4-amino-3-chloro-6-(3-chloro-4,5-dihydroisoxazol-5-yl)picolinate

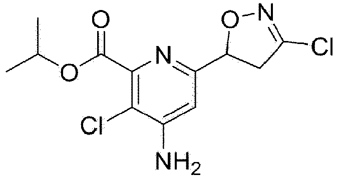

To 4-amino-3-chloro-6-(3-chloro-4,5-dihydroisoxazol-5-yl)picolinic acid (250 mg, 0.75 mmol) were added isopropyl alcohol (0.14 ml, 1.81 mmol) and titanium isopropoxide (54 ml, 0.18 mmol). The reaction mixture was heated under reflux for 18 hours. After completion of the reaction, the mixture was cooled to room temperature, and the combined organic layers were washed with brine, dried (MgSO₄), filtered, and concentrated under reduced pressure. The residue was purified by silica gel column chromatography (40% EtOAc/hexane) to afford the title compound (198 mg, 69%).
¹H NMR(300MHz, CDCl₃) δ 6.93(s, 1H), 5.71 (dd, *J*=11.1, 6.5Hz, 1H), 5.32(m, 1H), 4.97(brs, 2H), 3.63(dd, *J*=17.5, 11.1Hz, 1H), 3.47(dd, *J*=17.4, 6.5Hz, 1H), 1.41 (d, *J*=6.4Hz, 6H)

### Example 19. Preparation of isobutyl 4-amino-3-chloro-6-(3-chloro-4,5-dihydroisoxazol-5-yl)picolinate

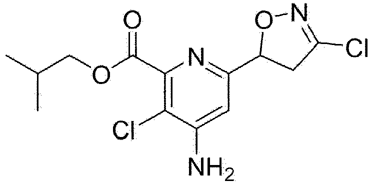

To a solution of 4-amino-3-chloro-6-(3-chloro-4,5-dihydroisoxazol-5-yl)picolinic acid (150 mg, 0.54 mmol) in DMF (1.5 ml) were added potassium carbonate (K₂CO₃) (188 mg, 1.36 mmol) and 1-iodo-2-methylpropane (94 *µ*ℓ, 0.82 mmol). The reaction mixture was stirred at room temperature for 2 hours and the combined organic layers were washed with brine, dried (MgSO₄), filtered, and concentrated under reduced pressure. The residue was purified by silica gel column chromatography (40% EtOAc/hexane) to afford the title compound (122 mg, 60%).
¹H NMR(300MHz, CDCl₃) δ 6.96(s, 1H), 5.72(dd, *J*=11.1, 6.4Hz, 1H), 5.02(brs, 2H), 4.18(d, *J*=6.8Hz, 2H), 3.62(dd, *J*=17.5, 11.1Hz, 1H), 3.48(dd, *J*=17.4, 6.4Hz, 1H), 2.10(m, 1H), 1.02(d, *J*=6.8Hz, 6H)

### Example 20. Preparation of benzyl 4-amino-3-chloro-6-(3-chloro-4,5-dihydroisoxazol-5-yl)picolinate

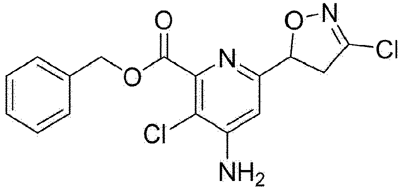

To a solution of 4-amino-3-chloro-6-(3-chloro-4,5-dihydroisoxazol-5-yl)picolinic acid (350 mg, 1.09 mmol) in DMF (5 ml) were added potassium carbonate (K₂CO₃) (302 mg, 2.18 mmol) and benzyl bromide (0.16 ml, 1.31 mmol). The reaction mixture was stirred at room temperature for 1 hour and partitioned between ethyl acetate and brine. The aqueous layer was separated and extracted with ethyl acetate (x 3) and the combined organic layers were dried (MgSO₄), filtered, and concentrated under reduced pressure. The residue was purified by silica gel column chromatography (40% EtOAc/hexane) to afford the title compound (405 mg, 90%).
¹H NMR(300MHz, CDCl₃) δ 7.42-7.51 (m, 2H), 7.30-7.42(m, 3H), 6.96(s, 1H), 5.72(dd, *J*=11.1, 6.5Hz, 1H), 5.42(s, 2H), 4.98(brs, 2H), 3.63(dd, *J*=17.5, 11.1Hz, 1H), 3.45(dd, *J*=17.5, 6.5Hz, 1H)

### Example 21. Preparation of 4-methylbenzyl 4-amino-3-chloro-6-(3-chloro-4,5-dihydroisoxazol-5-yl)picolinate

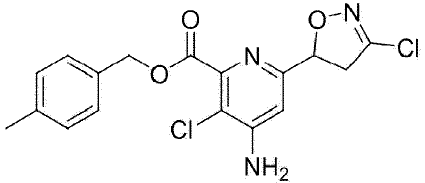

To a solution of 4-amino-3-chloro-6-(3-chloro-4,5-dihydroisoxazol-5-yl)picolinic acid (250 mg, 0.91 mmol) in DMF (3 ml) were added potassium carbonate (K₂CO₃) (350 mg, 1.81 mmol) and 4-methylbenzyl chloride (180 *µℓ*, 1.36 mmol). The reaction mixture was stirred at room temperature for 3 hours and partitioned between ethyl acetate and brine. The aqueous layer was separated and extracted with ethyl acetate (x 3) and the combined organic layers were dried (MgSO₄), filtered, and concentrated under reduced pressure. The residue was purified by silica gel column chromatography (40% EtOAc/hexane) to afford the title compound (229 mg, 66%).
¹H NMR(300MHz, CDCl₃) δ 7.34(d, *J*=7.9Hz, 2H), 7.17(d, *J*=7.9Hz, 2H), 6.91 (s, 1H), 5.68(dd, *J*=11.0, 6.5Hz, 1H), 5.37(s, 2H), 4.96(brs, 2H), 3.59(dd, *J*=17.5, 11.0Hz, 1H), 3.44(dd, *J*=17.5, 6.5Hz, 1H), 2.34(s, 3H)

### Example 22. Preparation of 4-trifluoromethylbenzyl 4-amino-3-chloro-6-(3-chloro-4,5-dihydroisoxazol-5-yl)picolinate

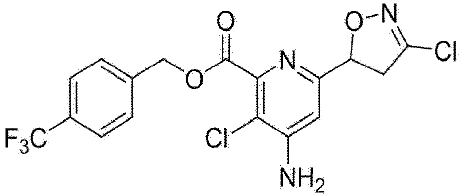

To a solution of picolinic acid (50 mg, 0.18 mmol) in DMF (1 ml) were added potassium carbonate (K₂CO₃) (50 mg, 0.36 mmol) and 4-trifluoromethylbenzyl bromide(42 *µℓ*, 0.27 mmol). The reaction mixture was stirred at room temperature for 3 hours and partitioned between ethyl acetate and brine. The aqueous layer was separated and extracted with ethyl acetate (x 3) and the combined organic layers were dried (MgSO₄), filtered, and concentrated under reduced pressure. The residue was purified by silica gel column chromatography (40% EtOAc/hexane) to afford the title compound (56 mg, 71%).
¹H NMR(300MHz, CDCl₃) δ 7.65(d, *J*=8.3Hz, 2H), 7.58(d, *J*=8.3Hz, 2H), 6.97(s, 1H), 5.72(dd, *J*=11.1, 6.5Hz, 1H), 5.47(s, 2H), 4.90(brs, 2H), 3.64(dd, *J*=17.5, 11.1 Hz, 1H), 3.46(dd, *J*=17.5, 6.5Hz, 1H)

### Example 23. Preparation of 4-methoxybenzyl 4-amino-3-chloro-6-(3-chloro-4,5-dihydroisoxazol-5-yl)picolinate

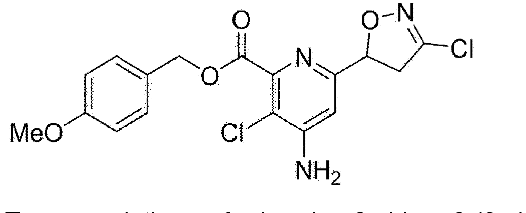

To a solution of 4-amino-3-chloro-6-(3-chloro-4,5-dihydroisoxazol-5-yl)picolinic acid (50mg, 0.18mmol) in DMF (1 ml) were added potassium carbonate (K₂CO₃) (50 mg, 0.36 mmol) and 4-methoxybenzyl chloride (30 *µℓ*, 0.23 mmol). The reaction mixture was stirred at room temperature for 3 hours and partitioned between ethyl acetate and brine. The aqueous layer was separated and extracted with ethyl acetate (x 3) and the combined organic layers were dried (MgSO₄), filtered, and concentrated under reduced pressure. The residue was purified by silica gel column chromatography (40% EtOAc/hexane) to afford the title compound (32 mg, 43%).
¹H NMR(500MHz, CDCl₃) δ 7.40(d, J=8.7Hz, 2H), 6.93(s, 1H), 6.90(d, J=8.7Hz, 2H), 5.71 (dd, J=11.1, 6.5Hz, 1H), 5.36(s, 2H), 4.86(brs, 2H), 3.81 (s, 3H), 3.62(dd, J=17.5, 11.1Hz, 1H), 3.44(dd, J=17.5, 6.5Hz, 1H)

### Example 24. Preparation of 4-bromobenzyl 4-amino-3-chloro-6-(3-chloro-4,5-dihydroisoxazol-5-yl)picolinate

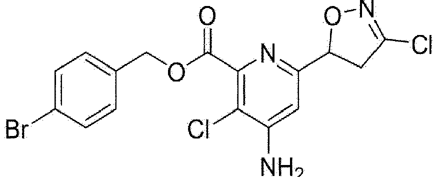

To a solution of 4-amino-3-chloro-6-(3-chloro-4,5-dihydroisoxazol-5-yl)picolinic acid(250mg, 0.91 mmol) in DMF (3 ml) were added potassium carbonate (K₂CO₃) (250 mg, 1.81 mmol) and 4-bromobenzyl bromide (339 mg, 1.36 mmol). The reaction mixture was stirred at room temperature for 3 hours and partitioned between ethyl acetate and brine. The aqueous layer was separated and extracted with ethyl acetate (x 3) and the combined organic layers were dried (MgSO₄), filtered, and concentrated under reduced pressure. The residue was purified by silica gel column chromatography (40% EtOAc/hexane) to afford the title compound (253 mg, 63%).
¹H NMR(500MHz, CDCl₃) δ 7.50(d, *J*=8.3Hz, 2H), 7.32(d, *J*=8.3Hz, 2H), 6.95(s, 1H), 5.69(dd, *J*=11.1, 6.4Hz, 1H), 5.35(s, 2H), 5.03(brs, 2H), 3.61(dd, *J*=17.3, 11.1Hz, 1H), 3.45(dd, *J*=17.3, 6.4Hz, 1H)

### Example 25. Preparation of 4-fluorobenzyl 4-amino-3-chloro-6-(3-chloro-4,5-dihydroisoxazol-5-yl)picolinate

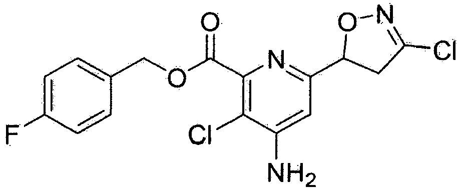

To a solution of 4-amino-3-chloro-6-(3-chloro-4,5-dihydroisoxazol-5-yl)picolinic acid(50 mg, 0.18 mmol) in DMF (1 ml) were added potassium carbonate (K₂CO₃) (50 mg, 0.36 mmol) and 4-fluorobenzyl bromide (27 *µ*ℓ, 0.22 mmol). The reaction mixture was stirred at room temperature for 3 hours and partitioned between ethyl acetate and brine. The aqueous layer was separated and extracted with ethyl acetate (x 3) and the combined organic layers were dried (MgSO₄), filtered, and concentrated under reduced pressure. The residue was purified by silica gel column chromatography (40% EtOAc/hexane) to afford the title compound (41 mg, 60%).
¹H NMR(500MHz, CDCl₃) δ 7.44(m, 2H), 7.07(m, 2H), 6.95(s, 1H), 5.71 (dd, *J*=11.2, 6.4Hz, 1H), 5.38(s, 2H), 4.90(brs, 2H), 3.62(dd, *J*=17.4, 11.2Hz, 1H), 3.45(dd, *J*=17.4, 6.4Hz, 1H)

### Example 26. Preparation of phenyl 4-amino-3-chloro-6-(3-chloro-4,5-dihydroisoxazol-5-yl)picolinate

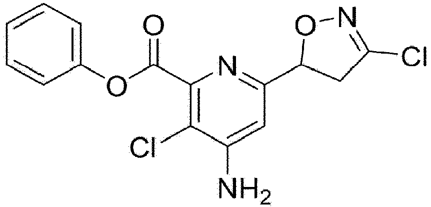

To a solution of 4-amino-3-chloro-6-(3-chloro-4,5-dihydroisoxazol-5-yl) picolinic acid (200 mg, 0.72 mmol) in tetrahydrofuran (3 ml) were added phenol (0.13 ml, 1.45 mmol), *N*-(3-dimethylaminopropyl)-*N*'-ethylcarboimide hydrochloride (278 mg, 1.45 mmol) and 4-(dimethylamino)pyridine (44 mg, 0.36 mmol). And the reaction mixture was stirred at room temperature for 16 hours. After completion of the reaction, the mixture was extracted with ethyl acetate (x 3) and the combined organic layers were washed with saturated aqueous sodium hydrogen carbonate (NaHCO₃) solution, dried (MgSO₄), filtered, and concentrated under reduced pressure. The residue was purified by silica gel column chromatography (40% EtOAc/hexane) to afford the title compound (65 mg, 26%).
¹H NMR(500MHz, CDCl₃) δ 7.44(m, 2H), 7.29(m, 3H), 7.02(s, 1H), 5.77(dd, *J*=11.2, 6.4Hz, 1H), 5.00(brs, 2H), 3.66(dd, *J*=17.6, 11.2Hz, 1H), 3.53(dd, *J*=17.6, 6.4Hz, 1H)

### Example 27. Preparation of lithium 4-amino-3-chloro-6-(3-chloro-4,5-dihydroisoxazol-5-yl)picolinate

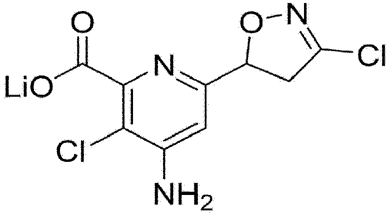

To a solution of 4-amino-3-chloro-6-(3-chloro-4,5-dihydroisoxazol-5-yl)picolinic acid (50 mg, 0.17 mmol) in tetrahydrofuran (1 ml) was added a solution of lithium hydroxide (8 mg, 0.19 mmol) in water (0.5 ml) and the reaction mixture was stirred at room temperature for 1 hour. After completion of the reaction, the mixture was concentrated under reduced pressure to afford the title compound (47 mg) without the purification by silica gel column chromatography.
¹H NMR(500MHz, CD₃OD) δ 6.78(s, 1H), 5.61 (dd, *J*=11.1, 7.3Hz, 1H), 3.63(dd, *J*=17.4, 1.11 Hz, 1H), 3.41 (dd, *J*=17.4, 7.3Hz, 1H)

### Example 28. Preparation of sodium 4-amino-3-chloro-6-(3-chloro-4,5-dihydroisoxazol-5-yl)picolinate

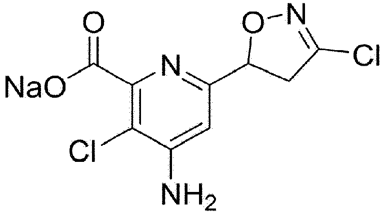

To a solution of 4-amino-3-chloro-6-(3-chloro-4,5-dihydroisoxazol-5-yl)picolinic acid (50 mg, 0.17 mmol) in tetrahydrofuran (1 ml) was added a solution of sodium hydroxide (7.6 mg, 0.19 mmol) in water (0.5 ml) and the reaction mixture was stirred at room temperature for 1 hour. After completion of the reaction, the mixture was concentrated under reduced pressure to afford the title compound (51 mg) without the purification by silica gel column chromatography.
¹H NMR(500MHz, CD₃OD) δ 6.90(s, 1H), 5.26(dd, *J*=9.6, 7.3Hz, 1H), 3.16(dd, *J*=15.6, 9.6Hz, 1H), 2.69(dd, *J*=15.6, 7.3Hz, 1H)

### Example 29. Preparation of ethyl 4-amino-6-(3-bromo-4,5-dihydroisoxazol-5-yl)-3-chloropicolinate

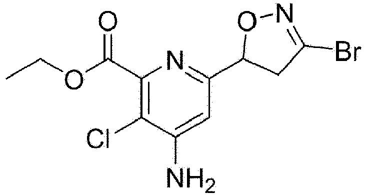

To a solution of 4-amino-3-chloro-6-(3-bromo-4,5-dihydroisoxazol-5-yl)picolinic acid (100 mg, 0.31 mmol) in DMF (1 ml) were added potassium carbonate (K₂CO₃) (65 mg, 0.47 mmol) and iodoethane (38 *µℓ*, 0.47 mmol) and the reaction mixture was stirred at room temperature for 2 hours. After completion of the reaction, the mixture was extracted with ethyl acetate (x 3) and the combined organic layers were washed with brine, dried (MgSO₄), filtered, and concentrated under reduced pressure. The residue was purified by silica gel column chromatography (40% EtOAc/hexane) to afford the title compound (105 mg, 97%).
¹H NMR(300MHz, CDCl₃) δ 6.96(s, 1H), 5.62(dd, *J*=11.1, 6.5Hz, 1H), 5.00(brs, 2H), 4.42(q, *J*=7.2Hz, 2H), 3.63(dd, *J*=17.5, 11.1Hz, 1H), 3.45(dd, *J*=17.4, 6.5Hz, 1H), 1.42(t, *J*=7.2Hz, 3H)

### Example 30. Preparation of isopropyl 4-amino-6-(3-bromo-4,5-dihydroisoxazol-5-yl)-3-chloropicolinate

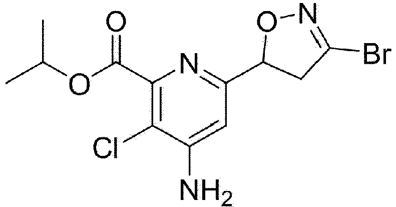

To a solution of 4-amino-3-chloro-6-(3-bromo-4,5-dihydroisoxazol-5-yl)picolinic acid (100 mg, 0.31 mmol) in DMF (1 ml) were added potassium carbonate (K₂CO₃) (65 mg, 0.47 mmol) and 2-bromopropane (44*µ*ℓ, 0.47 mmol) and the reaction mixture was stirred at room temperature for 2 hours. After completion of the reaction, the mixture was extracted with ethyl acetate (x 3) and the combined organic layers were washed with brine, dried (MgSO₄), filtered, and concentrated under reduced pressure. The residue was purified by silica gel column chromatography (40% EtOAc/hexane) to afford the title compound (81 mg, 72%).
¹H NMR(300MHz, CDCl₃) δ 6.94(s, 1H), 5.65(dd, *J*=11.1, 6.5Hz, 1H), 5.32(m, 1H), 5.00(brs, 2H), 3.67(dd, *J*=17.5, 11.1Hz, 1H), 3.51 (dd, *J*=17.4, 6.5Hz, 1H), 1.41 (d, *J*=6.3Hz, 6H)

### Example 31. Preparation of isobutyl 4-amino-6-(3-bromo-4,5-dihydroisoxazol-5-yl)-3-chloropicolinate

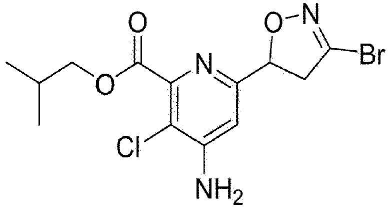

To a solution of 4-amino-3-chloro-6-(3-bromo-4,5-dihydroisoxazol-5-yl)picolinic acid (60 mg, 0.19 mmol) in DMF (1 ml) were added potassium carbonate (K₂CO₃) (65 mg, 0.47 mmol) and 1-iodo-2-methylpropane (32 *µℓ*, 0.28 mmol) and the reaction mixture was stirred at room temperature for 2 hours. After completion of the reaction, the mixture was diluted with water. The solution was extracted with ethyl acetate (x 3) and the combined organic layers were washed with brine, dried (MgSO₄), filtered, and concentrated under reduced pressure. The residue was purified by silica gel column chromatography (40% EtOAc/hexane) to afford the title compound (65 mg, 92%).
¹H NMR(500MHz, CDCl₃) δ 6.95(s, 1H), 5.65(dd, *J*=11.1, 6.4Hz, 1H), 4.95(brs, 2H), 4.18(d, *J*=6.9Hz, 2H), 3.67(dd, *J*=17.5, 11.1Hz, 1H), 3.52(dd, *J*=17.4, 6.4Hz, 1H), 2.09(m, 1H), 1.02(d, *J*=6.4Hz, 6H)

### Example 32. Preparation of benzyl 4-amino-3-chloro-6-(3-bromo-4,5-dihydroisoxazol-5-yl)picolinate

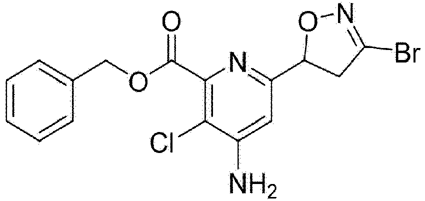

To a solution of 4-amino-3-chloro-6-(3-bromo-4,5-dihydroisoxazol-5-yl)picolinic acid (200 mg, 0.62 mmol) in DMF (3 ml) were added potassium carbonate (K₂CO₃) (129 mg, 0.94 mmol) and benzyl bromide (0.11 ml, 0.94 mmol) and the reaction mixture was stirred at room temperature for 2 hours. After completion of the reaction, the mixture was diluted with water. The solution was extracted with ethyl acetate (x 3) and the combined organic layers were washed with brine, dried (MgSO₄), filtered, and concentrated under reduced pressure. The residue was purified by silica gel column chromatography (40% EtOAc/hexane) to afford the title compound (211 mg, 82%).
¹H NMR(500MHz, CDCl₃) δ 7.4-7.46(m, 2H), 7.32-7.39(m, 3H), 6.93(s, 1H), 5.61 (dd, *J*=11.1, 6.5Hz, 1H), 5.42(s, 2H), 4.93(brs, 2H), 3.62(dd, *J*=17.5, 11.1Hz, 1H), 3.47(dd, *J*=17.5, 6.5Hz, 1H)

### Example 33. Preparation of 4-methylbenzyl 4-amino-6-(3-bromo-4,5-dihydroisoxazol-5-yl)-3-chloropicolinate

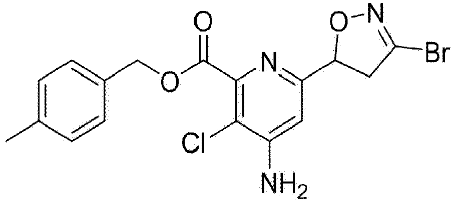

To a solution of 4-amino-3-chloro-6-(3-bromo-4,5-dihydroisoxazol-5-yl)picolinic acid (100 mg, 0.31 mmol) in DMF (1 ml) were added potassium carbonate (K₂CO₃) (65 mg, 0.47 mmol) and 4-methylbenzyl chloride (62 *µ*ℓ, 0.47 mmol) and the reaction mixture was stirred at room temperature for 4 hours. After completion of the reaction, the mixture was diluted with water. The solution was extracted with ethyl acetate (x 3) and the combined organic layers were washed with brine, dried (MgSO₄), filtered, and concentrated under reduced pressure. The residue was purified by silica gel column chromatography (40% EtOAc/hexane) to afford the title compound (123 mg, 93%).
¹H NMR(500MHz, CDCl₃) δ 7.35(d, *J*=8.0Hz, 2H), 7.18(d, *J*=8.0Hz, 2H), 6.92(s, 1H), 5.63(dd, *J*=11.1, 6.5Hz, 1H), 5.38(s, 2H), 4.88(brs, 2H), 3.65(dd, *J*=17.5, 11.1 Hz, 1H), 3.48(dd, *J*=17.5, 6.5Hz, 1H), 2.35(s, 3H)

### Example 34. Preparation of 4-trifluoromethylbenzyl 4-amino-6-(3-bromo-4,5-dihydroisoxazol-5-yl)-3-chloropicolinate

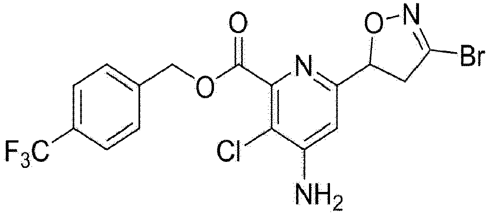

To a solution of 4-amino-3-chloro-6-(3-bromo-4,5-dihydroisoxazol-5-yl)picolinic acid (100 mg, 0.31 mmol) in DMF (1 ml) were added potassium carbonate (K₂CO₃) (65 mg, 0.47 mmol) and 4-trifluoromethylbenzyl bromide (72 *µℓ*, 0.47 mmol) and the reaction mixture was stirred at room temperature for 3 hours. After completion of the reaction, the mixture was diluted with water. The solution was extracted with ethyl acetate (x 3) and the combined organic layers were washed with brine, dried (MgSO₄), filtered, and concentrated under reduced pressure. The residue was purified by silica gel column chromatography (40% EtOAc/hexane) to afford the title compound (100 mg, 67%).
¹H NMR(500MHz, CDCl₃) δ 7.66(d, *J*=8.3Hz, 2H), 7.59(d, *J*=8.3Hz, 2H), 6.97(s, 1H), 5.66(dd, *J*=11.1, 6.5Hz, 1H), 5.48(s, 2H), 4.88(brs, 2H), 3.69(dd, *J*=17.5, 11.1Hz, 1H), 3.50(dd, *J*=17.5, 6.5Hz, 1H)

### Example 35. Preparation of 4-methoxybenzyl 4-amino-6-(3-bromo-4,5-dihydroisoxazol-5-yl)-3-chloropicolinate

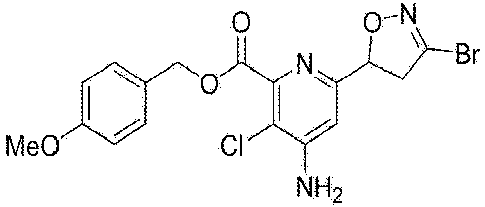

To a solution of 4-amino-3-chloro-6-(3-bromo-4,5-dihydroisoxazol-5-yl)picolinic acid (100 mg, 0.31 mmol) in DMF (1 ml) were added potassium carbonate (K₂CO₃) (65 mg, 0.47 mmol) and 4-methoxybenzyl chloride (63 *µ*ℓ, 0.47 mmol) and the reaction mixture was stirred at room temperature for 3 hours. After completion of the reaction, the mixture was diluted with water. The solution was extracted with ethyl acetate (x 3) and the combined organic layers were washed with brine, dried (MgSO₄), filtered, and concentrated under reduced pressure. The residue was purified by silica gel column chromatography (40% EtOAc/hexane) to afford the title compound (80 mg, 58%).
¹H NMR(500MHz, CDCl₃) δ 7.39(d,J=8.3Hz, 2H), 6.91(d, J=8.3Hz, 2H), 6.89(s, 1H), 5.62(dd, J=11.1, 6.5Hz, 1H), 5.35(s, 2H), 4.90(brs, 2H), 3.81(s, 3H), 3.65(dd, J=17.5, 11.1Hz, 1H), 3.48(dd, J=17.5, 6.5Hz, 1H)

### Example 36. Preparation of 4-bromobenzyl 4-amino-6-(3-bromo-4,5-dihydroisoxazol-5-yl)-3-chloropicolinate

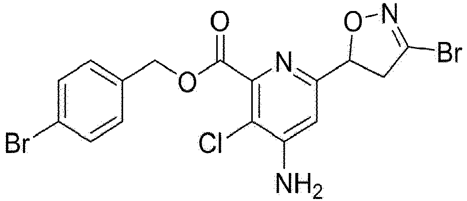

To a solution of 4-amino-3-chloro-6-(3-bromo-4,5-dihydroisoxazol-5-yl)picolinic acid (60 mg, 0.19 mmol) in DMF (1 ml) were added potassium carbonate (K₂CO₃) (39 mg, 0.28 mmol) and 4-bromobenzyl bromide (70 mg, 0.47 mmol) and the reaction mixture was stirred at room temperature for 3 hours. After completion of the reaction, the mixture was diluted with water. The solution was extracted with ethyl acetate (x 3) and the combined organic layers were washed with brine, dried (MgSO₄), filtered, and concentrated under reduced pressure. The residue was purified by silica gel column chromatography (40% EtOAc/hexane) to afford the title compound (70 mg, 76%).
¹H NMR(500MHz, CDCl₃) δ 7.52(d,*J*=8.3Hz, 2H), 7.34(d, *J*=8.3Hz, 2H), 6.96(s, 1H), 5.65(dd, *J*=11.2, 6.4Hz, 1H), 5.37(s, 2H), 4.91 (brs, 2H), 3.67(dd, *J*=17.4, 11.2Hz, 1H), 3.50(dd, *J*=17.4, 6.4Hz, 1H)

### Example 37. Preparation of 4-fluorobenzyl 4-amino-6-(3-bromo-4,5-dihydroisoxazol-5-yl)-3-chloropicolinate

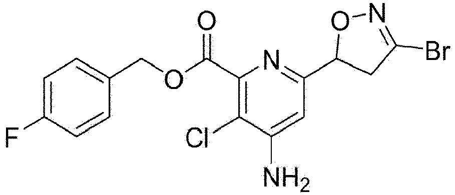

To a solution of 4-amino-3-chloro-6-(3-bromo-4,5-dihydroisoxazol-5-yl)picolinic acid (60 mg, 0.19 mmol) in DMF (1 ml) were added potassium carbonate (K₂CO₃) (39 mg, 0.28 mmol) and 4-fluorobenzyl bromide (35 *µ*ℓ, 0.47 mmol) and the reaction mixture was stirred at room temperature for 3 hours. After completion of the reaction, the mixture was diluted with water. The solution was extracted with ethyl acetate (x 3) and the combined organic layers were washed with brine, dried (MgSO₄), filtered, and concentrated under reduced pressure. The residue was purified by silica gel column chromatography (40% EtOAc/hexane) to afford the title compound (63 mg, 77%).
¹H NMR(500MHz, CDCl₃) δ 7.44(m, 2H), 7.07(m, 2H), 6.94(s, 1H), 5.63(dd, *J*=11.2, 6.4Hz, 1H), 5.38(s, 2H), 4.90(brs, 2H), 3.66(dd, *J*=17.4, 11.2Hz, 1H), 3.49(dd, *J*=17.4, 6.4Hz, 1H)

### Example 38. Preparation of phenyl 4-amino-3-chloro-6-(3-bromo-4,5-dihydroisoxazol-5-yl)picolinate

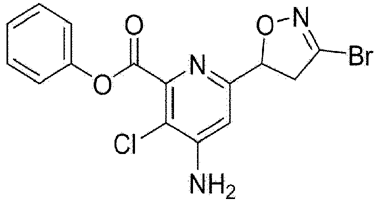

### Process 1) phenyl 4-acetamido-6-(3-bromo-4,5-dihydroisoxazol-5-yl)-3-chloropicolinate

To a solution of 4-acetamido-6-(3-bromo-4,5-dihydroisoxazol-5-yl)-3-chloropicolinic acid (390 mg, 1.08 mmol) in tetrahydrofuran (5 ml) were added phenol (0.19 ml, 2.15 mmol), *N*-(3-dimethylaminopropyl)-*N*'-ethylcarboimide hydrochloride (412 mg, 2.15 mmol), and 4-(dimethylamino)pyridine (197 mg, 1.61 mmol). And then the reaction mixture was stirred at room temperature for 16 hours. After completion of the reaction, the mixture was diluted with water. The solution was extracted with ethyl acetate (x 3) and the combined organic layers were washed with sodium hydrogen carbonate (NaHCO₃) aqueous solution, dried (MgSO₄), filtered, and concentrated under reduced pressure. The residue was purified by silica gel column chromatography (40% EtOAc/hexane) to afford the title compound (230 mg, 49%).
¹H NMR(500MHz, CDCl₃) δ 8.76(s,1H), 8.05(brs,1H), 7.45(m,2H), 7.28(m,3H), 5.75(dd, J=11.2, 6.4Hz, 1H), 3.70(dd, *J*=17.6, 11.2Hz, 1H), 3.67(dd, *J*=17.6, 6.4Hz, 1H), 2.31 (s, 3H)

### Process 2) phenyl 4-amino-3-chloro-6-(3-bromo-4,5-dihydroisoxazol-5-yl)picolinate

To a solution of phenyl 4 -acetamido-3-chloro- 6 -(3-bromo-4,5-dihydroisoxazol-5-yl)picolinate (255 mg, 0.58 mmol) in MeOH (3 ml) was slowly added BF₃-OEt₂ (0.32 ml, 2.62 mmol) at 0 °C and the reaction mixture was heated under reflux for 1 hour. After completion of the reaction, the mixture was diluted with water. The solution was extracted with ethyl acetate (x 3) and the combined organic layers were washed with sodium hydrogen carbonate (NaHCO₃) aqueous solution, dried (MgSO₄), filtered, and concentrated under reduced pressure. The residue was purified by silica gel column chromatography (40% EtOAc/hexane) to afford the title compound (204 mg, 89%).
¹H NMR(300MHz, CDCl₃) δ 7.44(m,2H), 7.30(m,3H), 7.03(s,1H), 5.69(dd, *J*=11.2, 6.4Hz, 1H), 5.01(brs, 2H), 3.71(dd, *J*=17.6, 11.2Hz, 1H), 3.57(dd, *J*=17.6, 6.4Hz, 1H)

### Example 39. Preparation of methyl 6-amino-5-chloro-2-(3-bromo-4,5-dihydroisoxazol-5-yl)pyrimidine-4-carboxylate

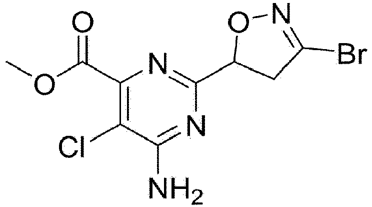

### Process 1) 2,5,6-trichloro-pyrimidine-4-carboxylic acid methyl ester

To 5-chloro-2,4-dihydroxy-6-methoxycarboxylpyrimidine (3 g, 14.67 mmol) was slowly added phosphoryl chloride (POCl₃) (55 ml, 0.59 mol) at 10 °C. After cooling to 0 °C, N,N'-diethyl aniline (3.5 ml, 22 mmol) was slowly added to the mixture, and the reaction mixture was warmed to room temperature and heated under reflux 18 hours. After completion of the reaction, the mixture was cooled to room temperature and concentrated under reduced pressure. After pouring the residue to ice water, the mixture was extracted with ethyl acetate (x 3), dried (MgSO₄), filtered, and concentrated under reduced pressure. The residue was purified by silica gel column chromatography (40% EtOAc/hexane) to afford the title compound (2.95 g, 83%).
¹H NMR(500MHz, CDCl₃) δ 4.04(s, 3H)

### Process 2) 6-amino-2,5-dichloro-pyrimidine-4-carboxylic acid methyl ester

To a solution of 2,5,6-trichloro-pyrimidine-4-carboxylic acid methyl ester (100 mg, 0.41 mmol) in tetrahydrofuran (5 ml) was added 30% aqueous ammonia (0.04 ml, 2.07 mmol) at 0 °C and the reaction mixture was stirred for 1 hour. After completion of the reaction, the mixture was filtered and concentrated under reduced pressure. The residue was washed with hexane to afford the title compound (91 mg, 99%) without the purification by silica gel column chromatography.
¹H NMR(500MHz, DMSO-*d*₆) δ 8.56(brs, 1H), 7.93(brs, 1H), 3.88(s, 3H)

### Process 3) 6-amino-5-chloro-2-vinyl-pyrimidine-4-carboxylic acid methyl ester

To a solution of 6-amino-2,5-dichloro-pyrimidine-4-carboxylic acid methyl ester (1 g, 4.05 mmol) in MeOH were added potassium trifluoro(prop-1-ene-2-yl)borate (905 mg, 6.76 mmol), [1,1'-bis(diphenylphosphino)ferrocene]dichloropalladium(II) (74 mg, 0.09 mmol), and triethylamine (0.94 ml, 6.76 mmol) at room temperature. The mixture was degassed with nitrogen for 1 hour and heated under reflux for 16 hours under nitrogen. The reaction mixture was cooled to room temperature, filtered through celite, concentrated under reduced pressure, and partitioned between ethyl acetate and water. The aqueous layer was separated and extracted with ethyl acetate (x 3) and the combined organic layers were dried (MgSO₄), filtered, and concentrated under reduced pressure. The residue was purified by silica gel column chromatography (30% EtOAc/hexane) to afford the title compound (667 mg, 69%).
¹H NMR(500MHz, CDCl₃) δ 6.69(dd, *J*=17.3, 10.45Hz, 1H), 6.50(dd, *J*=17.3, 1.92Hz, 1H), 5.74(brs, 2H), 5.69(dd, *J*=10.45, 1.29Hz, 1H), 3.99(s, 3H)

### Process 4) methyl 6-amino-5-chloro-2-(3-bromo-4,5-dihydroisoxazol-5-yl)pyrimidine-4-carboxylate

To a solution of 6-amino-5-chloro-2-vinyl-pyrimidine-4- carboxylic acid methyl ester (665 mg, 3.11 mmol) in ethyl acetate (8 ml) were added dibromoaldoxime (758 mg, 3.74 mmol) and a solution of sodium hydrogen carbonate (NaHCO₃) (654 mg, 7.78 mmol) in water (4 ml), and the reaction mixture was heated at 80 °C under reflux for 18 hours. After completion of the reaction, the mixture was partitioned between ethyl acetate and water. The aqueous layer was separated and extracted with ethyl acetate (x 3) and the combined organic layers were dried (MgSO₄), filtered, and concentrated under reduced pressure. The residue was purified by silica gel column chromatography (50% EtOAc/hexane) to afford the title compound (672 mg, 64%).
¹H NMR(300MHz, CDCl₃) δ 6.38(brs, 2H), 5.62(dd, *J*=11.1, 7.8Hz, 1H), 3.99(s, 3H), 3.70(dd, *J*=17.4, 11.1 Hz, 1H), 3.59(dd, *J*=17.4, 7.8Hz, 1H)

### Example 40. Preparation of 6-amino-5-chloro-2-(3-bromo-4,5-dihydroisoxazol-5-yl)pyrimidine-4-carboxylic acid

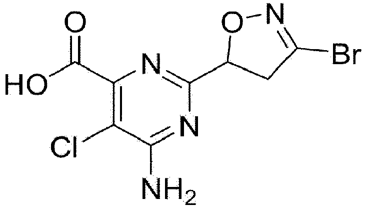

To a solution of 6-amino-2-(3-bromo-4,5-dihydroisoxazol-5-yl)-5-chloropyrimidine-4-carboxylic acid methyl ester (100 mg, 0.30 mmol) in tetrahydrofuran (2 ml) was added a solution of lithium hydroxide (19 mg, 0.45 mmol) in water (1 ml) and the reaction mixture was stirred at room temperature for 1 hour. After completion of the reaction, the mixture was acidified with 1N HCl, extracted with ethyl acetate and the organic layer was dried (MgSO₄), filtered and concentrated under reduced pressure to afford the title compound (93 mg, 97%) without the purification by silica gel column chromatography.
¹H NMR(300MHz, CD₃OD) δ 5.53(dd, *J*=11.1, 7.7Hz, 1H), 3.75(dd, *J*=17.4, 11.1Hz, 1H), 3.62(dd, *J*=17.4, 7.7Hz, 1H)

### Example 41. Preparation of methyl 6-amino-5-chloro-2-(3-chloro-4,5-dihydroisoxazol-5-yl)pyrimidine-4-carboxylate

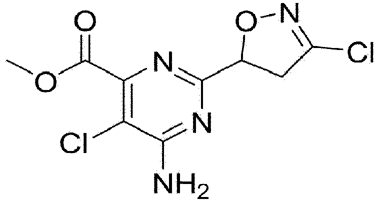

### Process 1) methyl 6-acetamido-5-chloro-2-(3-bromo-4,5-dihydroisoxazol-5-yl)pyrimidine-4-carboxylate

To a solution of 6-amino-2-(3-bromo-4,5-dihydroisoxazol-5-yl)-5-chloropyrimidine-4-carboxylic acid methyl ester (300 mg, 0.89 mmol) in toluene (3 ml) and chloroform (1 ml) were added acetic anhydride (Ac₂O) (0.10 ml, 1.07 mmol) and a catalytic amount of sulfuric acid (H₂SO₄) at room temperature, and the reaction mixture was heated at 60 °C for 16 hours. After completion of the reaction, the mixture was partitioned between ethyl acetate and water. The aqueous layer was separated and extracted with ethyl acetate (x 3) and the combined organic layers were washed with saturated aqueous sodium hydrogen carbonate (NaHCO₃) solution, dried (MgSO₄), filtered, and concentrated under reduced pressure. The residue was purified by silica gel column chromatography (30% EtOAc/hexane) to afford the title compound (203 mg, 60%).
¹H NMR(300MHz, CDCl₃) δ 8.28(brs, 1H), 5.71 (dd, *J*=10.2, 7.5Hz, 1H), 4.02(s, 3H), 3.65(m, 2H), 2.64(s, 3H)

### Process 2) methyl 6-amino-5-chloro-2-(3-chloro-4,5-dihydroisoxazol-5-yl)pyrimidine-4-carboxylate

To 6-acetylamino-2-(3-bromo-4,5-dihydroisoxazol-5-yl)-5-chloropyrimidine-4-carboxylic acid methyl ester (120 mg, 0.32 mmol) was added 4M HCl solution in dioxane (2.4 ml) and the reaction mixture was stirred at room temperature for 16 hours. After completion of the reaction, the mixture was partitioned between ethyl acetate and water. The combined organic layer was dried (MgSO₄), filtered, and concentrated under reduced pressure to afford the title compound (105 mg, 99%) without the purification by silica gel column chromatography.
¹H NMR(500MHz, CDCl₃) δ 6.28(brs, 2H), 5.69(dd, *J*=11.5, 7.8Hz, 1H), 3.99(s, 3H), 3.66(dd, *J*=17.4, 11.5Hz, 1H), 3.54(dd, *J*=17.4, 7.8Hz, 1H)

### Example 42. Preparation of 6-amino-5-chloro-2-(3-chloro-4,5-dihydroisoxazol-5-yl)pyrimidine-4-carboxylic acid

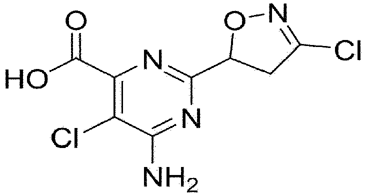

To a solution of 6-amino-2-(3-chloro-4,5-dihydroisoxazol-5-yl)-5-chloropyrimidine-4-carboxylic acid methyl ester (35 mg, 0.12 mmol) in tetrahydrofuran (0.5 ml) was added a solution of lithium hydroxide (8 mg, 0.18 mmol) in water (0.25 ml) and the reaction mixture was stirred at room temperature for 1 hours. After completion of the reaction, the mixture was acidified with 1N HCl, extracted with ethyl acetate and the organic layer was dried (MgSO₄), filtered and concentrated under reduced pressure to afford the title compound (30 mg, 90%) without the purification by silica gel column chromatography.
¹H NMR(500MHz, CD₃OD) δ 5.61(dd, J=11.1, 7.8Hz, 1H), 3.72(dd, *J*=17.4, 11.1Hz, 1H), 3.59(dd, *J*=17.4, 7.8Hz, 1H)

### Example 43. Preparation of methyl 3-chloro-6-(3-chloro-4,5-dihydroisoxazol-5-yl)-4-(methylamino)picolinate

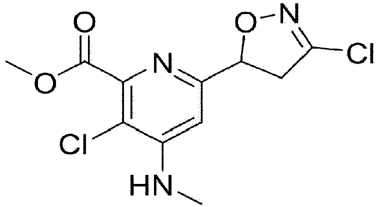

To a solution of methyl 4-amino-3-chloro-6-(3-chloro-4,5-dihydroisoxazol- 5 -yl)picolinate (150 mg, 0.45 mmol) in acetonitrile (5 ml) were added iodomethane (0.14 ml, 2.24 mmol), and cecium carbonate (Cs₂CO₃) (292 mg, 0.90 mmol) at room temperature. The mixture was heated to 80 °C under reflux for 15 hours. After completion of the reaction, the mixture was extracted with ethyl acetate and the organic layer was dried (MgSO₄), filtered and concentrated under reduced pressure. The residue was purified by silica gel column chromatography (20% EtOAc/hexane) to afford the title compound (94 mg, 69%).
¹H NMR(300MHz, CDCl₃) δ 6.84(s, 1H), 5.76(dd, *J*=11.1, 7.0Hz, 1H), 5.22(brs, 1H), 3.97(s, 3H), 3.66(dd, *J*=17.5, 11.1 Hz, 1H), 3.48(dd, *J*=17.5, 7.0Hz, 1H), 3.00(d, *J*=4.9Hz, 3H)

### Example 44. Preparation of methyl 3-chloro-6-(3-chloro-4,5-dihydroisoxazol-5-yl)-4-(dimethylamino)picolinate

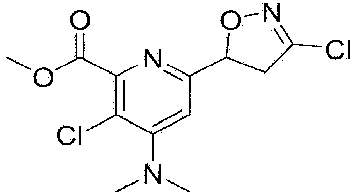

To a solution of methyl 3-chloro-6-(3-chloro-4,5-dihydroisoxazol-5-yl)-4-(methylamino)picolinate (35 mg, 0.12 mmol) in acetonitrile (1 ml) were added iodomethane (11 *µ*ℓ), and cecium carbonate (Cs₂CO₃) (112 mg, 0.34 mmol) at room temperature. The mixture was heated to 80 °C under reflux for 16 hours. After completion of the reaction, the mixture was extracted with ethyl acetate and the organic layer was dried (MgSO₄), filtered and concentrated under reduced pressure. The residue was purified by silica gel column chromatography (20% EtOAc/hexane) to afford the title compound (36 mg, 98%).
¹H NMR(300MHz, CDCl₃) δ 7.06(s, 1H), 5.76(dd, *J*=11.0, 6.9Hz, 1H), 3.98(s, 3H), 3.64(dd, *J*=17.6, 11.0Hz, 1H), 3.50(dd, *J*=17.6, 6.9Hz, 1H), 3.03(s, 6H)

### Example 45. Preparation of 3-chloro-6-(3-chloro-4,5-dihydroisoxazol-5-yl)-4-(dimethylamino)picolinic acid

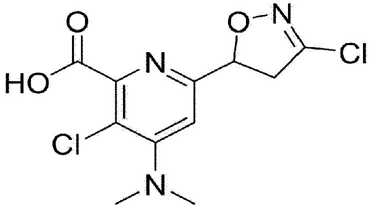

To a solution of methyl 3-chloro-6-(3-chloro-4,5-dihydroisoxazol-5-yl)-4-(dimethylamino)picolinate (40 mg, 0.13 mmol) in tetrahydrofuran (1 ml) was added a solution of lithium hydroxide (6 mg, 0.15 mmol) in water (0.5 ml) and the reaction mixture was stirred at room temperature for 1 hour. After completion of the reaction, the mixture was acidified with 1N HCl, extracted with ethyl acetate and the organic layer was dried (MgSO₄), filtered and concentrated under reduced pressure to afford the title compound (38 mg, 99%) without the purification by silica gel column chromatography.
¹H NMR(300MHz, CD₃OD) δ 7.08(s, 1H), 5.76(dd, *J*=10.9, 7.9Hz, 1H), 3.68(dd, *J*=17.3, 10.9Hz, 1H), 3.52(dd, *J*=17.3, 7.9Hz, 1H), 3.08(s, 6H)

### Example 46. Preparation of methyl 4-amino-3,5-dichloro-6-(3-chloro-4,5-dihydroisoxazol-5-yl)picolinate

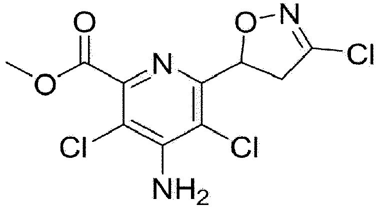

### Process 1) methyl 4-acetamido-3,5,6-trichloropicolinate

To a solution of methyl 4-acetamido-3,5,6-trichloropicolinate (4.1 g, 16.05 mmol) in toluene (65 ml) and chloroform (5 ml) were added acetic anhydride (Ac₂O) (1.8 ml, 19.26 mmol) and a catalytic amount of sulfuric acid (H₂SO₄) at room temperature, and the reaction mixture was heated at 60 °C for 16 hours. After completion of the reaction, the mixture was partitioned between ethyl acetate and water. The aqueous layer was separated and extracted with ethyl acetate (x 3) and the combined organic layers were washed with saturated aqueous sodium hydrogen carbonate (NaHCO₃) solution, dried (MgSO₄), filtered, and concentrated under reduced pressure. The residue was purified by silica gel column chromatography (30% EtOAc/hexane) to afford the title compound (4.15 g, 87%).
¹H NMR(300MHz, CDCl₃) δ 4.02(s, 3H), 2.33(s, 3H)

### Process 2) methyl 4-acetamido-3,5-dichloro-6-vinylpicolinate

To a solution of methyl 4-acetamido-3,5,6-trichloropicolinate (4 g, 13.44 mmol) in EtOH were added potassium trifluoro(prop-1-ene-2-yl)borate (2.70 g, 20.17 mmol), [1,1'-bis(diphenylphosphino)ferrocene]dichloropalladium(II) (197 mg, 0.27 mmol), and triethylamine (2.81 ml, 20.17 mmol) at room temperature. The mixture was degassed with nitrogen for 1 hour and heated under reflux for 16 hours under nitrogen. The reaction mixture was cooled to room temperature, filtered through celite, concentrated under reduced pressure, and partitioned between ethyl acetate and water. The aqueous layer was separated and extracted with ethyl acetate (x 3) and the combined organic layers were dried (MgSO₄), filtered, and concentrated under reduced pressure. The residue was purified by silica gel column chromatography (40% EtOAc/hexane) to afford the title compound (2.93 g, 75%).
¹H NMR(300MHz, CDCl₃) δ 7.53(brs, 1H), 7.15(dd, *J*=17.0Hz, 10.6Hz, 1H), 6.53(d, *J*=17.05Hz, 1H), 5.68(d, *J*=10.6Hz, 1H), 4.00(s, 3H), 2.25(s, 3H)

### Process 3) methyl 4-acetamido-3,5-dichloro-6-(3-chloro-4,5-dihydroisoxazol-5-yl)picolinate

To a solution of 2-(hydroxyimino)acetic acid (739 mg, 8.30 mmol) in DME (5 ml) was added N-chlorosuccinimide (2.22 g, 16.60 mmol), and then the reaction mixture was heated to 110 °C for 1 hour and cooled to room temperature. To the reaction mixture were added methyl 4-acetamido-3,5-dichloro-6-vinylpicolinate (2 g, 6.92 mmol) and a solution of sodium hydrogen carbonate (NaHCO₃) (1.45 g, 2.5 mmol) in water and the reaction mixture was heated at 80 °C under reflux for 18 hours. After completion of the reaction, the mixture was partitioned between ethyl acetate and water. The aqueous layer was separated and extracted with ethyl acetate (x 3) and the combined organic layers were dried (MgSO₄), filtered, and concentrated under reduced pressure. The residue was purified by silica gel column chromatography (50% EtOAc/hexane) to afford the title compound (1.78 g, 70%).
¹H NMR(300MHz, CDCl₃)δ 7.62(s, 1H), 6.17(dd, *J*=11.0, 8.0Hz, 1H), 4.07(dd, *J*=17.3, 8.0Hz, 1H), 3.99(s, 3H), 3.43(dd, *J*=17.3, 11.0Hz, 1H), 2.28(s, 3H)

### Process 4) methyl 4-amino-3,5-dichloro-6-(3-chloro-4,5-dihydroisoxazol-5-yl)picolinate

To a solution of methyl 4-acetamido-3,5-dichloro-6-(3-chloro-4,5-dihydroisoxazol-5-yl)picolinate (1 g, 2.73 mmol) in MeOH (10 ml) was slowly added acetyl chloride (1.6 ml, 21.82 mmol) at 0 °C and the reaction mixture was heated under reflux for 1 hour. After completion of the reaction, the mixture was cooled to room temperature and diluted with water. The solution was extracted with ethyl acetate (x 3) and the combined organic layers were dried (MgSO₄), filtered, and concentrated under reduced pressure. The residue was purified by silica gel column chromatography (50% EtOAc/hexane) to afford the title compound (773 mg, 87%).
¹H NMR(300MHz, CDCl₃) δ 6.10(dd, *J*=11.0, 8.5Hz, 1H), 5.33(brs, 2H), 4.06(dd, *J*=17.3, 8.0Hz, 1H), 3.98(s, 3H), 3.43(dd, *J*=17.3, 11.0Hz, 1H)

### Example 47. Preparation of 4-amino-3,5-dichloro-6-(3-chloro-4,5-dihydroisoxazol-5-yl)picolinic acid

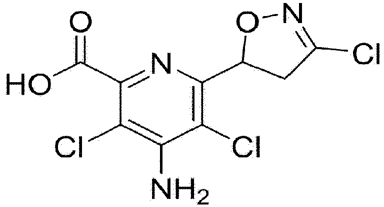

To a solution of methyl 4-amino-3,5-dichloro-6-(3-chloro-4,5-dihydroisoxazol-5-yl)picolinate (150 mg, 0.46 mmol) in tetrahydrofuran (1 ml) was added a solution of lithium hydroxide (21 mg, 0.51 mmol) in water (0.5 ml) and the reaction mixture was stirred at room temperature for 1 hour. After completion of the reaction, the mixture was acidified with 1N HCl, extracted with ethyl acetate and the organic layer was dried (MgSO₄), filtered and concentrated under reduced pressure to afford the title compound (142 mg, 98%) without the purification by silica gel column chromatography.
¹H NMR(300MHz, CD₃OD) δ 6.16(dd, *J*=11.0, 8.2Hz, 1H), 3.98(dd, *J*=17.3, 8.2Hz, 1H), 3.50(dd, *J*=17.3, 11.0Hz, 1H)

### Example 48. Preparation of methyl 4-amino-3-chloro-6-(3-chloro-4,5-dihydroisoxazol-5-yl)-5-fluoropicolinate

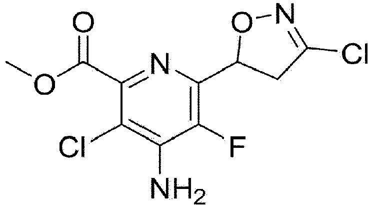

### Process 1) methyl 4-amino-3,6-dichloro-5-fluoropicolinate

To a solution of methyl 4-amino-3,6-dichloropicolinate in acetonitrile (75 ml) was added selectfluor (12.0 g, 33.93 mmol) and the reaction mixture was heated at 70°C for 7 hours. After completion of the reaction, the mixture was cooled to room temperature and diluted with water. The aqueous layer was extracted with ethyl acetate (x 3) and the organic layer was dried (MgSO₄), filtered and concentrated under reduced pressure. The residue was purified by silica gel column chromatography (30% EtOAc/hexane) to afford the title compound (3.74 g, 69%).
¹H NMR(300MHz, CDCl₃) δ 5.05(brs, 2H), 3.97(s, 3H)

### Process 2) methyl 4-acetamido-3,6-dichloro-5-fluoropicolinate

To a solution of methyl 4-amino-3,6-dichloro-5-fluoropicolinate (3.1 g, 13.2 mmol) in toluene (40 ml) and chloroform (5 ml) were added acetic anhydride (Ac₂O) (2.5 ml, 26.5 mmol) and a catalytic amount of sulfuric acid (H₂SO₄) at room temperature, and the reaction mixture was heated at 60 °C for 16 hours. After completion of the reaction, the mixture was partitioned between ethyl acetate and saturated aqueous sodium hydrogen carbonate (NaHCO₃) solution. The organic layer was dried (MgSO₄), filtered, and concentrated under reduced pressure. The residue was purified by silica gel column chromatography (30% EtOAc/hexane) to afford the title compound (2.7 g, 71%)
¹H NMR(300MHz, CDCl₃) δ 7.31 (brs, 1H), 4.01 (s, 3H), 2.31 (s, 3H)

### Process 3) methyl 4-acetamido-3-chloro-5-fluoro-6-vinylpicolinate

To a solution of methyl 4-acetamido-3,6-dichloro-5-fluoropicolinate (2.63 g, 9.39 mmol) in EtOH (40 ml) were added potassium trifluoro(prop-1-ene-2-yl)borate (1.89 g, 14.09 mmol), [1,1'-bis(diphenylphosphino)ferrocene]dichloropalladium(II) (137 mg, 0.19 mmol), and triethylamine (1.96 ml, 14.09 mmol) at room temperature. The mixture was degassed with nitrogen for 1 hour and heated under reflux for 18 hours under nitrogen. The reaction mixture was cooled to room temperature, filtered through celite, concentrated under reduced pressure, and partitioned between ethyl acetate and water. The aqueous layer was separated and extracted with ethyl acetate (x 3) and the combined organic layers were dried (MgSO₄), filtered, and concentrated under reduced pressure. The residue was purified by silica gel column chromatography (40% EtOAc/hexane) to afford the title compound (2.44 g, 95%).
¹H NMR(300MHz, CDCl₃) δ 7.29(brs, 1H), 6.94(ddd, *J*=17.3, 11.0, 1.6Hz, 1H), 6.48(dd, *J*=17.3, 1.6Hz, 1H), 3.50(dd, *J*=11.0, 1.6Hz, 1H), 4.00(s, 3H), 2.28(s, 3H)

### Process 4) methyl 4-acetamido-6-(3-bromo-4,5-dihydroisoxazol-5-yl)-3-chloro-5-fluoropicolinate

To a solution of methyl 4-acetamido-3-chloro-5-fluoro-6-vinylpicolinate (500 mg, 1.83 mmol) in ethyl acetate (3 ml) were added dibromoaldoxime (558 mg, 2.75 mmol) and a solution of sodium hydrogen carbonate (NaHCO₃) (385 mg, 4.58 mmol) in water (2 ml), and the reaction mixture was heated at 80 °C under reflux for 18 hours. After completion of the reaction, the mixture was partitioned between ethyl acetate and water. The aqueous layer was separated and extracted with ethyl acetate (x 3) and the combined organic layers were dried (MgSO₄), filtered, and concentrated under reduced pressure. The residue was purified by silica gel column chromatography (40% EtOAc/hexane) to afford the title compound (325 mg, 45%).
¹H NMR(300MHz, CDCl₃) δ 7.40(brs, 1H), 6.00(ddd, *J*=11.1, 8.3, 1.2Hz, 1H), 3.95-4.06(m, 4H), 3.53(dd, *J*=17.1, 11.1 Hz, 1H), 2.31 (s, 3H)

### Process 5) methyl 4-acetamido-6-(3-chloro-4,5-dihydroisoxazol-5-yl)-3-chloro-5-fluoropicolinate

To methyl 4-acetamido-6-(3-bromo-4,5-dihydroisoxazol-5-yl)-3-chloro-5-fluoropicolinate (310 mg, 0.79 mmol) was added 4M HCl solution in dioxane (5.9 ml) at room temperature, and the reaction mixture was heated at 40 °C for 18 hours. After completion of the reaction, the aqueous layer was separated and extracted with ethyl acetate (x 3) and the combined organic layers were dried (MgSO₄), filtered, and concentrated under reduced pressure. The residue was purified by silica gel column chromatography (40% EtOAc/hexane) to afford the title compound (240 mg, 87%).
¹H NMR(300MHz, CDCl₃) δ 7.43(s, 1H), 6.05(ddd, *J*=11.1, 8.4, 1.4Hz, 1H), 3.92-4.02(m, 4H), 3.41-3.53(m, 1H), 2.28-2.30(s, 3H)

### Process 6) methyl 4-amino-3-chloro-6-(3-chloro-4,5-dihydroisoxazol-5-yl)-5-fluoropicolinate

To a solution of methyl 4-acetamido-6-(3-chloro-4,5-dihydroisoxazol-5-yl)-3-chloro-5-fluoropicolinate (145 mg, 0.41 mmol) in MeOH (2 ml) was slowly added acetyl chloride (0.24 ml, 3.31 mmol) at 0 °C and the reaction mixture was heated under reflux for 1 hour. After completion of the reaction, the mixture was cooled to room temperature and diluted with water. The solution was extracted with ethyl acetate (x 3) and the combined organic layers were dried (MgSO₄), filtered, and concentrated under reduced pressure. The residue was purified by silica gel column chromatography (40% EtOAc/hexane) to afford the title compound (88 mg, 69%).
¹H NMR(300MHz, CDCl₃) δ 5.97(dd, *J*=11.3, 9.3Hz, 1H), 5.04(brs, 2H), 3.97(s, 3H), 3. 42(dd, *J*=17.0, 9.3Hz, 1H), 3.42(dd, *J*=17.0, 11.3Hz, 1H)

### Example 49. Preparation of 4-amino-3-chloro-6-(3-chloro-4,5-dihydroisoxazol-5-yl)-5-fluoropicolinic acid

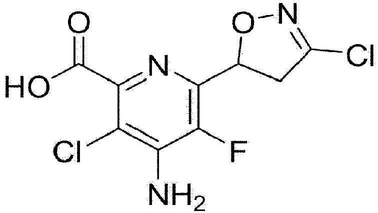

To a solution of 4-amino-3-chloro-6-(3-chloro-4,5-dihydroisoxazol-5-yl)-5-fluoropicolinate (45 mg, 0.15 mmol) in tetrahydrofuran (1 ml) was added a solution of lithium hydroxide (6.7 mg, 0.16 mmol) in water (0.5 ml) and the reaction mixture was stirred at room temperature for 1 hour. After completion of the reaction, the mixture was acidified with 1N HCl, extracted with ethyl acetate and the organic layer was dried (MgSO₄), filtered and concentrated under reduced pressure to afford the title compound (40 mg, 92%) without the purification by silica gel column chromatography.
¹H NMR(300MHz, CD₃OD) δ 6.00(ddd, *J*=11.1, 8.7, 1.1Hz, 1H), 3.88(dd, *J*=17.2, 8.7Hz, 1H), 3.55(dd, *J*=17.2, 11.1Hz, 1H)

### [Preparations]

The pyridine-based compound containing an isoxazoline ring represented by Formula 1 according to the present disclosure may be useful for use as a herbicide. Accordingly, a herbicide including, as an active ingredient, a compound selected from a compound represented by Formula 1 and an agrochemically acceptable salt thereof is included in the scope of the present disclosure.

The herbicide according to the present disclosure may be applied to whole plants or parts of plants. The term 'plant' used herein refers to all plants and plant populations, such as wild plants or crops (including wild crops), which are wanted or unwanted. 'Crops' include plant varieties and transgenic plants that can be protected or not protected by plant breeding assurances, and may be plants that are obtainable by conventional breeding and optimization methods, biotechnological and recombinant methods, or a combination of these methods. The term 'part of a plant' refers to a part or organ of a plant above or under the ground, for example, buds, leaves, flowers, and roots. For example, the term 'part of a plant' may include leaves, needles, stalks, trunks, flowers, fruit bodies, fruits, seeds, roots, tubers, and rootstalks. Some of the plants also include nutritive and reproductive material, such as seedlings, tubers, rootstalks, grafting and seeds. The applying of the herbicide according to the present disclosure into whole plants or parts of a plant may be performed in a direct contact manner or a conventional process method, such as spray, incorporation, soaking, coating, or fumigation.

In preparing a herbicidal composition including the compound represented by Formula 1 as an active ingredient, a carrier, a surfactant, a diluent, a dispersant, an adjuvant, and the like, which are conventionally used in formulating pesticides, are combined to provide a liquid or solid preparation. The liquid or solid preparation may be used directly or diluted in suitable media for treatment. The spray amount may be from several hundred liters to several thousand liters per hectare (ha).

The herbicidal composition according to an embodiment of the present disclosure may include: at least one or two kinds selected from compounds represented by Formula 1 and agrochemically acceptable salts thereof as an active ingredient, in an amount of about 0.1 wt% to about 99.9 wt%; and at least one additive selected from a surfactant, a solid diluent, and a liquid diluent, in an amount of about 0.1 wt% to about 99.9 wt%. The herbicidal composition according to an embodiment of the present disclosure may be formulated in any one selected from wettable powder, suspensions, emulsions, fine suspensions, liquids, dispersible liquids, granular wettable powder, granules, powder, liquid wettable powder, floating granules, and tablets.

Table 2 shows active ingredients and additives including surfactants and diluents, which constitute the herbicidal composition according to the present disclosure, and amounts thereof. However, the composition ratio of the herbicidal composition according to is not limited to Table 2.

**[Table 2]**

| Formulation | Content (unit: wt%) | | |
|---|---|---|---|
| | Active ingredient | Diluent | Surfactant |
| Wettable powder | 10∼90 | 0∼80 | 1∼10 |
| Suspensions | 3∼50 | 40∼95 | 0∼15 |
| Emulsions/Liquids | 3∼50 | 40∼95 | 0∼15 |
| Granules | 0.1∼95 | 4∼98.9 | 1∼15 |

The amount of the active ingredient of the herbicidal composition according to the present disclosure may be controlled according to use, and in some cases, the surfactant needs to be used in a greater amount than the active ingredient.

The surfactant included in the herbicidal composition according to the present disclosure is a substance having a large surface activity and is an amphipathic substance having a hydrophilic molecular group and a lipophilic molecular group in the molecular structure thereof. The surfactant has excellent properties in terms of detergency, dispersibility, emulsifying characteristics, solubilization characteristics, wetting characteristics, sterilization characteristics, bubbling characteristics, and penetration characteristics, and accordingly, the surfactant may disintegrate, collapse, disperse, or emulsify the active ingredient to induce drug efficacy effectively. The surfactant may include an anionic surfactant selected from a sodium salt or calcium salt of a sulfonate, such as (C₈ to C₁₂ alkyl)benzenesulfonate, (C₃ to C₆ alkyl)naphthalene sulfonate, di(C₃ to C₆ alkyl)naphthalene sulfonate, di(C₈ to C₁₂ alkyl)sulfosuccinate, lignin sulfonate, naphthalene sulfosuccinate formalin condensate, (C₈ to C₁₂ alkyl)naphthalene sulfonate formalin condensate, polyoxyethylene(C₈ to C₁₂ alkyl)phenyl sulfonate; a sodium salt or calcium salt of sulfate, such as (C₈ to C₁₂ alkyl)sulfate, polyoxyethylene(C₈ to C₁₂ alkyl)sulfate, or polyoxyethylene(C₈ to C₁₂ alkyl)phenyl sulfate; and a sodium salt or calcium salt of polyoxy alkylene succinate. The surfactant may include a non-ionic surfactant, such as polyoxyethylene(C₈ to C₁₂ alkyl)ether, polyoxyethylene(C₈ to C₁₂ alkyl)phenylether, or polyoxyethylene(C₈ to C₁₂ alkyl)phenylpolymer. The surfactants described above may be used alone or in combination of two or more. Surfactants available for the present disclosure are not limited to those exemplified above.

The diluent contained in the herbicidal composition according to the present disclosure may be classified as a solid diluent and a liquid diluent, depending on the nature of the diluent. As a solid diluent, a highly absorbing diluent is suitable for the preparation of a wetting agent. The liquid diluent and the solvent may retain their stability even at a temperature of 0 ° C without experiencing of phase separation. In one embodiment, the liquid diluent may be selected from water, toluene, xylene, petroleum ether, vegetable oil, acetone, methyl ethyl ketone, cyclohexanone, acid anhydride, acetonitrile, acetophenone, amyl acetate, 2-butanone, butyrin carbonate, chlorobenzene, cyclohexane, cyclohexanol, alkyl ester of acetic acid, diacetone alcohol, 1,2-dichloropropane, diethanolamine, p-diethyl benzene, diethylene glycol, diethylene glycol butyl ether, diethylene glycol ethyl ether, diethylene glycol methyl ether, *N,N-*dimethylformamide, dimethyl sulfoxide, 1,4-dioxane, dipropylene glycol, dipropylene glycol methyl ether, dipropylene glycol dibenzoate, diproxitol, alkyl pyrrolidone, ethyl acetate, 2-ethyl hexanol, ethylene carbonate, 1,1,1-trichloroethane, 2-heptanone, alpha-pinene, d-limonene, ethyl lactate, ethylene glycol, ethylene glycol butyl ether, ethylene glycol methyl ether, gamma-butyrolactone, glycerol, glycerol acetate, glycerol diacetate, glycerol triacetate, hexadecane, hexylene glycol, isoamyl acetate, isobornyl acetate, isooctane, isophorone, isopropyl benzene, isopropyl myristate, lactic acid, lauryl amine, mesityl oxide, methoxypropanol, methyl isoamyl ketone, methyl isobutyl ketone, methyl laurate, methyl octanoate, methyl olate, methylene chloride, *m*-xylene, *n-*hexane, *n*-octylamine, octadecanoic acid, octylamine acetate, oleic acid, oleylamine, *o*-xylene, phenol, polyethylene glycol(PEG 400), propionic acid, propyl lactate, propylene carbonate, propylene glycol, propylene glycol methyl ether, *p*-xylene, toluene, triethyl phosphate, triethylene glycol, xylene sulfonic acid, paraffin, mineral oil, trichloroethylene, perchloroethylene, ethyl acetate, amyl acetate, butyl acetate, propylene glycol methyl ether, diethylene glycol methyl ether, methanol, ethanol, isopropanol, and high molecular weight alcohol such as, amyl alcohol, tetrahydrofurfuryl alcohol, hexanol, octanol, ethylene glycol, propylene glycol, glycerol, and *N*-methyl-2-pyrrolidone. In one embodiment, the solid diluent may be selected from talc, titanium dioxide, pyroclastic clay, silica, ata perlite clay, diatomite, limestone, calcium carbonate, bentonite, calcium montmorillonite, cottonseed shell, wheatmeal, peas powder, peanuts, wood flour, walnut shells, and lignin.

The herbicidal composition may further include a small amount of other additives to prevent bubbling, caking, corrosion, and microbial production, which occur during the preparation of the herbicidal composition. The preparation method is performed by using a conventional method. In the case of liquid preparations, it is sufficient to merely mix the constituents, and the fine solid phase composition may be mixed and pulverized in a hammer or a flow mill. Suspensions may be prepared by admixing in a wet mill, and granules may be prepared by spraying an active ingredient on a granular carrier.

Preparation examples for the representative preparations using the compound according to the present disclosure will now be described in detail.

### Preparation 1: Wettable powder

The following components were completely mixed and the mixture was mixed with a liquid surfactant while the liquid surfactant was sprayed on solid components. The resultant mixture was crushed in a hammer mill to obtain a particle size of 100 *µ*m or less.
- 20 wt% of active component
- 2 wt% of dodecylphenol polyethylene glycol ether
- 4 wt% of sodium lignin sulfonate
- 6 wt% of sodium silicon aluminate
- 68 wt% of montmorillonite

### Preparation 2: Wettable powder

The following components were mixed and crushed in a hammer mill until the particle size reached to 25 *µ*m or less, and packaged.
- 80 wt% of active component
- 2 wt% of sodium alkyl naphthalene sulfonate
- 2 wt% of sodium lignin sulfonate
- 3 wt% of synthetic amorphous silica
- 13 wt% of kaolinite

### Preparation 3: Emulsions

The following components were mixed and uniformly dissolved to prepare emulsions.
- 30 wt% of active component
- 20 wt% of cyclohexanone
- 11 wt% of polyoxyethylene alkylarylether
- 4 wt% of calcium alkylbenzene sulfonate
- 35 wt% of methylnaphthalene

### Preparation 4: granules

The following components were uniformly crashed, and then, 20 parts by weight of water was added to 100 parts by weight of the resultant mixture, followed by mixing. Then, the reactant was processed into granules by using the mesh of 14 to 32 by using an extruder, and then dried to produce granules.
- 5 wt% of active component
- 2 wt% of sodium lauryl alcohol sulfate ester salt
- 5 wt% of sodium lignin sulfonate
- 2 wt% of carboxy methyl cellulose
- 16 wt% of potassium sulfate
- 70 wt% of plaster

The preparations according to the present disclosure were diluted to an appropriate concentration for practical use.

### [Use]

The pyridine-based compound containing an isoxazoline ring according to the present disclosure has a high selectivity to rice, wheat, and maize during soil treatment and foliage treatment, and selectively controls weeds including Quamoclit pennata, Abutilon theophrasti Medicus, Aeschynomene indica, Xanthium strumarium, and Catchweed bedstraw. Accordingly, the pyridine-based compound containing an isoxazoline ring is useful for farming wheat, maize, and rice.

The herbicidal composition according to the present disclosure may be used in an amount of about 10 g to about 1 kg per hectare (ha) based on the active ingredient, for example about 50 g to about 200 g, but the amount of the herbicidal composition is not limited thereto. The amount of the herbicidal composition may vary depending on factors such as the amount of weed, the degree of growth, and the preparation.

In one embodiment, the herbicidal composition according to the present disclosure may include, as the active ingredient, the compound represented by Formula 1 alone or in combination with active ingredients that are known to be active as pesticides. In one embodiment, the herbicidal composition according to the present disclosure may include, in addition to the compound represented by Formula 1, at least one herbicidal active composition selected from an acetyl-CoA carboxylase (ACCase) inhibitor, an acetolactate synthase (ALS) inhibitor, an auxin herbicide, an auxin transport inhibitor, a carotenoid biosynthesis inhibitor, an 5-enolpyruvylshikimate 3-phosphate synthase(ESPS) inhibitor, a glutamine synthetase inhibitor, a lipid biosynthesis inhibitor, a mitotic inhibitor, a protoporphyrinogen IX oxidase inhibitor, a photosynthesis inhibitor, a synergist, a growth material, a cell wall biosynthesis inhibitor, and any known herbicide, which are examples of the active ingredients that are known to be active as pesticides.

The acetyl-CoA carboxylase (ACCase) inhibitor may be cyclohexenone oxime, such as ether, alloxydim, clethodim, cloproximid, cycloxydim, cetoxydim, tralcoxydim, butroxydim, cleopoxidum, or tepaloxydim; or phenoxyphenoxypropionic acid ester, such as metamifop, cyhalofop-butyl, dichlofop-methyl, fenoxaprop-ethyl, fenoxaprop-P-ethyl, pentiaprop-ethyl, fluazifop-butyl, fluazifop-P-butyl, haloxyfop-ethoxyethyl, haloxyfop-methyl, haloxyfop-P-methyl, isoxapirifop, proppaquizafop, quizalofop-ethyl, quizalofop-P-ethyl, or quizalofop-tefuryl.

The acetolactate synthase (ALS) inhibitor may be imidazolinone, such as imazapyr, imazaquin, imazamethabenz-methyl, imazamox, imazapic, imazethapyr, or imazamethapyr; pyrimidylether, such as pyrithiobac acid, pyrithiobac-sodium, bispyribac sodium or pyribenzoxim; sulfonamide, such as florasulam, flumetsulam, or methosulam; or sulfonylurea, such as amidosulfuron, azimulsulfuron, bensulfuron-methyl, chlorimuron-ethyl, chlorsulfuro, cinosulfuron, cyclosulfamuron, ethametsulfuron-methyl, ethoxysulfuron, flazasulfuron, halosulfuron-methyl, imazosulfuron, methsulfuron-methyl, nicosulfuron, primisulfuron-methyl, prosulfuron, pyrazosulfuron-ethyl, rimsulfuron, sulfometuron-methyl, thiophenesulfuron-methyl, triasulfuron, tribenuron-methyl, triflusulfuron-methyl, sulfosulfuron, flucetosulfuron, or iodosulfuron.

For the oxine herbicide, as pyridine carboxylic acid, clofyralide, picloram, 2,4-D or benazolin may be used.

The auxin transport inhibitor may be naptalam or diflufenzopyr, and the carotenoid biosynthesis inhibitor may be benzofenap, clomazone, diflufenican, fluorochloridone, fluridone, pyrazolinate, pyrazoxyfene, isoxaflutole, isoxachlortole, mesotrion, sulcotrion(chlormesulon), ketospyradox, flurtamone, norflurazone, or amitrole.

The 5-enolpyruvylshikimate 3-phosphate synthase (ESPS) inhibitor may be glyphosate or sulfocate, and the glutamine synthetase may be bilanafos(bialafos) or glufosinate-ammonium.

The lipid biosynthesis inhibitor may be anilide, such as anilofos or mefenacet; chloroacetanilide, such as dimethenamide, S-dimethenamide, acetochlor, alachlor, butachlor, buthenachlor, diethatyl-ethyl, dimethachlor, methazachlor, metholachlor, S-metholachlor, pretilachlor, propachlor, prinachlor, therbuchlor, thenylchlor or xylachlor; or thiourea, such as butylate, cycloate, dialate, dimepiperate, EPTC, esprocarb, molinate, febulate, prosulfocarb, thiobencarb(benthiocarb), tri-alate or bernolate, benfulecate, or perfluidone.

The mitotic inhibitor may be carbamate, such as asulam, carbetamid, chloropropam, orbencarb, pronamide(propizamide), propam, or thiocarbzyl; dinitroanilin, such as benefin, butraline, dinitramine, ethalfluraline, fluchloralin, orizaline, pendimethalin, prodiamine, or trifluraline; or pyridine, such as dithiopyr, or thiazopyr, butamifos, chlortal-dimethyl(DCPA), or maletic hydrazid.

The protoporphyrinogene IX oxidase inhibitor may be diphenyl ether, such as acifluorfen, acifluorfen-sodium, aclonifene, bifenox, chlornitropene (CNP), ethoxyphen, fluorodifen, fluoro glycophene-ethyl, fomesafen, furyloxyphen, lactophen, nitrofen, nitrofluorpen, or oxyfluorpen; oxadiazol, such as oxadiargyl or oxadiazon; cyclic imide, such as azaphenidine, butaphenacyl, carpentrazone-ethyl, cinidon-ethyl, flumicholac-pentyl, flumioxazin, flumipropin, flupropacyl, fluthiacet-methyl, sulpentrazon, or thidiazimin; or pyrazol, such as pyraflufen-ethyl.

The photosynthesis inhibitor may be propanil, pyridate pyridapol; benzothiadiazinone, such as bentazone; dinitrophenol, such as bromofenoxim, dinoseb, dinoseb-acetate, dinoterb, or DNOC; dipyridylene, such as ciferquart-chloride, difenzoquat-methylsufate, diquat, or paraquat-dichloride; urea, such as chlorbromuron, chlorotoluron, diphenoxuron, dimefuron, diuron, ethidimuron, fenuron, fluometuron, isoproturon, isouron, linuron, methabenzthiazuron, methazole, methobenzuron, methoxuron, monolinuron, neburon, ciduron, or tebuthiuron; phenol, such as bromoxynil or ioxynil, chloridazon; triazine, such as ametrin, atrazine, cyanazine, desmethrin, dimethamethrin, hexazinone, prometone, promethrin, propazin, cimazine, cimetrin, terbumeton, terbutrin, terbutylazine, or trietazine; triazinone, such as metamitron or metribuzine; uracil, such as bromacil, lenacil, or terbacil; or biscarbamate, such as desmedipham or phenmedipham.

The synergist may be oxyrane, such as tridipan, the growth material may be aryloxyalkansanic acid, such as 2,4-DB, chlomeprop, dichlorprop, dichlorprop-P(2,4-DP-P), fluoroxypyr, MCPA, MCPB, mecoprop, mecoprop-P, or trichlopyr; benzoic acid, such as chloramben, or dicamba; or quinolinecarboxylic acid, such as quinclorac or quinmerac, and the cell wall biosynthesis inhibitor may be isoxabene or dichlorbenil.

The known herbicide may be dichloropropionic acid, such as dalapon; dihydrobenzofuran, such as ethofumesate; phenylacetic acid, such as chlorfenac (fenac); or aziprotrin, barban, vensulide, benzthiazuron, benzofluoro, buminafos, buthidazole, buturon, cafenstrole, chlorbufam, chlorfenprop-methyl, chloroxuron, cinmethylin, cumyluron, cycluron, cyprazine, cyprazole, dibenzyluron, dipropetryn, dymron, eglinazine-ethyl, endothal, ethiozin, flucarbazone, fluorbentranil, flupoxam, isocarbamid, isopropalin, carbutylate, mefluidide, monuron, napropamid, naproanilide, nitralin, oxacyclomefone, phenisopham, piperofos, procyazine, profluraline, pyributicarb, secbumeton, sulpharate (CDEC), terbucarb, triaziflam, triaphenamide or trimeturon, or an environmentally friendly salt of these.

The compounds according to the present disclosure have significant herbicidal activities against harmful plants of dicotyledonous plants. There are several representative examples of weeds that can be controlled by the compounds. However, such weeds are not limited thereto.

The following is specific examples of weed species that can be killed by herbicides according to the present disclosure.

Examples of *dicotyledonous* weeds include *Cyperaceae* weeds, such as *Cyperus amuricus, Cyperus microiria, Cyperus rotundus L., Cyperus serotinus Rottb., Eleocharis acicularis for. longiseta, Scirpus juncoides Roxb., Eleocharis Kuroguwai Ohwi, or Scirpus fluviatilis;* Compositae weeds, such as *Eclipta prostrata (L.) L., Siegesbeckia pubescens, Centipeda minima, Senecio vulgaris, Artemisia princeps Pampanini, Bidens frondosa L., Taraxacum officinale, Erigeron annuus (L.) Pers., Erigeron canadensis L.,* Galinsoga ciliata (Raf.) Blake, Hemistepta lyrata (Bunge) Fisch.&C.A.Mey., *Lapsana humilis, Artemisia capillaris Thunb., Bidens bipinnata L., Siegesbeckia glabrescens MAKINO., Ambrosia trifida L., Ixeris dentata NAKAI, Crepidiastrum sonchifolium (Bunge) Pak & Kawano, Lactuca indica var. laciniata (O. Kuntze) Hara, Aster pilosus, Breea segeta, Taraxacum platycarpum,* Ambrosia artemisiifolia var. elatior, or *Helianthus tuberosus, Erechtites hieracifolia Raf.;* Labiatae weeds, such as *Elscholtzia patrini GARCK., Stachys japonica Miq., Mosla punctulata (J. F. Gmel.) Nakai,* or *Leonurus sibiricus L*.; Euphorbiaceae weeds, such as *Acalypha australis L., Euphorbia maculata L.,* or *Chamaesyce supina MOLD.;* Scrophulariaceae weeds, such as *Mazus pumilus (Burm. fil.) van Steenis,* or Lindernia procumbens (Krock.) Borbas; Solanaceae weeds, such as *Solanum nigrum L.,* or *Solanum americanum Mill.;* Amaranthaceae weeds, such as *Amaranthus lividus,* or *Amaranthus patulus Bertol.;* Oxalidaceae weeds, such as Oxalis corniculata or *Oxalis stricta; Geraniaceae* weeds, such as *Geranium sibiricum,* or *Geranium thunbergii Siebold & Zucc.;* Malvaceae weeds, such as *Hibiscus trionum,* or *Abutilon theophrasti Medicus;* Cannabaceae weeds, such as *Humulus japonicus Siebold & Zucc.,* or *Cannabis sativa; Onagraceae* weeds, such as *Ludwigia prostrata Roxb.* or *Oenothera biennis L.;* Portulacaceae weeds, such as *Portulaca oleracea L.;* Equisetaceae weeds, such as *Equisetum arvense L.;* Araceae weeds, such as *Pinellia ternata; Umbelliferae*/*Apiaceae* weeds, such as *Torilis japonica (Houttuyn) DC.; Aizoaceae* weeds, such as *Mollugo pentaphylla;* Commelinaceae weeds, such as *Commelina communis; Crassulaceae* weeds, such as *Sedum sarmentosum; Papaveraceae* weeds, such as *Chelidonium majus var. asiat;* Asclepiadaceae weeds, such as *Metaplexis japonica; Violaceae* weeds, such as *Viola mandshurica; Caryophyllaceae* weeds, such as *Stellaria aquatica;* Urticaceae weeds, such as *Pilea mongolica; Boraginaceae* weeds, such as *Trigonotis peduncularis; Plantaginaceae* weeds, such as *Plantago asiatica L.;* Rosaceae weeds, such as *Potentilla supina; and Pontederiaceae* weeds, such as *Monochoria vaginalis var. plantaginea (Roxb.)* Solms.

### Experimental Example 1. Herbicidal activity test regarding pre-weeds emergence treatment

A rectangular plastic pot with a surface area of 300 cm² was filled with sandy loam soil and mixed soil at a ratio of 1:1, and four kinds of weeds including Quamoclit pennata, Abutilon theophrasti Medicus, and Aeschynomene indica, Xanthium strumarium were sown therein. In another pot made in the same way, three kinds of crop seeds including maize, wheat, and rice were sown. The pots were watered and one day later, a medicament was sprayed thereon. The spraying was performed by using a track sprayer (R&D Sprayer, USA) equipped with Teejet 8002EVS (Spraying Systems Co., USA) nozzle, and the spray amount was controlled to be 1000 L/ha. A spray solution was prepared by dissolving the compound of each example or a control material in acetone, and then, adding the same amount of 0.1%(v/v) tween 20 aqueous solution thereto. The spray amount was 200 g/ha. The pots were placed in a greenhouse in which the temperature was maintained at 25 °C to 30 °C during the day and 15 °C to 25 °C during the night, and was regularly watered. After 2 to 3 weeks from the spraying of the compounds or the control material, the efficacy and the damages on each weed and each crop were assessed according to the scale of 0-10 (0: no effect, 10: complete death) scale and the results are shown in Table 3 below. The compounds represented by formula 1 according to the present disclosure showed high selectivity to rice, wheat and maize and showed strong herbicidal activity on weeds.

**[Table 3]**

| Compound | PRE | | | | | | |
|---|---|---|---|---|---|---|---|
| | ZEAMX | TRZAW | ORYSA | IPOAN | ABUTH | AESIN | XANST |
| 1 | 0 | 1 | 0 | 9 | 8 | 10 | 9 |
| 3 | 0 | 0 | 0 | 8 | 10 | 10 | 8 |
| 4 | 0 | 0 | 0 | 7 | 6 | 8 | 6 |
| 7 | 0 | 0 | 0 | 9 | 6 | 9 | 9 |
| 37 | 0 | 0 | 0 | 10 | 10 | 10 | 10 |
| 38 | 0 | 2 | 7 | 10 | 10 | 10 | 10 |
| 39 | 0 | 0 | 1 | 6 | 4 | 8 | 6 |
| fluroxypyr | 3 | 3 | 8 | 9 | 7 | 9 | 9 |

| | | | | | | | |
|---|---|---|---|---|---|---|---|
| ZEAMX: maize, TRZAW: wheat, ORYSA: rice, IPOAN: Quamoclit pennata, ABUTH: Abutilon theophrasti Medicus, AESIN: Aeschynomene indica, XANST: Xanthium strumarium | | | | | | | |

Pots for crops and weeds were prepared in the same manner as the test method illustrated in Table 3, and three kinds of weeds selected from Quamoclit pennata, Abutilon theophrasti Medicus, and Aeschynomene indica, Xanthium strumarium were used. The following treatment and evaluation methods were the same as described above and the results are shown in Table 4 below. The compounds represented by formula 1 according to the present disclosure showed high selectivity to rice, wheat and maize and showed strong herbicidal activity on weeds.

**[Table 4]**

| Compound | PRE | | | | | | |
|---|---|---|---|---|---|---|---|
| | ZEAMX | TRZAW | ORYSA | IPOAN | ABUTH | AESIN | XANST |
| 33 | 2 | 0 | 0 | 6 | 6 | 10 | 10 |
| 34 | 0 | 0 | 0 | 10 | 9 | 10 | 10 |
| 37 | 0 | 0 | 1 | 7 | 10 | 10 | 10 |
| 53 | 0 | 0 | 0 | 6 | 10 | 10 | 9 |
| 54 | 0 | 0 | 0 | 5 | 10 | 10 | 8 |
| 73 | 0 | 0 | 0 | 6 | 10 | 10 | 9 |
| 83 | 0 | 0 | 0 | 5 | 0 | 5 | 2 |
| 84 | 0 | 0 | 1 | 5 | 4 | 10 | 10 |
| 85 | 0 | 0 | 0 | 6 | 8 | 10 | 10 |
| 86 | 0 | 0 | 0 | 3 | 3 | 6 | 2 |
| 87 | 0 | 0 | 0 | 7 | 6 | 9 | 5 |
| 88 | 0 | 0 | 0 | 3 | 5 | 10 | 9 |
| 89 | 0 | 0 | 0 | 8 | 8 | 10 | 10 |
| 90 | 0 | 0 | 0 | 3 | 3 | 10 | 7 |
| 91 | 0 | 0 | 0 | 2 | 10 | 6 | 6 |
| 94 | 2 | 0 | 7 | 10 | 10 | 10 | 10 |
| 95 | 0 | 0 | 6 | 0 | 10 | 10 | 10 |
| fluroxypyr | 4 | 3 | 8 | 9 | 7 | 10 | 9 |

| | | | | | | | |
|---|---|---|---|---|---|---|---|
| ZEAMX: maize, TRZAW: wheat, ORYSA: rice, IPOAN: Quamoclit pennata, ABUTH: Abutilon theophrasti Medicus, AESIN: Aeschynomene indica, XANST: Xanthium strumarium | | | | | | | |

### Experimental Example 2. Herbicidal activity test regarding post-weeds emergence treatment

Pots for crops and weeds were prepared in the same manner as in Experimental Example 1, and then, when the crops and the weeds were raised up to 2 weeks roughly on a third-leaf stage, the compounds and the control material were sprayed thereon in the same manner as described above. The spray amount was 200 g/ha. After 2 to 3 weeks from the spraying of the test substance or the control substance, the efficacy and the damages on each weed and each crop were assessed according to the scale of 0-10 (0: no effect, 10: complete death) scale. The results are shown in Table 5 below.

The compounds represented by formula 1 according to the present disclosure showed high selectivity to rice, wheat and maize and showed strong herbicidal activity on weeds.

**[Table 5]**

| Compound | POST | | | | | | |
|---|---|---|---|---|---|---|---|
| | ZEAMX | TRZAW | ORYSA | IPOAN | ABUTH | AESIN | XANST |
| 1 | 0 | 1 | 1 | 8 | 8 | 10 | 10 |
| 3 | 0 | 0 | 0 | 6 | 3 | 10 | 10 |
| 4 | 0 | 0 | 0 | 3 | 3 | 10 | 8 |
| 7 | 0 | 0 | 0 | 4 | 2 | 5 | 6 |
| 37 | 0 | 1 | 1 | 8 | 10 | 10 | 10 |
| 38 | 1 | 0 | 3 | 9 | 10 | 10 | 10 |
| 39 | 0 | 0 | 0 | 4 | 3 | 10 | 10 |
| fluroxypyr | 0 | 3 | 4 | 8 | 10 | 10 | 10 |

| | | | | | | | |
|---|---|---|---|---|---|---|---|
| ZEAMX: maize, TRZAW: wheat, ORYSA: rice, IPOAN: Quamoclit pennata, ABUTH: Abutilon theophrasti Medicus, AESIN: Aeschynomene indica, XANST: Xanthium strumarium | | | | | | | |

Pots for crops and weeds were prepared in the same manner as the test method illustrated in Table 5, and four kinds of weeds selected from Quamoclit pennata, Abutilon theophrasti Medicus, and Aeschynomene indica, Xanthium strumarium were used. The spray amount was 200 g/ha for crops and 50 g/ha for weeds. The following treatment and evaluation methods were the same as described above and the results are shown in Table 6 below. The compounds represented by formula 1 according to the present disclosure showed high selectivity to rice, wheat and maize and showed strong herbicidal activity on weeds.

**[Table 6]**

| Compound | POST | | | | | | |
|---|---|---|---|---|---|---|---|
| | ZEAMX | TRZAW | ORYSA | IPOAN | ABUTH | AESIN | XANST |
| 33 | 0 | 0 | 0 | 5 | 7 | 9 | 10 |
| 34 | 0 | 0 | 2 | 5 | 6 | 10 | 10 |
| 37 | 0 | 0 | 0 | 8 | 10 | 10 | 10 |
| 53 | 0 | 1 | 0 | 4 | 6 | 9 | 9 |
| 54 | 0 | 0 | 0 | 5 | 4 | 6 | 8 |
| 73 | 0 | 1 | 0 | 4 | 6 | 9 | 9 |
| 83 | 0 | 0 | 0 | 5 | 4 | 5 | 7 |
| 84 | 0 | 0 | 0 | 6 | 6 | 9 | 10 |
| 85 | 0 | 0 | 0 | 5 | 5 | 9 | 10 |
| 86 | 0 | 0 | 3 | 5 | 4 | 2 | 8 |
| 87 | 1 | 0 | 0 | 4 | 6 | 9 | 10 |
| 88 | 0 | 0 | 0 | 6 | 5 | 8 | 10 |
| 89 | 0 | 0 | 0 | 5 | 6 | 8 | 9 |
| 90 | 0 | 0 | 0 | 4 | 5 | 7 | 10 |
| 91 | 0 | 0 | 0 | 5 | 5 | 6 | 10 |
| 94 | 0 | 0 | 3 | 8 | 10 | 10 | 10 |
| 95 | 1 | 1 | 1 | 8 | 10 | 10 | 10 |
| fluroxypyr | 3 | 0 | 3 | 6 | 7 | 3 | 10 |

| | | | | | | | |
|---|---|---|---|---|---|---|---|
| ZEAMX: maize, TRZAW: wheat, ORYSA: rice, IPOAN: Quamoclit pennata, ABUTH: Abutilon theophrasti Medicus, AESIN: Aeschynomene indica, XANST: Xanthium strumarium | | | | | | | |

## Claims

1. A compound selected from a pyridine-based compound containing an isoxazoline ring represented by Formula 1 and an agrochemically acceptable salt thereof: 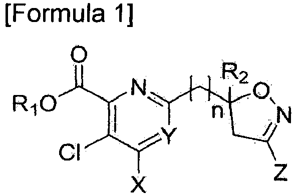
wherein, in Formula 1, R₁ is H, a C₁ to C₄ alkyl group, or a benzyl group;
R₂ is H or a C₁ to C₂ alkyl group;
n is an integer of 0 to 3;
X is a halogen or -NR₃R₄ wherein R₃ and R4 are each independently H, a C₁ to C₂ alkyl group, or a C(O)methyl group;
Y is CH, C-halogen, or N; and
Z is H, halogen, a C₁ to C₄ alkyl group, a C₁ to C₄ halo alkyl group, a C₁ to C₄ alkoxy group, a C₁ to C₄ alkoxy carbonyl group, a phenyl group substituted with at least one R₅ (wherein R₅ is halogen, a C₁ to C₄ alkyl group, a C₁ to C₄ alkoxy group, or a C₁ to C₄ halo alkyl group), or a heterocyclic group wherein a heterocyclic group is a 5-membered or 6-membered saturated or unsaturated ring containing at least one selected from N, O, and S.

2. The compound of claim 1, wherein R₁ is H, a methyl group, an ethyl group, or a benzyl group;
R₂ is H or a methyl group;
n is an integer of 0 to 1;
X is Cl or -NR₃R₄ wherein R₃ and R₄ are each independently H, a C₁ to C₂ alkyl group, or a C(O)methyl group;
Y is CH, CCI, CF, or N; and
Z is H, Br, Cl, a C₁ to C₄ alkyl group, a C₁ to C₄ halo alkyl group, a C₁ to C₄ alkoxy group, a C₁ to C₄ alkoxy carbonyl group, a phenyl group substituted with at least one R₅ wherein R₅ is F, Cl, or a methoxy group, or a heterocyclic group selected from

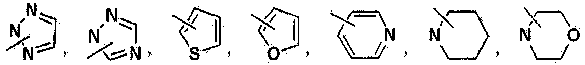

3. The compound of claim 1, wherein
R₁ is H, a methyl group, an ethyl group, or a benzyl group;
R₂ is H or a methyl group;
n is an integer of 0 to 1;
X is -NR₃R₄ wherein R₃ and R₄ are each independently H, a methyl group, or a C(O)methyl group;
Y is CH, CCI, CF, or N; and
Z is H, Br, Cl, a methyl group, a tert-butyl group, or a trifluoromethyl group.

4. The compound of claim 1, wherein the compound is any one compound selected from
methyl 4-amino-3-chloro-6-(3-chloro-4,5-dihydroisoxazol-5-yl)picolinate;
4-amino-3-chloro-6-(3-chloro-4,5-dihydroisoxazol-5-yl)picolinic acid;
methyl 4-amino-3-chloro-6-(3-methyl-4,5-dihydroisoxazol-5-yl)picolinate;
4-amino-3-chloro-6-(3-methyl-4,5-dihydroisoxazol-5-yl)picolinic acid;
methyl 4-amino-3-chloro-6-(3-bromo-4,5-dihydroisoxazol-5-yl)picolinate;
4-amino-3-chloro-6-(3-bromo-4,5-dihydroisoxazol-5-yl)picolinic acid;
methyl 4-amino-3-chloro-6-(3-trifluoromethyl-4,5-dihydroisoxazol-5-yl)picolinate;
4-amino-3-chloro-6-(3-trifluoromethyl-4,5-dihydroisoxazol-5-yl)picolinic acid;
methyl 4-amino-3-chloro-6-(3-tert-butyl-4,5-dihydroisoxazol-5-yl)picolinate;
4-amino-3-chloro-6-(3-tert-butyl-4,5-dihydroisoxazol-5-yl)picolinic acid;
methyl 4-amino-3-chloro-6-(4,5-dihydroisoxazol-5-yl)picolinate;
methyl 4-amino-3-chloro-6-(3-bromo-5-methyl-4,5-dihydroisoxazol-5-yl)picolinate;
4-amino-3-chloro-6-(3-bromo-5-methyl-4,5-dihydroisoxazol-5-yl)picolinic acid;
methyl 4-amino-3-chloro-6-(3-chloro-5-methyl-4,5-dihydroisoxazol-5-yl)picolinate;
methyl 4-amino-6-((3-chloro-4,5-dihydroisoxazol-5-yl)methyl)-3-chloropicolinate;
4-amino-6-((3-chloro-4,5-dihydroisoxazol-5-yl)methyl)-3-chloropicolinic acid;
ethyl 4-amino-3-chloro-6-(3-chloro-4,5-dihydroisoxazol-5-yl)picolinate;
isopropyl 4-amino-3-chloro-6-(3-chloro-4,5-dihydroisoxazol-5-yl)picolinate;
isobutyl 4-amino-3-chloro-6-(3-chloro-4,5-dihydroisoxazol-5-yl)picolinate;
benzyl 4-amino-3-chloro-6-(3-chloro-4,5-dihydroisoxazol-5-yl)picolinate;
4-methylbenzyl 4-amino-3-chloro-6-(3-chloro-4,5-dihydroisoxazol-5-yl)picolinate;
4-trifluoromethylbenzyl 4-amino-3-chloro-6-(3-chloro-4,5-dihydroisoxazol-5-yl)picolinate;
4-methoxybenzyl 4-amino-3-chloro-6-(3-chloro-4,5-dihydroisoxazol-5-yl)picolinate;
4-bromobenzyl 4-amino-3-chloro-6-(3-chloro-4,5-dihydroisoxazol-5-yl)picolinate;
4-fluorobenzyl 4-amino-3-chloro-6-(3-chloro-4,5-dihydroisoxazol-5-yl)picolinate;
phenyl 4-amino-3-chloro-6-(3-chloro-4,5-dihydroisoxazol-5-yl) picolinate;
lithium 4-amino-3-chloro-6-(3-chloro-4,5-dihydroisoxazol-5-yl)picolinate;
sodium 4-amino-3-chloro-6-(3-chloro-4,5-dihydroisoxazol-5-yl)picolinate;
ethyl 4-amino-6-(3-bromo-4,5-dihydroisoxazol-5-yl)-3-chloropicolinate;
isopropyl 4-amino-6-(3-bromo-4,5-dihydroisoxazol-5-yl)-3-chloropicolinate;
isobutyl 4-amino-6-(3-bromo-4,5-dihydroisoxazol-5-yl)-3-chloropicolinate;
benzyl 4-amino-3-chloro-6-(3-bromo-4,5-dihydroisoxazol-5-yl)picolinate;
4-methylbenzyl 4-amino-6-(3-bromo-4,5-dihydroisoxazol-5-yl)-3-chloropicolinate;
4-trifluoromethylbenzyl 4-amino-6-(3-bromo-4,5-dihydroisoxazol-5-yl)-3-chloropicolinate;
4-methoxybenzyl 4-amino-6-(3-bromo-4,5-dihydroisoxazol-5-yl)-3-chloropicolinate;
4-bromobenzyl 4-amino-6-(3-bromo-4,5-dihydroisoxazol-5-yl)-3-chloropicolinate;
4-fluorobenzyl 4-amino-6-(3-bromo-4,5-dihydroisoxazol-5-yl)-3-chloropicolinate;
phenyl 4-amino-3-chloro-6-(3-bromo-4,5-dihydroisoxazol-5-yl)picolinate;
methyl 6-amino-5-chloro-2-(3-bromo-4,5-dihydroisoxazol-5-yl)pyrimidine-4-carboxylate;
6-amino-5-chloro-2-(3-bromo4,5-dihydroisoxazol-5-yl)pyrimidine-4-carboxylic acid;
methyl 6-amino-5-chloro-2-(3-chloro-4,5-dihydroisoxazol-5-yl)pyrimidine-4-carboxylate;
6-amino-5-chloro-2-(3-chloro-4,5-dihydroisoxazol-5-yl)pyrimidine-4-carboxylic acid;
methyl 3-chloro-6-(3-chloro-4,5-dihydroisoxazol-5-yl)-4-(methylamino)picolinate;
methyl 3-chloro-6-(3-chloro-4,5-dihydroisoxazol-5-yl)-4-(dimethylamino)picolinate;
3-chloro-6-(3-chloro-4,5-dihydroisoxazol-5-yl)-4-(dimethylamino)picolinic acid;
methyl 4-amino-3,5-dichloro-6-(3-chloro-4,5-dihydroisoxazol-5-yl)picolinate;
4-amino-3,5-dichloro-6-(3-chloro-4,5-dihydroisoxazol-5-yl)picolinic acid;
methyl 4-amino-3-chloro-6-(3-chloro-4,5-dihydroisoxazol-5-yl)-5-fluoropicolinate;
4-amino-3-chloro-6-(3-chloro-4,5-dihydroisoxazol-5-yl)-5-fluoropicolinic acid; and
agrochemically acceptable salts thereof.

5. A herbicide comprising, as an active ingredient, the compound of any one of claims 1-4, the agrochemically acceptable salt thereof, or a combination of the compound and the agrochemically acceptable salt thereof.

6. The herbicide of claim 5, wherein the herbicide has selectivity to a cultivated crop, and is used to remove a weed having broad leaves during pre-emergence of weeds or post-emergence of weeds.

7. The herbicide of claim 6, wherein the cultivated crop is selected from maize, wheat, and rice, and the weed having broad leaves is selected from Quamoclit pennata, Abutilon theophrasti Medicus, Aeschynomene indica, Xanthium strumarium, and Catchweed bedstraw.

8. A herbicide composition comprising, as an active ingredient, a compound selected from a pyridine-based compound containing an isoxazoline ring represented by Formula 1 and an agrochemically acceptable salt thereof, or a combination of the pyridine-based compound containing an isoxazoline ring represented by Formula 1 and the agrochemically acceptable salt thereof, in an amount of about 0.1 wt% to about 99.9 wt%; and
at least one additive selected from a surfactant, a solid diluent, and a liquid diluent, in an amount of about 0.1 wt% to about 99.9 wt%. 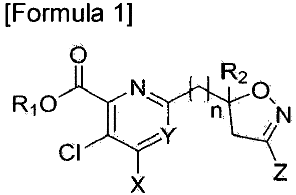
wherein, in Formula 1, R₁ is H, a C₁ to C₄ alkyl group, or a benzyl group;
R₂ is H or a C₁ to C₂ alkyl group;
n is an integer of 0 to 3;
X is a halogen or -NR₃R₄ wherein R₃ and R₄ are each independently H, a C₁ to C₂ alkyl group, or a C(O)methyl group;
Y is CH, C-halogen, or N; and
Z is H, halogen, a C₁ to C₄ alkyl group, a C₁ to C₄ halo alkyl group, a C₁ to C₄ alkoxy group, a C₁ to C₄ alkoxy carbonyl group, a phenyl group substituted with at least one R₅ wherein R₅ is halogen, a C₁ to C₄ alkyl group, a C₁ to C₄ alkoxy group, or a C₁ to C₄ halo alkyl group, or a heterocyclic group wherein a heterocyclic group is a 5-membered or 6-membered saturated or unsaturated ring containing at least one selected from N, O, and S.

9. The herbicide composition of claim 8, wherein the herbicidal composition is prepared into any one formulation selected from wettable powder, suspensions, emulsions, fine suspensions, liquids, dispersible liquids, granular wettable powder, granules, powder, liquid wettable powder, floating granules, and tablets.

10. The herbicide composition of claim 8, further comprising, in addition to the active ingredient, at least one component selected from an acetyl-CoA carboxylase (ACCase) inhibitor, an acetolactate synthase (ALS) inhibitor, an auxin herbicide, an auxin transport inhibitor, a carotenoid biosynthesis inhibitor, an 5-enolpyruvylshikimate 3-phosphate synthase (ESPS) inhibitor, a glutamine synthetase inhibitor, a lipid biosynthesis inhibitor, a mitotic inhibitor, a protoporphyrinogen IX oxidase inhibitor, a photosynthesis inhibitor, a synergist, a growth material, a cell wall biosynthesis inhibitor, and any known herbicide.

11. A method of preparing the pyridine-based compound containing an isoxazoline ring represented by Formula 1 by reacting a compound represented by Formula 2 with a compound represented by Formula 3, 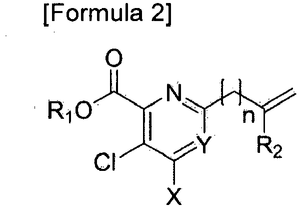 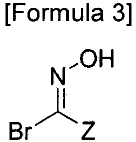 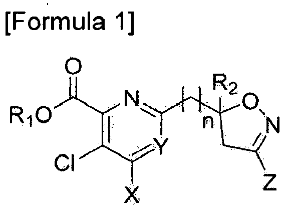 R₁, R₂, n, X, Y, and Z in Formulae 1, 2, and 3 are the same as described in claim 1.

12. The method of claim 11, the method comprising Reaction Scheme 1 in which, under base conditions, a compound represented by Formula 2 in which a 6th position is substituted with a vinyl group or an allyl group is reacted with a bromo oxime compound represented by Formula 3:

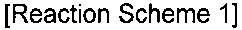

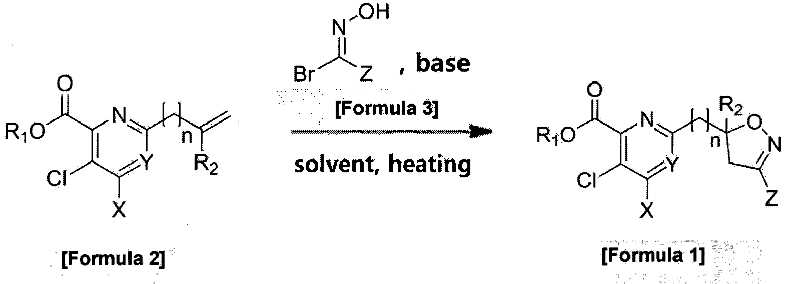

## Patentansprüche

1. Verbindung, ausgewählt aus Pyridin-basierender Verbindung, enthaltend einen Isoxazolinring, repräsentiert durch Formel 1 und ein agrochemisch verträgliches Salz davon: 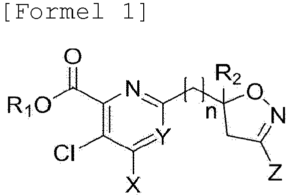
wobei in Formel 1 R₁ H, eine C₁-C₄-Alkylgruppe oder eine Benzylgruppe ist;
R₂ H oder eine C₁-C₂-Alkylgruppe ist;
n eine ganze Zahl von 0 bis 3 ist;
X ein Halogen oder -NR₃R₄ ist, wobei R₃ und R₄ jeweils unabhängig H, eine C₁-C₂-Alkylgruppe oder eine C (O) Methylgruppe sind;
Y CH, C-Halogen oder N ist und
Z H, Halogen, eine C₁-C₄-Alkylgruppe, eine C₁-C₄-Halogenalkylgruppe, eine C₁-C₄-Alkoxygruppe, eine C₁-C₄-Alkoxycarbonylgruppe, eine Phenylgruppe, substituiert mit mindestens einem R₅ (wobei R₅ ein Halogen, eine C₁-C₄-Alkylgruppe, eine C₁-C₄-Alkoxygruppe oder eine C₁-C₄-Halogenalkylgruppe ist) oder eine heterocyclische Gruppe ist, wobei eine heterocyclische Gruppe ein 5-gliedriger oder ein 6-gliedriger gesättigter oder ungesättigter Ring ist, enthaltend mindestens eines ausgewählt aus N, O und S.

2. Verbindung gemäß Anspruch 1, wobei R₁ H, eine Methylgruppe, eine Ethylgruppe oder eine Benzylgruppe ist;
R₂ H oder eine Methylgruppe ist;
n eine ganze Zahl von 0 bis 1 ist;
X Cl oder -NR₃R₄ ist, wobei R₃ und R₄ jeweils unabhängig H, eine C₁-C₂-Alkylgruppe oder eine C(O)Methylgruppe sind;
Y CH, CCl, CF oder N ist und
Z H, Br, Cl, eine C₁-C₄-Alkylgruppe, eine C₁-C₄-Halogenalkylgruppe, eine C₁-C₄-Alkoxygruppe, eine C₁-C₄-Alkoxycarbonylgruppe, eine Phenylgruppe, substituiert mit mindestens einem R₅, wobei R₅ F, Cl oder eine Methoxygruppe ist, oder eine heterocyclische Gruppe, ausgewählt aus 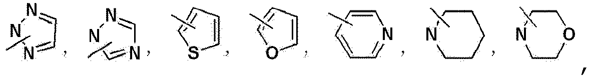 ist.

3. Verbindung gemäß Anspruch 1, wobei
R₁ H, eine Methylgruppe, eine Ethylgruppe oder eine Benzylgruppe ist;
R₂ H oder eine Methylgruppe ist;
n eine ganze Zahl von 0 bis 1 ist;
X -NR₃R₄ ist, wobei R₃ und R₄ jeweils unabhängig H, eine Methylgruppe oder eine C(O)Methylgruppe sind;
Y CH, CCl, CF oder N ist und
Z H, Br, Cl, eine Methylgruppe, eine tert-Butylgruppe oder eine Trifluormethylgruppe ist.

4. Verbindung gemäß Anspruch 1, wobei die Verbindung eine beliebige Verbindung ist, ausgewählt aus:
Methyl-4-amino-3-chlor-6-(3-chlor-4,5-dihydroisoxazol-5-yl)picolinat;
4-Amino-3-chlor-6-(3-chlor-4,5-dihydroisoxazol-5-yl)picolinsäure;
Methyl-4-amino-3-chlor-6-(3-methyl-4,5-dihydroisoxazol-5-yl)picolinat;
4-Amino-3-chlor-6-(3-methyl-4,5-dihydroisoxazol-5-yl)picolinsäure;
Methyl-4-amino-3-chlor-6-(3-brom-4,5-dihydroisoxazol-5-yl)picolinat;
4-Amino-3-chlor-6-(3-brom-4,5-dihydroisoxazol-5-yl)picolinsäure;
Methyl-4-amino-3-chlor-6-(3-trifluormethyl-4,5-dihydroisoxazol-5-yl)picolinat;
4-Amino-3-chlor-6-(3-trifluormethyl-4,5-dihydroisoxazol-5-yl)picolinsäure;
Methyl-4-amino-3-chlor-6-(3-tert-butyl-4,5-dihydroisoxazol-5-yl)picolinat;
4-Amino-3-chlor-6-(3-tert-butyl-4,5-dihydroisoxazol-5-yl)picolinsäure;
Methyl-4-amino-3-chlor-6-(4,5-dihydroisoxazol-5-yl)picolinat;
Methyl-4-amino-3-chlor-6-(3-brom-5-methyl-4,5-dihydroisoxazol-5-yl)picolinat;
4-Amino-3-chlor-6-(3-brom-5-methyl-4,5-dihydroisoxazol-5-yl)picolinsäure;
Methyl-4-amino-3-chlor-6-(3-chlor-5-methyl-4,5-dihydroisoxazol-5-yl)picolinat;
Methyl-4-amino-6-((3-chlor-4,5-dihydroisoxazol-5-yl)methyl)-3-chlorpicolinat;
4-Amino-6-((3-chlor-4,5-dihydroisoxazol-5-yl)methyl)-3-chlorpicolinsäure;
Ethyl-4-amino-3-chlor-6-(3-chlor-4,5-dihydroisoxazol-5-yl)picolinat;
Isopropyl-4-amino-3-chlor-6-(3-chlor-4,5-dihydroisoxazol-5-yl)picolinat;
Isobutyl-4-amino-3-chlor-6-(3-chlor-4,5-dihydroisoxazol-5-yl)picolinat;
Benzyl-4-amino-3-chlor-6-(3-chlor-4,5-dihydroisoxazol-5-yl)picolinat;
4-Methylbenzyl-4-amino-3-chlor-6-(3-chlor-4,5-dihydroisoxazol-5-yl)picolinat;
4-Trifluormethylbenzyl-4-amino-3-chlor-6-(3-chlor-4,5-dihydroisoxazol-5-yl)picolinat;
4-Methoxybenzyl-4-amino-3-chlor-6-(3-chlor-4,5-dihydroisoxazol-5-yl)picolinat;
4-Brombenzyl-4-amino-3-chlor-6-(3-chlor-4,5-dihydroisoxazol-5-yl)picolinat;
4-Fluorbenzyl-4-amino-3-chlor-6-(3-chlor-4,5-dihydroisoxazol-5-yl)picolinat;
Phenyl-4-amino-3-chlor-6-(3-chlor-4,5-dihydroisoxazol-5-yl)picolinat;
Lithium-4-amino-3-chlor-6-(3-chlor-4,5-dihydroisoxazol-5-yl)picolinat;
Natrium-4-amino-3-chlor-6-(3-chlor-4,5-dihydroisoxazol-5-yl)picolinat;
Ethyl-4-amino-6-(3-brom-4,5-dihydroisoxazol-5-yl)-3-chlorpicolinat;
Isopropyl-4-amino-6-(3-brom-4,5-dihydroisoxazol-5-yl)-3-chlorpicolinat;
Isobutyl-4-amino-6-(3-brom-4,5-dihydroisoxazol-5-yl)-3-chlorpicolinat;
Benzyl-4-amino-3-chlor-6-(3-brom-4,5-dihydroisoxazol-5-yl)picolinat;
4-Methylbenzyl-4-amino-6-(3-brom-4,5-dihydroisoxazol-5-yl)-3-chlorpicolinat;
4-Trifluormethylbenzyl-4-amino-6-(3-brom-4,5-dihydroisoxazol-5-yl)-3-chlorpicolinat;
4-Methoxybenzyl-4-amino-6-(3-brom-4,5-dihydroisoxazol-5-yl)-3-chlorpicolinat;
4-Brombenzyl-4-amino-6-(3-brom-4,5-dihydroisoxazol-5-yl)-3-chlorpicolinat;
4-Fluorbenzyl-4-amino-6-(3-brom-4,5-dihydroisoxazol-5-yl)-3-chlorpicolinat;
Phenyl-4-amino-3-chlor-6-(3-brom-4,5-dihydroisoxazol-5-yl)picolinat;
Methyl-6-amino-5-chlor-2-(3-brom-4,5-dihydroisoxazol-5-yl)pyrimidin-4-carboxylat;
6-Amino-5-chlor-2-(3-brom-4,5-dihydroisoxazol-5-yl)pyrimidin-4-carbonsäure;
Methyl-6-amino-5-chlor-2-(3-chlor-4,5-dihydroisoxazol-5-yl)pyrimidin-4-carboxylat;
6-Amino-5-chlor-2-(3-chlor-4,5-dihydroisoxazol-5-yl)pyrimidin-4-carbonsäure;
Methyl-3-chlor-6-(3-chlor-4,5-dihydroisoxazol-5-yl)-4-(methylamino)picolinat;
Methyl-3-chlor-6-(3-chlor-4,5-dihydroisoxazol-5-yl)-4-(dimethylamino)picolinat;
3-Chlor-6-(3-chlor-4,5-dihydroisoxazol-5-yl)-4-(dimethylamino)picolinsäure;
Methyl-4-amino-3,5-dichlor-6-(3-chlor-4,5-dihydroisoxazol-5-yl)picolinat;
4-Amino-3,5-dichlor-6-(3-chlor-4,5-dihydroisoxazol-5-yl)picolinsäure;
Methyl-4-amino-3-chlor-6-(3-chlor-4,5-dihydroisoxazol-5-yl)-5-fluorpicolinat;
4-Amino-3-chlor-6-(3-chlor-4,5-dihydroisoxazol-5-yl)-5-fluorpicolinsäure und
agrochemisch verträgliche Salze davon.

5. Herbizid umfassend, als aktiver Inhaltsstoff, die Verbindung gemäß einem der Ansprüche 1 bis 4, das agrochemisch verträgliche Salz davon oder eine Kombination der Verbindung und des agrochemisch verträglichen Salzes davon.

6. Herbizid gemäß Anspruch 5, wobei das Herbizid Selektivität bezüglich einer Kulturpflanze besitzt und verwendet wird um Unkraut, welches breite Blätter hat, vor und nach dem Pflanzenaufgang des Unkrauts zu entfernen.

7. Herbizid gemäß Anspruch 6, wobei die Kulturpflanze ausgewählt ist aus Mais, Weizen und Reis und das Unkraut, welches breite Blätter hat, ausgewählt ist aus Quamoclit pennata, Abutilon theophrasti Medicus, Aeschynomene indica, Xanthium strumarium und Kletten-Labkraut.

8. Herbizidzusammensetzung, umfassend als aktiver Inhaltsstoff eine Verbindung, ausgewählt aus einer Pyridin-basierten Verbindung enthaltend einen Isoxazolinring, repräsentiert durch Formel 1 und ein agrochemisch verträgliches Salz davon oder eine Kombination der Pyridin-basierten Verbindung enthaltend einen Isoxazolinring, repräsentiert durch Formel 1, und das agrochemisch verträgliche Salz davon, in einer Menge von ungefähr 0,1 Gew.-% bis ungefähr 99,9 Gew.-% und
mindestens einen Zusatzstoff, ausgewählt aus einem Tensid, einem festen Verdünnungsstoff und einem flüssigen Verdünnungsstoff in einer Menge von ungefähr 0,1 Gew.-% bis ungefähr 99,9 Gew.-%. 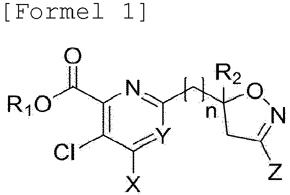
wobei in Formel 1 R₁ H, eine C₁-C₄-Alkylgruppe oder eine Benzylgruppe ist;
R₂ H oder eine C₁-C₂-Alkylgruppe ist;
n eine ganze Zahl von 0 bis 3 ist;
X ein Halogen oder -NR₃R₄ ist, wobei R₃ und R₄ jeweils unabhängig H, eine C₁-C₂-Alkylgruppe oder eine C (O) Methylgruppe sind;
Y CH, C-Halogen oder N ist; und
Z H, Halogen, eine C₁-C₄-Alkylgruppe, eine C₁-C₄-Halogenalkylgruppe, eine C₁-C₄-Alkoxygruppe, eine C₁-C₄-Alkoxycarbonylgruppe, eine Phenylgruppe, substituiert mit mindestens einem R₅, wobei R₅ ein Halogen, eine C₁-C₄-Alkylgruppe, eine C₁-C₄-Alkoxygruppe oder eine C₁-C₄-Halogenalkylgruppe ist, oder eine heterocyclische Gruppe ist, wobei eine heterocyclische Gruppe ein 5-gliedriger oder ein 6-gliedriger gesättigter oder ungesättigter Ring ist, enthaltend mindestens eines ausgewählt aus N, O und S.

9. Herbizidzusammensetzung gemäß Anspruch 8, wobei die Herbizidzusammensetzung hergestellt wird in eine beliebige Formulierung, ausgewählt aus benetzbarem Pulver, Suspensionen, Emulsionen, feinen Suspensionen, Flüssigkeiten, dispergierbaren Flüssigkeiten, körnigem benetzbaren Pulver, Granulat, Pulver, flüssigem benetzbaren Pulver, schwimmendem Granulat und Tabletten.

10. Herbizidzusammensetzung gemäß Anspruch 8, zusätzlich umfassend, zusätzlich zum aktiven Inhaltsstoff, mindestens eine Komponenten, ausgewählt aus einem Acetyl-CoA-Carboxylase (ACCase)-Inhibitor, einem Acetolactatsynthase (ALS)-Inhibitor, einem Auxinherbizid, einem Auxintransportinhibitor, einem Carotenoid-Biosynthese-Inhibitor, einem 5-Enolpyruvylshikimat-3-Phosphatsynthase (ESPS)-Inhibitor, einem Glutamin-Synthetase-Inhibitor, einem Lipid-Biosynthese-Inhibitor, einem mitotischen Inhibitor, einem Protoporphyrinogen-IX-Oxidase-Inhibitor, einem Photosynthese-Inhibitor, einem Synergisten, einem Wachstumsmaterial, einem Zellwand-Biosynthese-Inhibitor und jeden bekannten Herbizid.

11. Verfahren zur Herstellung der Pyrimidin-basierten Verbindung enthaltend einen Isoxazolinring, repräsentiert durch Formel 1, durch Umsetzen einer Verbindung, repräsentiert durch Formel 2, mit einer Verbindung, repräsentiert durch Formel 3, 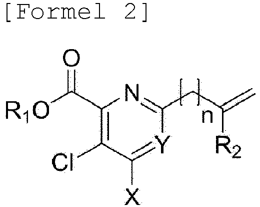 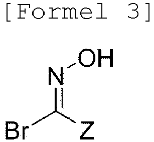 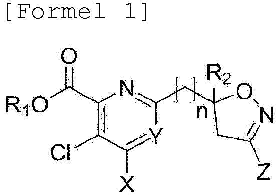 R₁, R₂, n, X, Y und Z in Formel 1, 2 und 3 sind dieselben wie in Anspruch 1 beschrieben.

12. Verfahren gemäß Anspruch 11, wobei das Verfahren umfasst: das Reaktionsschema 1, in welchem unter basischen Bedingungen eine Verbindung repräsentiert durch Formel 2, in welcher eine sechste Position mit einer Vinylgruppe oder einer Allylgruppe substituiert ist, mit einer Bromoxim-Verbindung, repräsentiert durch Formel 3, umgesetzt wird:

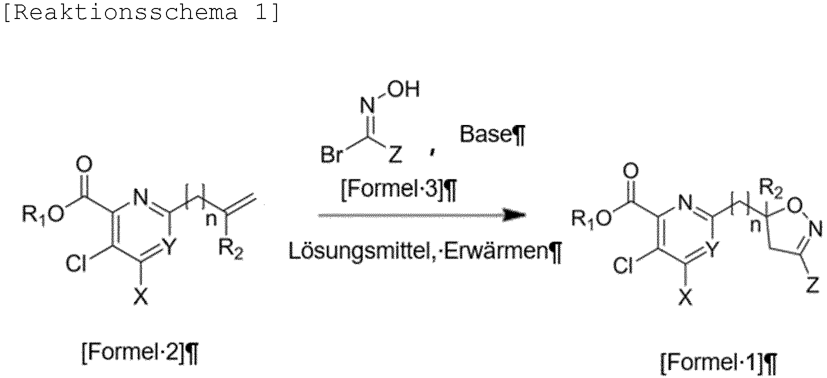

## Revendications

1. Composé choisi parmi un composé à base de pyridine contenant un cycle isoxazoline représenté par la Formule 1 et un sel agrochimiquement acceptable de celui-ci : 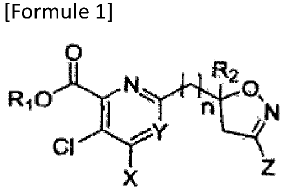
dans lequel, dans la Formule 1, R₁ représente H, un groupe alkyle en C₁ à C₄, ou un groupe benzyle ;
R₂ représente H ou un groupe alkyle en C₁ à C₂ ;
n représente un nombre entier de 0 à 3 ;
X représente un halogène ou -NR₃R₄ dans lequel
R₃ et R₄ représentent chacun indépendamment H, un groupe alkyle en C₁ à C₂, ou un groupe C(O)méthyle ;
Y représente CH, C-halogène, ou N ; et
Z représente H, un halogène, un groupe alkyle en C₁ à C₄, un groupe haloalkyle en C₁ à C₄, un groupe alcoxy en C₁ à C₄, un groupe alcoxycarbonyle en C₁ à C₄, un groupe phényle substitué par au moins un R₅ (où R₅ représente un halogène, un groupe alkyle en C₁ à C₄, un groupe alcoxy en C₁ à C₄ ou un groupe haloalkyle en C₁ à C₄), ou un groupe hétérocyclique dans lequel
un groupe hétérocyclique est un cycle saturé ou insaturé à 5 ou 6 chaînons contenant au moins un élément choisi parmi N, O et S.

2. Composé selon la revendication 1, dans lequel R₁ représente H, un groupe méthyle, un groupe éthyle, ou un groupe benzyle ;
R₂ représente H ou un groupe méthyle ;
n représente un nombre entier de 0 à 1 ;
X représente Cl ou -NR₃R₄ où
R₃ et R₄ représentent chacun indépendamment H, un groupe alkyle en C₁ à C₂, ou un groupe C(O)méthyle ;
Y représente CH, CCl, CF ou N ; et
Z représente H, Br, Cl, un groupe alkyle en C₁ à C₄, un groupe haloalkyle en C₁ à C₄, un groupe alcoxy en C₁ à C₄, un groupe alcoxycarbonyle en C₁ à C₄, un groupe phényle substitué par au moins un R₅ où R₅ représente F, Cl ou un groupe méthoxy, ou un groupe hétérocyclique choisi parmi

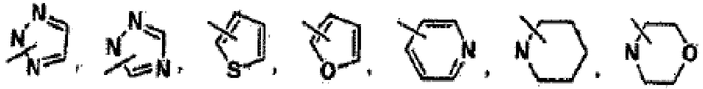

3. Composé selon la revendication 1, dans lequel
R₁ représente H, un groupe méthyle, un groupe éthyle, ou un groupe benzyle ;
R₂ représente H ou un groupe méthyle ;
n représente un nombre entier de 0 à 1 ;
X représente -NR₃R₄ où
R₃ et R₄ représentent chacun indépendamment H, un groupe méthyle, ou un groupe C(O)méthyle ;
Y représente CH, CCl, CF ou N ; et
Z représente H, Br, Cl, un groupe méthyle, un groupe tert-butyle, ou un groupe trifluorométhyle.

4. Composé selon la revendication 1, dans lequel le composé est un composé quelconque choisi parmi
le 4-amino-3-chloro-6-(3-chloro-4,5-dihydroisoxazol-5-yl)picolinate de méthyle ;
l'acide 4-amino-3-chloro-6-(3-chloro-4,5-dihydroisoxazol-5-yl)picolinique ;
le 4-amino-3-chloro-6-(3-méthyl-4,5-dihydroisoxazol-5-yl)picolinate de méthyle ;
l'acide 4-amino-3-chloro-6-(3-méthyl-4,5-dihydroisoxazol-5-yl)picolinique ;
le 4-amino-3-chloro-6-(3-bromo-4,5-dihydroisoxazol-5-yl)picolinate de méthyle ;
l'acide 4-amino-3-chloro-6-(3-bromo-4,5-dihydroisoxazol-5-yl)picolinique ;
le 4-amino-3-chloro-6-(3-trifluorométhyl-4,5-dihydroisoxazol-5-yl)picolinate de méthyle ;
l'acide 4-amino-3-chloro-6-(3-trifluorométhyl-4,5-dihydroisoxazol-5-yl)picolinique ;
le 4-amino-3-chloro-6-(3-tert-butyl-4,5-dihydroisoxazol-5-yl)picolinate de méthyle ;
l'acide 4-amino-3-chloro-6-(3-tert-butyl-4,5-dihydroisoxazol-5-yl)picolinique ;
le 4-amino-3-chloro-6-(4,5-dihydroisoxazol-5-yl)picolinate de méthyle ;
le 4-amino-3-chloro-6-(3-bromo-5-méthyl-4,5-dihydroisoxazol-5-yl)picolinate de méthyle ;
l'acide 4-amino-3-chloro-6-(3-bromo-5-méthyl-4,5-dihydroisoxazol-5-yl)picolinique ;
le 4-amino-3-chloro-6-(3-chloro-5-méthyl-4,5-dihydroisoxazol-5-yl)picolinate de méthyle ;
le 4-amino-6-((3-chloro-4,5-dihydroisoxazol-5-yl)méthyl)-3-chloropicolinate de méthyle ;
l'acide 4-amino-6-((3-chloro-4,5-dihydroisoxazol-5-yl)méthyl)-3-chloropicolinique ;
le 4-amino-3-chloro-6-(3-chloro-4,5-dihydroisoxazol-5-yl)picolinate d'éthyle ;
le 4-amino-3-chloro-6-(3-chloro-4,5-dihydroisoxazol-5-yl)picolinate d'isopropyle ;
le 4-amino-3-chloro-6-(3-chloro-4,5-dihydroisoxazol-5-yl)picolinate d'isobutyle ;
le 4-amino-3-chloro-6-(3-chloro-4,5-dihydroisoxazol-5-yl)picolinate de benzyle ;
le 4-amino-3-chloro-6-(3-chloro-4,5-dihydroisoxazol-5-yl)picolinate de 4-méthylbenzyle ;
le 4-amino-3-chloro-6-(3-chloro-4,5-dihydroisoxazol-5-yl)picolinate de 4-trifluorométhylbenzyle ;
le 4-amino-3-chloro-6-(3-chloro-4,5-dihydroisoxazol-5-yl)picolinate de 4-méthoxybenzyle ;
le 4-amino-3-chloro-6-(3-chloro-4,5-dihydroisoxazol-5-yl)picolinate de 4-bromobenzyle ;
le 4-amino-3-chloro-6-(3-chloro-4,5-dihydroisoxazol-5-yl)picolinate de 4-fluorobenzyle ;
le 4-amino-3-chloro-6-(3-bromo-4,5-dihydroisoxazol-5-yl)picolinate de phényle ;
le 4-amino-3-chloro-6-(3-chloro-4,5-dihydroisoxazol-5-yl)picolinate de lithium ;
le 4-amino-3-chloro-6-(3-chloro-4,5-dihydroisoxazol-5-yl)picolinate de sodium ;
le 4-amino-6-(3-bromo-4,5-dihydroisoxazol-5-yl)-3-chloropicolinate d'éthyle ;
le 4-amino-6-(3-bromo-4,5-dihydroisoxazol-5-yl)-3-chloropicolinate d'isopropyle ;
le 4-amino-6-(3-bromo-4,5-dihydroisoxazol-5-yl)-3-chloropicolinate d'isobutyle ;
le 4-amino-3-chloro-6-(3-bromo-4,5-dihydroisoxazol-5-yl)picolinate de benzyle ;
le 4-amino-6-(3-bromo-4,5-dihydroisoxazol-5-yl)-3-chloropicolinate de 4-méthylbenzyle ;
le 4-amino-6-(3-bromo-4,5-dihydroisoxazol-5-yl)-3-chloropicolinate de 4-trifluorométhylbenzyle ;
le 4-amino-6-(3-bromo-4,5-dihydroisoxazol-5-yl)-3-chloropicolinate de 4-méthoxybenzyle ;
le 4-amino-6-(3-bromo-4,5-dihydroisoxazol-5-yl)-3-chloropicolinate de 4-bromobenzyle ;
le 4-amino-6-(3-bromo-4,5-dihydroisoxazol-5-yl)-3-chloropicolinate de 4-fluorobenzyle ;
le 4-amino-3-chloro-6-(3-bromo-4,5-dihydroisoxazol-5-yl)picolinate de phényle ;
le 6-amino-5-chloro-2-(3-bromo-4,5-dihydroisoxazol-5-yl)pyrimidine-4-carboxylate de méthyle ;
l'acide 6-amino-5-chloro-2-(3-bromo-4,5-dihydroisoxazol-5-yl)pyrimidine-4-carboxylique ;
le 6-amino-5-chloro-2-(3-chloro-4,5-dihydroisoxazol-5-yl)pyrimidine-4-carboxylate de méthyle ;
l'acide 6-amino-5-chloro-2-(3-chloro-4,5-dihydroisoxazol-5-yl)pyrimidine-4-carboxylique ;
le 3-chloro-6-(3-chloro-4,5-dihydroisoxazol-5-yl)-4-(méthylamino)picolinate de méthyle ;
le 3-chloro-6-(3-chloro-4,5-dihydroisoxazol-5-yl)-4-(diméthylamino)picolinate de méthyle ;
l'acide 3-chloro-6-(3-chloro-4,5-dihydroisoxazol-5-yl)-4-(diméthylamino)picolinique ;
le 4-amino-3,5-dichloro-6-(3-chloro-4,5-dihydroisoxazol-5-yl)picolinate de méthyle ;
l'acide 4-amino-3,5-dichloro-6-(3-chloro-4,5-dihydroisoxazol-5-yl)picolinique ;
le 4-amino-3-chloro-6-(3-chloro-4,5-dihydroisoxazol-5-yl)-5-fluoropicolinate de méthyle ;
l'acide 4-amino-3-chloro-6-(3-chloro-4,5-dihydroisoxazol-5-yl)-5-fluoropicolinique ; et
des sels agrochimiquement acceptable de ceux-ci.

5. Herbicide comprenant, en tant qu'ingrédient actif, le composé selon l'une quelconque des revendications 1 à 4, le sel agrochimiquement acceptable de celui-ci, ou une combinaison du composé et le sel agrochimiquement acceptable de celui-ci.

6. Herbicide selon la revendication 5, dans lequel l'herbicide a une sélectivité pour une culture cultivée, et est utilisé pour éliminer une mauvaise herbe ayant des feuilles larges pendant la pré-émergence des mauvaises herbes ou la post-émergence des mauvaises herbes.

7. Herbicide selon la revendication 6, dans lequel la culture cultivée est choisie parmi le maïs, le blé et le riz, et la mauvaise herbe ayant des feuilles larges est choisie parmi Quamoclit pennata, Abutilon theophrasti Medicus, Aeschynomene indica, Xanthium strumarium et Catchweed bedstraw.

8. Composition herbicide comprenant, comme un ingrédient actif, un composé choisi parmi un composé à base de pyridine contenant un cycle isoxazoline représenté par la Formule 1 et un sel agrochimiquement acceptable de celui-ci, ou une combinaison du composé à base de pyridine contenant un cycle isoxazoline représenté par la Formule 1 et le sel agrochimiquement acceptable de celui-ci, en une quantité d'environ 0,1 % en poids à environ 99,9 % en poids ; et
au moins un additif choisi parmi un agent tensioactif, un diluant solide et un diluant liquide, en une quantité d'environ 0,1 % en poids à environ 99,9 % en poids. 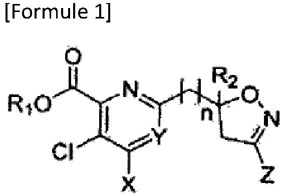
dans laquelle, dans la Formule 1, R₁ représente H, un groupe alkyle en C₁ à C₄, ou un groupe benzyle ;
R₂ représente H ou un groupe alkyle en C₁ à C₂ ;
n représente un nombre entier de 0 à 3 ;
X représente un halogène ou -NR₃R₄ où
R₃ et R₄ sont chacun indépendamment H, un groupe alkyle en C₁ à C₂, ou un groupe C(O)méthyle ;
Y représente CH, C-halogène ou N ; et
Z représente H, un halogène, un groupe alkyle en C₁ à C₄, un groupe haloalkyle en C₁ à C₄, un groupe alcoxy en C₁ à C₄, un groupe alcoxycarbonyle en C₁ à C₄, un groupe phényle substitué par au moins un R₅ où
R₅ représente un halogène, un groupe alkyle en C₁ à C₄, un groupe alcoxy en C₁ à C₄ ou un groupe haloalkyle en C₁ à C₄, ou un groupe hétérocyclique où un groupe hétérocyclique est un cycle saturé ou insaturé à 5 ou 6 chaînons contenant au moins un élément choisi parmi N, O et S.

9. Composition herbicide selon la revendication 8, dans laquelle la composition herbicide est préparée en une formulation quelconque choisie parmi une poudre mouillable, des suspensions, des émulsions, des suspensions fines, des liquides, des liquides dispersibles, une poudre mouillable en granulés, des granules, une poudre, une poudre liquide mouillable, des granules flottants et des comprimés.

10. Composition herbicide selon la revendication 8, comprenant en outre, en plus de l'ingrédient actif, au moins un composant choisi parmi un inhibiteur d'acétyl-CoA carboxylase (ACCase), un inhibiteur d'acétolactate synthase (ALS), un herbicide d'auxine, un inhibiteur de transport d'auxine, un inhibiteur de biosynthèse de caroténoïde, un inhibiteur de 5-enolpyruvylshikimate 3-phosphate synthase (ESPS), un inhibiteur de glutamine synthétase, un inhibiteur de biosynthèse de lipides, un inhibiteur mitotique, un inhibiteur de protoporphyrinogène IX oxydase, un inhibiteur de photosynthèse, un synergiste, un matériau de croissance, un inhibiteur de biosynthèse de paroi cellulaire, et tout herbicide connu.

11. Procédé de préparation du composé à base de pyridine contenant un cycle isoxazoline représenté par la Formule 1 en faisant réagir un composé représenté par la Formule 2 avec un composé représenté par la Formule 3, 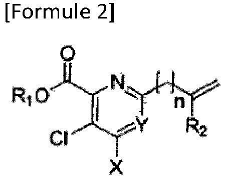 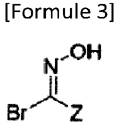 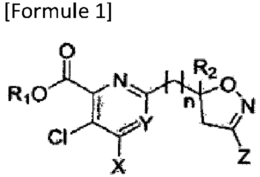 R₁, R₂, n, X, Y et Z dans les Formules 1, 2 et 3 sont les mêmes que ceux décrits dans la revendication 1.

12. Procédé selon la revendication 11, le procédé comprenant le Schéma de Réaction 1 dans lequel, dans des conditions de base, un composé représenté par la Formule 2 dans laquelle une 6ème position est substituée par un groupe vinyle ou un groupe allyle est mis en réaction avec un composé bromo-oxime représenté par la Formule 3 :
